(19) Europäisches Patentamt
European Patent Office
Office européen des brevets

(11) Publication number: 0 167 139 B1

(12) # EUROPEAN PATENT SPECIFICATION

(45) Date of publication of patent specification: 27.02.91

(51) Int. Cl.5: C07D 487/04, A61K 31/40, C07D 519/00

(21) Application number: 85108135.6

(22) Date of filing: 01.07.85

The file contains technical information submitted after the application was filed and not included in this specification

(54) **Carbapenems having an externally alkylated 2-quaternary pyridiniumalkylthio substituent.**

(30) Priority: 02.07.84 US 626579

(43) Date of publication of application: 08.01.86 Bulletin 86/02

(45) Publication of the grant of the patent: 27.02.91 Bulletin 91/09

(84) Designated Contracting States: AT BE CH DE FR GB IT LI LU NL SE

(56) References cited:
EP-A- 0 007 614
EP-A- 0 021 082
EP-A- 0 072 014
DE-A- 3 334 937

(73) Proprietor: MERCK & CO. INC.
126, East Lincoln Avenue P.O. Box 2000
Rahway New Jersey 07065-0900(US)

(72) Inventor: Christensen, Burton G.
770 Anderson Avenue, Apt. 19H
Cliffside Park New Jersey 07010(US)
Inventor: Schmitt, Susan M.
50 M Southwyck Village
Scotch Plains New Jersey 07076(US)
Inventor: Johnston, David B.R.
53 Round Top Road
Warren New Jersey 07076(US)

(74) Representative: Abitz, Walter, Dr.-Ing. et al
Abitz, Morf, Gritschneder, Freiherr von Wittgenstein Postfach 86 01 09
D-8000 München 86(DE)

EP 0 167 139 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

BACKGROUND OF THE INVENTION

The present invention is concerned with carbapenems antibiotics having a 2-position substituent which contains a quaternized pyridinium group which is substituted by an acidic side chain.

Thienamycin is a known carbapenem, broad spectrum antibiotic of the formula:

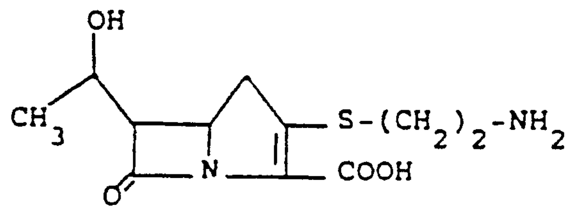

A

Derivatives of A in which the amino group is substituted by a quaternized heteroarylium group are known from EP-A1-0021082. Similar carbapenums containing a quaternized heteroarylium-$C_{1-6}$ alkylenethio 2-position substituent are disclosed in DE-A1-3334937. However, the compounds of the invention are not described as such in the prior art.

The present externally alkylated 2-quaternary pyridinium substituted carbapenems have an antibiotic spectrum equal to or better than A. The present carbapenems also are more resistant than A to degredation by the dehydropeptidase enzyme DHP-I.

SUMMARY OF THE INVENTION

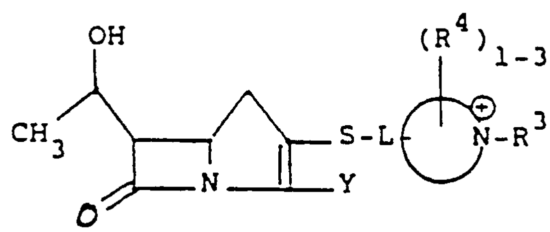

Carbapenems having the formula: wherein at least one of the $R^3$ and $R^4$ is an acidic side chain, $R^3$ is a quaternizing substitutent, $R^4$ is a ring hydrogen or substituent, L is a covalent bond

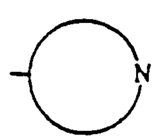

or a bridging group, is pyridiumium and

Y is a carboxy containing substituent.

DETAILED DESCRIPTION OF THE INVENTION

The invention is embodied in a compound having the formula: wherein:

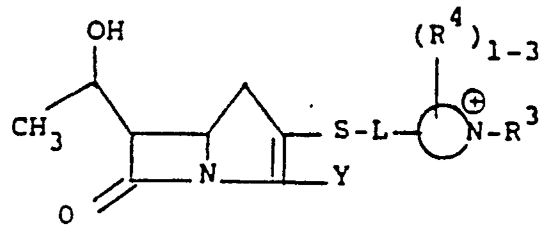

I

L is a covalent bond or a bridging group selected from $-(CH_2)_{1-4}S-$; $-(CH_2)_{1-4}O-$; $-(CH_2)_{1-4}-X''-(CH_2)_{1-4}$ where X" is O, or S; substituted or unsubstituted $C_1$-$C_4$ straight, $C_1$-$C_6$ branched alkyl or $C_3$-$C_7$ cycloalkyl groups wherein the substituents are selected from $C_1$-$C_6$ alkyl, O-$C_1$-$C_6$ alkyl, S-$C_1$-$C_6$ alkyl, halo, OH, $CF_3$, CN, $NH_2$, NH($C_1$-$C_6$ alkyl), N($C_1$-$C_6$ alkyl)$_2$, $CO_2H$, CO

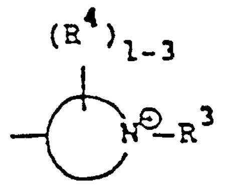

is a pyridinium group

$NH_2$, CONH($C_1$-$C_6$ alkyl), and CON($C_1$-$C_6$ alkyl)$_2$;
wherein at least one of $R^3$ and $R^4$ is: an acidic side-chain of the structure $-(CH_2)_n-X-(CH_2)_m-Y'-A$ which in the case of $R^4$ may also be simply -A where:
n = 0-4
m = 0-4

-A is a member selected from the group consisting essentially of carboxy($CO_2H$), phosphono[P = O-(OH)$_2$], alkylphosphono {P = O(OH)[O($C_1$-$C_4$-alkyl)]}, alkylphosphinyl [P = O(OH)($C_1$-$C_4$ alkyl)], substituted phosphoramido [P = O(OH)NH($C_1$-$C_4$ alkyl) and P = O(OH)NHR$^x$], sulfino ($SO_2H$), sulfo ($SO_3H$), 5-tetra-zolyl ($CN_4H$), arylsulfonamido ($SO_2NHR^x$) and acylsulfonamides represented by the structures $CONHSO_2$($C_1$-$C_4$ alkyl), $CONHSO_2N$($C_1$-$C_4$ alkyl)$_2$, $SO_2NHCO$($C_1$-C4 alkyl) and $SO_2NHCOR^x$ wherein $R^x$ is a heteroaryl group containing from 5 to 11 ring atoms of which up to five are heteroatoms; X = CHR$^s$, CH = CH, phenylene (-$C_6H_4$-), NH, N($C_1$-$C_4$ alkyl), O, S, S = O, C = O, $SO_2$, $SO_2NH$, $CO_2$, CONH, $OCO_2$, OC = O, NHC = O; $R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S; wherein $R^3$ optionally is:

1) an unsubstituted or substituted $C_1$-$C_6$ alkyl radical;
2) and unsubstituted or subsitituted $C_1$-$C_6$ alkenyl radical;
3) an unsubstituted or substitutes $C_1$-$C_6$ alkynyl radical;
4) a $C_3$-$C_7$ cycloalkyl radical in which the ring is substituted or unsubstituted and one or more atoms may be replaced by a heteroatom;
5) a $C_3$-$C_7$ cycloalkyl methyl radical in which the ring may be substituted and one or more atoms may be replaced by a heteroatom;
6) an unsubstituted or substituted $C_5$-$C_7$ cycloalkenyl radical;
7) and unsubstituted or substituted biavelent $C_2$-$C_6$ alkylidene radical, optionally interrupted by a heteroatom, and joined to the heteroarylium group to form a ring which is carbocyclic or in which one or more atoms is replaced by a heteroatom. The new ring may contain one or more double bonds;
8) an unsubstituted or substituted phenyl radical;
9) an unsubstituted or substituted penyl ($C_1$-$C_4$ alkyl) radical;
10) a cyano ($C_1$-$C_4$ alkyl) radical;
11) a carbamoyl ( $C_1$-$C_4$ alkyl) radical;
12) a hydroxy ($C_1$-$C_4$ alkyl) radical; or
13) an amino ($C_1$-$C_4$ alkyl) radical in which the nitrogen atom is unsubstituted or substituted with one to three $C_1$-$C_4$ alkyl groups; wherein the substitients in the above definitions of $R^3$ are independently selected from the group consisting of the definitions of $R^4$ set out below; and wherein $R^4$ optionally is independently selected from:

a) a trifluormethyl group;
b) a halogen atom;
c) an unsubstituted $C_1$-$C_4$ alkoxyl radical;
d) a hydroxy group;
e) an unsubstituted ($C_1$-$C_6$ alkyl) carbonyloxy radical;
f) a carbamoyloxy radical which is unsubstituted or substituted on nitrogen with one or two $C_1$-$C_4$ alkyl groups;
g) a $C_1$-$C_6$ alkylthio radical, $C_1$-$C_6$ alkylsulfinyl radical or $C_1$-$C_6$ alkylsulfinyl radical, each of which is unsubstituted on the alkyl group;
h) a sulfamoyl group which is unsubstituted or substituted on nitrogen by one or two $C_1$-$C_4$ alkyl groups; i) an amino group;
j) a mono ($C_1$-$C_4$ alkyl) amino or di($C_1$-$C_4$ alkyl)amino group, each of which is unsubstituted on the alkyl group;
k) a formylamino group;
l) an unsubstituted ($C_1$-$C_6$ alkyl)carbonylamino radical;
m) a ($C_1$-$C_4$ alkoxy) carbonylamino radical;
n) a ureido group in which the terminal nitrogen is unsubstituted or substituted with on or two $C_1$-$C_4$ alkyl groups;
o) a ($C_1$-$C_6$ alkyl) sulfonamido group;
p) a cyano group;
q) a formyl or cetalized formyl radical;
r) an unsubstitued ($C_1$-$C_6$) alkyl)carbonyl radical wherein the carbonyl is free or acetaliszed;
s) an unsubstituted phenylcarbonyl radical;
t) a hydroximinomethyl radical in which the oxygen or carbon atom is optionally substituted by a $C_1$-$C_4$ alkyl group;
u) a($C_1$-$C_6$ alkoxy)carbonyl radical;
v) a carbamoyl radical which is unsubstituted or substituted on nitrogen by one or two $C_1$-$C_4$ alkyl groups;
w) an N-hydroxycarbamoyl or N($C_1$-$C_4$ alkoxy)carbamoyl radical in which the nitrogen atom may be additionally substituted by a $C_1$-$C_4$ alkyl group:
x) a thiocarbamoyl group;

y) an amidino group 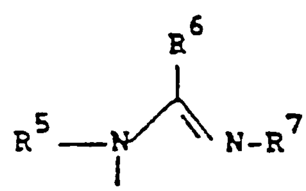

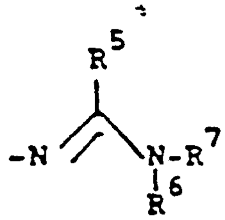

where $R^5$, $R^6$ and $R^7$ are independently hydrogen. $C_1$-$C_4$ alkyl or wherein two of the alkyl qroups together form a $C_2$-$C_6$ alXylidene radical optionally interrupted by a heteroatom and joined together to form a ring:

z) a carboxamidino group 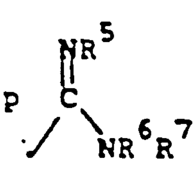 , where

$R^5$, $R^6$ and $R^7$ are as defined above;
aa) a guinidinyl group where $R^6$ in y) above is $NR^8R^9$

ab) hydrogen;
ac) an unsubstituted $C_1$-$C_6$ alkyl radical;
ad) an unsubstituted $C_1$-$C_6$ alkenyl radical;
ae) an unsubstituted $C_1$-$C_6$ alkynyl radical;
af) a $C_3$–$C_7$ cycloalkyl radical in which the ring is unsubstituted and one or more atoms may be replaced by a heteroatom;
ag) a $C_3$–$C_7$ cycloalkyl methyl radical in which one or more atoms may be replaced by a heteroatom;
ah) an unsubstituted $C_5$-$C_7$ cycloalkenyl radical;
ai) an unsubstituted radical; and
aj) an unsubstituted ($C_1$-$C_4$ alkyl) radical; and wherein heteroatom in the above definitions of A, $R^3$ and $R^4$ means nitrogen, oxygen or sulfur, independently selected where more than one heteroatom is involved; and

Y is selected from: i) COOH or a pharmaceutically acceptable ester or salt thereof.
ii) $COOR^1$ wherein $R^1$ is a removable carboxy protecting group.
iii) COOM wherein M is an alkali metal, or
iv) $COO^{\ominus}$: provided that when Y is other than iv) a counterion $Z^{\oplus}$ is provided.

As used herein, the term "heteroatom" means nitrogen, oxygen, or sulfur, independently selected where more than one heteroatom is involved.

Representative L groups are $-CH_2-$, $-CH(CH_3)-$, $-CH(C_2H_5)-$, $-(CH_2)_{2-4}$, $-CH(CH_3)-CH_2-$, $CH_2-CH(OCH_3)-$. $-CH(CH_3)-(CH_2)_2-$, $-CH(CH_2OH)-CH_2-$, $-CH(CF_3)-CH_2-$, $-CH_2-CH_2-S$, $-CH_2-CH_2-O$, $-(CH_2)_2-S-CH_2-$, $-(CH_2)_2-O-CH_2-O-CH_2$, or a single covalent bond .

A preferred L group is a substituted or unsubstituted $C_1$-$C_6$ linear or branched chain alkyl. A more preferred L group is $-CH_2-$, $-CH(CH_3)-$ or $-(CH_2)_2-$.

Examples of useful $R^3$ groups are $-CH_3$, $-(CH_2)_{1-3}-CH_3$

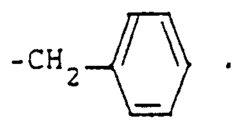 .

$-(CH_2)_{1-3}-O-CH_3$, $-CH_2-CN$ $CH_2-COO(C_1-C_3$ alkyl). $-(CH_2)_2-N(C_1-C_3$ alkyl$)_2$.

$-CH_2-COOH$, $-(CH_2)_2-SO_3H$, 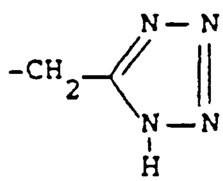 ,

$-CH_3-COOH$, $-(CH_3)_3-SO_3H$, $-CH_3$ or $-CH_2)_2-\overset{\oplus}{N}(CH_3)_3$.

Preferred $R^3$ groups are the $C_1$-$C_6$ alkyls, both substituted and unsubstituted.

Preferred substituents are CN, $CON(CH_3)_2$, $CONH_2$, $SOCH_3$, $SO_2CH_3$, $CO_2H$, $SO_3H$, $SO_2NH_2$ and

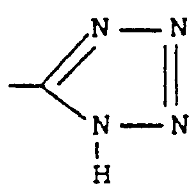

Examples of useful $R^4$ groups are OH, $NH_2$, $N(C_1$-$C_3$ alkyl), $OC_1$-$C_4$alkyl, $C_1$-$C_4$alkyl. CN, or $CF_3$.

Preferred $R^4$ groups are $CO_2H$, $CH_2CO_2H$, $SO_3H$, $CH_2SO_3H$, $CONH_2$, CN, $SO_2NH_2$,

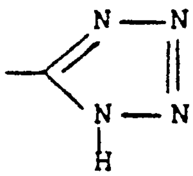

, or

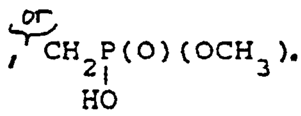

Of particular interest and the most preferred group are compounds of the present invention wherein the substituent on the N-containing mono- or bicyclic quaternary heteroaryl group in the 2-position is an acidic function as defined above and the Y substituent in the 3-position is $COO^{\ominus}$ as defined above, thus forming a zwitterion with the positive charge of the quaternary nitrogen. The acidic function is anionic and the compounds are thus anionic zwitterions, i.e., they have a net negative charge. This novel characteristic has been found to result in at least one surprising and important improvement in the biological properties of the compounds i.e., reduced CNS side-effects. ` I A more particular group of the compounds, those wherein the acidic function is a sulfoalkyl group of the formula ($C_{1-4}$ alkyl)$SO_3^{\ominus}$, have been found to have the additional surprising and important biological property of enhanced potency against Pseudomonas species, an especially important nosocomial pathogen.

Examples of useful -S-L- 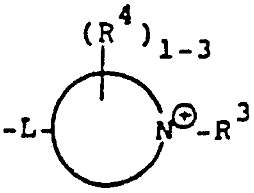 groups are:

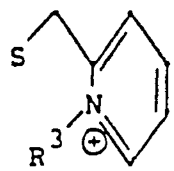
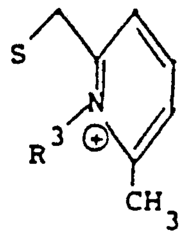
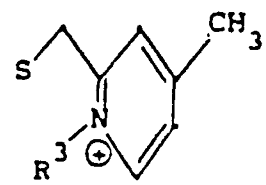
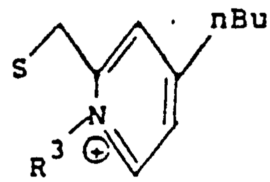

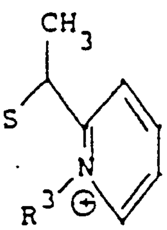
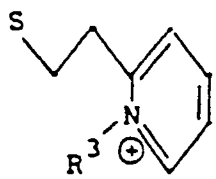
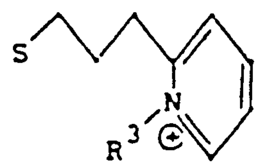

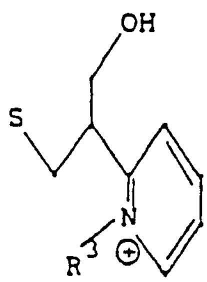
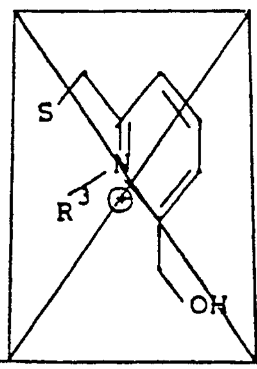
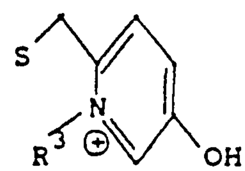
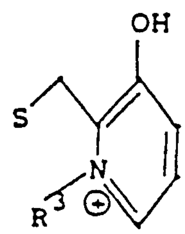

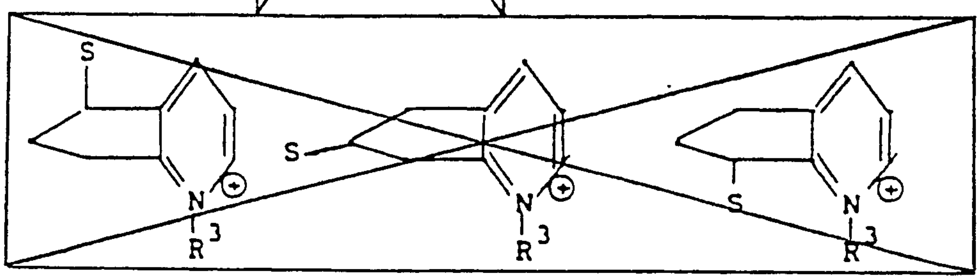

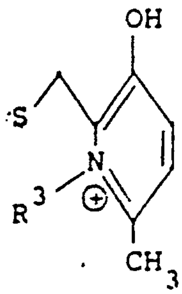
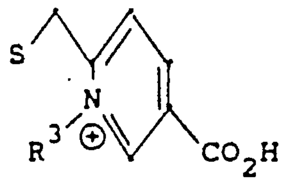
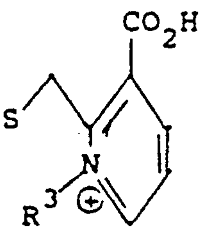
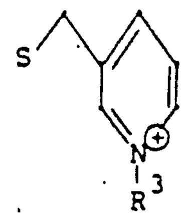

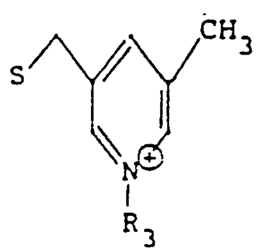
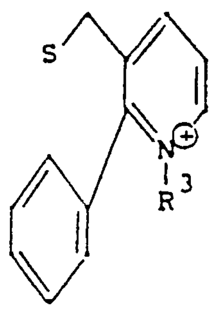
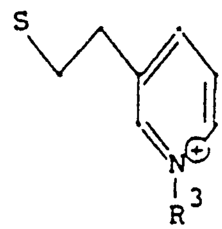
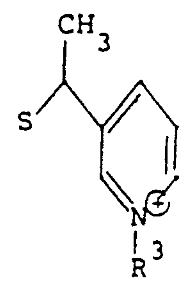

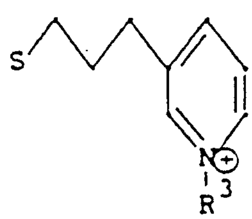
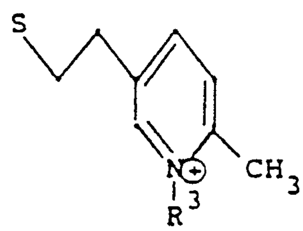
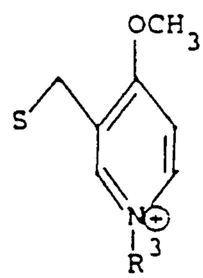

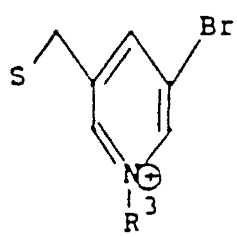
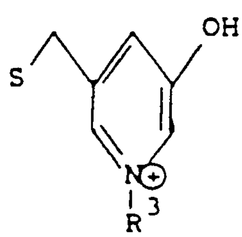
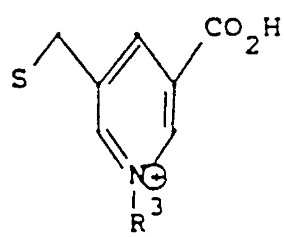

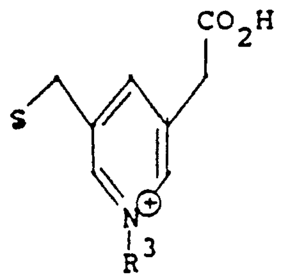
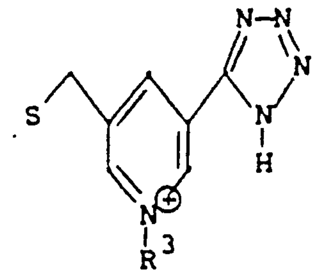
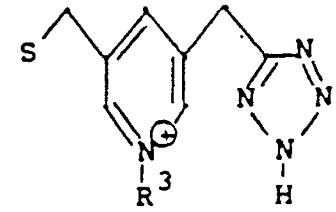

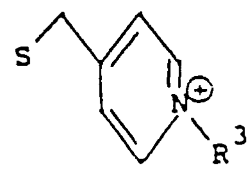

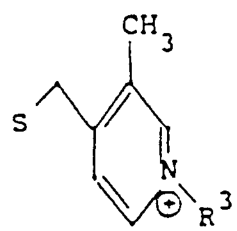
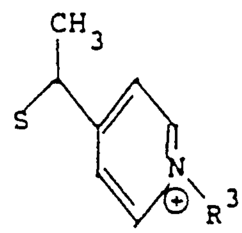
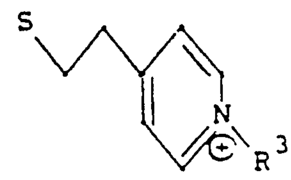

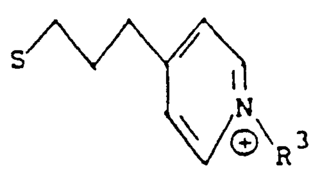
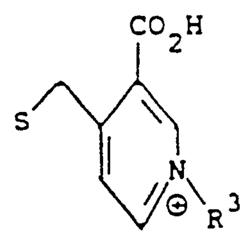
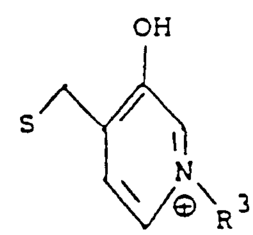

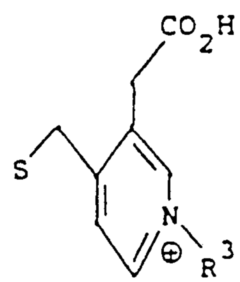
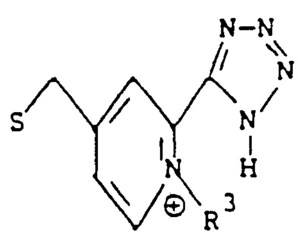

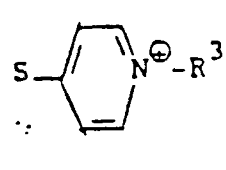
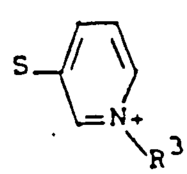
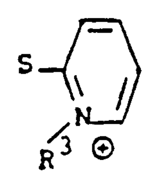

Preferred compounds of Formula I are of the structure:

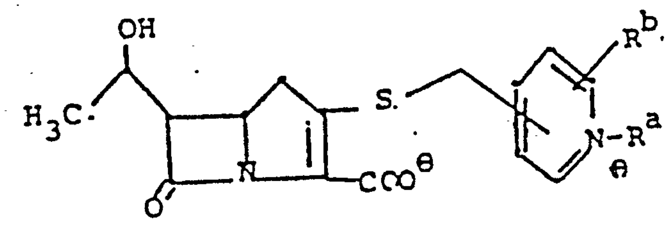

wherein

$R^a$ is $C_{1-4}$ alkyl; or an acidic side-chain of the structure $-(CH_2)_n$-X-$(CH_2)_m$-Y'-A where:
n = 0-4
m = 0-4
x = $CHR^s$, CH=CH, phenylene ($-C_6H_4-$), NH, N($C_1$-$C_4$ alkyl), 0, S, S=O, C=O, $SO_2$, $SO_2NH$, $CO_2$, CONH, $OCO_2$, OC=O, NHC=O;

$R^s$ = H, O($C_1$-$C_4$ alkyl), $NH_2$, NH($C_1$-$C_4$ alkyl), N($C_1$-$C_4$ alkyl$)_2$, CN, $CONH_2$, CON($C_1$-$C_4$' alkyl$)_2$, $CO_2H$, $SO_2NH_2$, $SO_2NH$($C_1$-$C_4$ alkyl);

Y' = single bond, NH, N($C_1$-$C_4$ alkyl), O, S;

A is as defined in claim 1;

$R^b$ is hydrogen; cyano; or an acidic side-chain of the structure -A or $-(CH_2)$n-X-$(CH_2)_m$-Y'-A where:
n = 0-4
m = 0-4
X = $CHR^s$, CH=CH, phenylene ($-C_6H_4-$), NH, N($C_1$-$C_4$ alkyl), O, S, S=O, C=O, $SO_2$, $SO_2NH$, $CO_2$, CONH, $OCO_2$, OC=O, NHC=O; $R^s$ = H, O($C_1$-$C_4$ alkyl), $NH_2$, NH($C_1$-$C_4$ alkyl), N($C_1$-$C_4$ alkyl$)_2$, CN, $CONH_2$, CON($C_1$-$C_4$ alkyl$)_2$, $CO_2H$, $SO_2NH_2$, $SO_2NH$($C_1$-$C_4$ alkyl);

Y' = single bond, NH, N($C_1$-$C_4$ alkyl), O, S;

A is as defined in claim 1; provided that $R^a$ or $R^b$ must be an acidic side-chain.

The compounds of Formula I include inner (Zwitterion) salts when Y is $cOO^{\ominus}$ e.g.

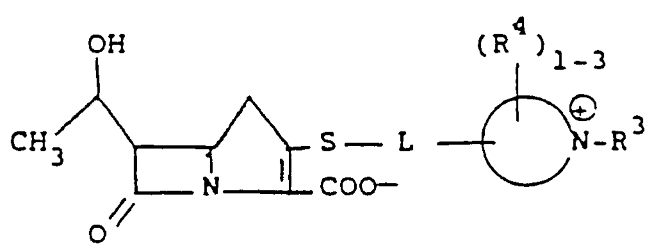

0 or, when Y is other than $COO^{\ominus}$, salts with an external, physiologically acceptable counterion $z^{\ominus}$ such as $Cl^{\ominus}$, $Br^{\ominus}$, $I^{\ominus}$, $OCH_3^{\ominus}$, or $OSO_2CF_3$, or OP(O)(O phenyl$)_2^{\ominus}$.

The inner salts are preferred.

Again, the compounds of Formula I include the stereoisomers as mixtures and as separate isomers.

A preferred isomer configuration is:

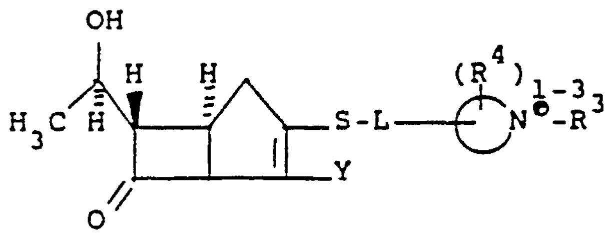

Ia

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella Pneumoniae, Bacillus subtilis, Salmonella typhosa, Pseudomonas and Bacterium proteus. The antibacterials of the invention are not limited to utility as medicaments: they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means: those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles. and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg to about 1500 mg of the active ingredient: however, in general, it is preferable to employ a dosage amount in the range of from about 250 mg to 1000 mg. In parenteral administration. the unit dosage is usually the pure compound I in sterile water solution or in the form of a soluble powder intended for solution:

The preferred method of administration of the formula I antibiotic is parenteral by i.v. infusion, i.v. bolus, or i.m. injection.

For adults, 5-50 mg of Formula I antibiotic per kg of body weight given 2, 3, or 4 times per day is preferred. Preferred dosage is 250 mg to 1000 mg of the Formula I antibiotic given two (b.i.d. ) three (t.i.d. ) or four (q.i.d. ) times per day. More specifically, for mild infections, and particularly urinary tract infections, a dose of 250 mg t.i.d. or q.i.d. is recommended. For moderate infections against highly susceptible grab positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibiotic compounds of Formula I are of the broad class known as carbapenems or I-carbadethiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102615.6, filed July 24, 1979 (Publication no. 7614 and No. 82107174.3, filed August 9, 1980 (publication no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.) disclose suitable inhibitors, combination compositions and methods of treatment. they are incorporated herein by reference. A preferred weight ratio of I compound:DHP inhibitor in the combination compositions is about 1:1. A preferred DHP inhibitor is 7-(L-2-amino-2-carboxyethylthio)-2-(2,2-dimethyl-cyclopropanecarboxamide)-2-heptenoic acid or a useful salt thereof. q

These combination compositions are another embodiment of the present invention.

The compounds of Formula I may be prepared by any convenient process.

A. One such process is illustrated in the following reaction equations:

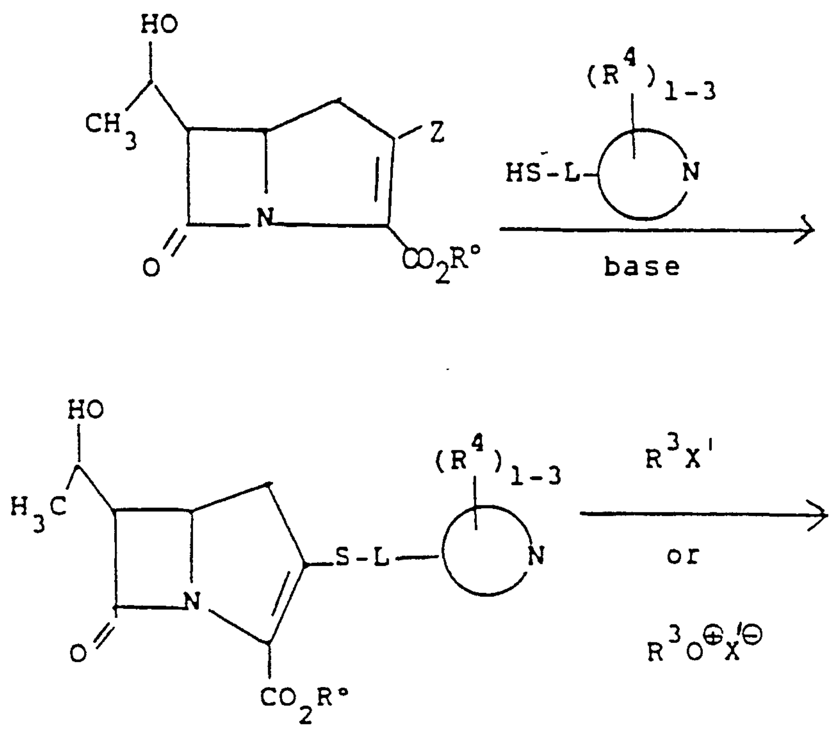

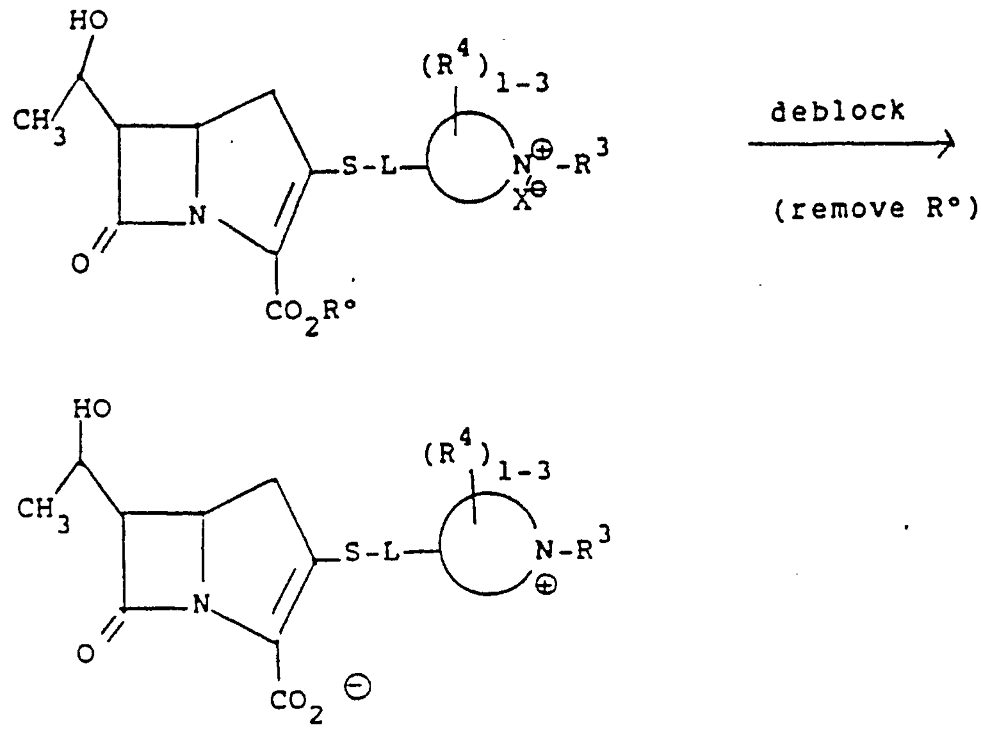


I

wherein Z is a leaving group such as -OPO(OØ)$_2$,

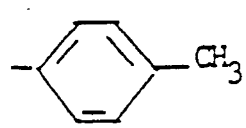

X' is a leaving group such as Br. I. $OSO_2CF_3$,

$-OSO_2F$, $OSO_2$

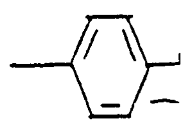

$CH_3$ or X'is an anionic group such as $BF_4$, $SbF_6$ or $PF_6$: and R° is a protecting group such as p-nitrobenzyl or allyl.

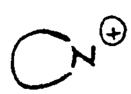

$R^3$, L and $R^4$ are as defined above.

The side chain addition reaction is carried out in a solvent such as acetonitrile, dimethylformamide, dimethylacetamide or N-ethyl-pyrrolidinone in the presence of a base such as N,N-diisopropylethylamine, triethylamine or 4-dimethylaminopyridine at a temperature of from -40°C to 25°C for a period of five minutes to ten hours. The alkylation reaction is conducted in a solvent such as dichloromethane, dimethylformamide. acetonitrile or dimethylacetamide at a temperature of from -20°C to 25°C for a period of 1 to 24 hours. The deblocking reaction wherein R° is p-nitrobenzyl is usually conducted in an aqueous system containing cosolvents such as tetrahydrofuran, ethanol, n-butanol, i -amyl alcohol, or ethyl acetate and a pH 6.8 to 7.0 aqueous buffer. Suitable buffers include phosphate buffers and buffers derived from

non-nucleophilic amines such as N-methylmorpholine or morpholinopropane sulfonic acid. The reaction is conducted at 0°C to 40°C for 0.5 to 5 hours under 1-100 atmospheres of hydrogen in the presence of a catalyst such as 10% palladium on carbon or 20% palladium hydroxide on carbon. The final products are purified by ion exchange chromatography and/or reverse phase chromatography. When a pharmaceutically acceptable ester of the final product is desired. the deblocking step is omitted and the appropriate R° group is incorporated into the starting material.

B. A second process is illustrated by the following set of equations:

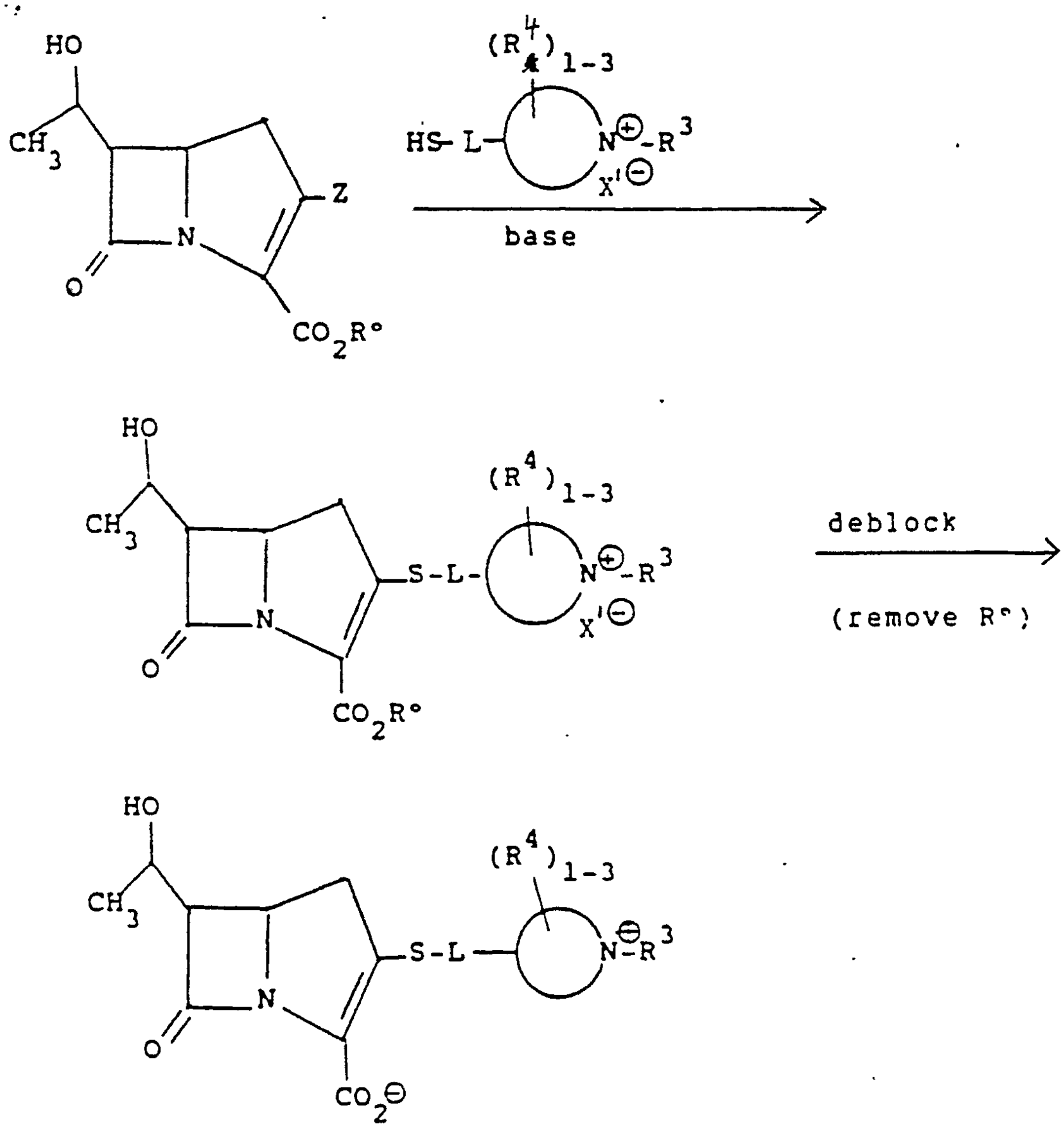


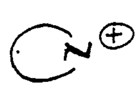

wherein Z, X', L, R°, $R^3$ and $R^4$ are as defined above.

The difference between the above process and that earlier described is that the side chain moiety is alkylated with the group $R^3$ prior to addition to the carbapenem nucleus. The side chain addition step and deblocking are conducted as described above.

C. A third process is illustrated by the following set of equations:

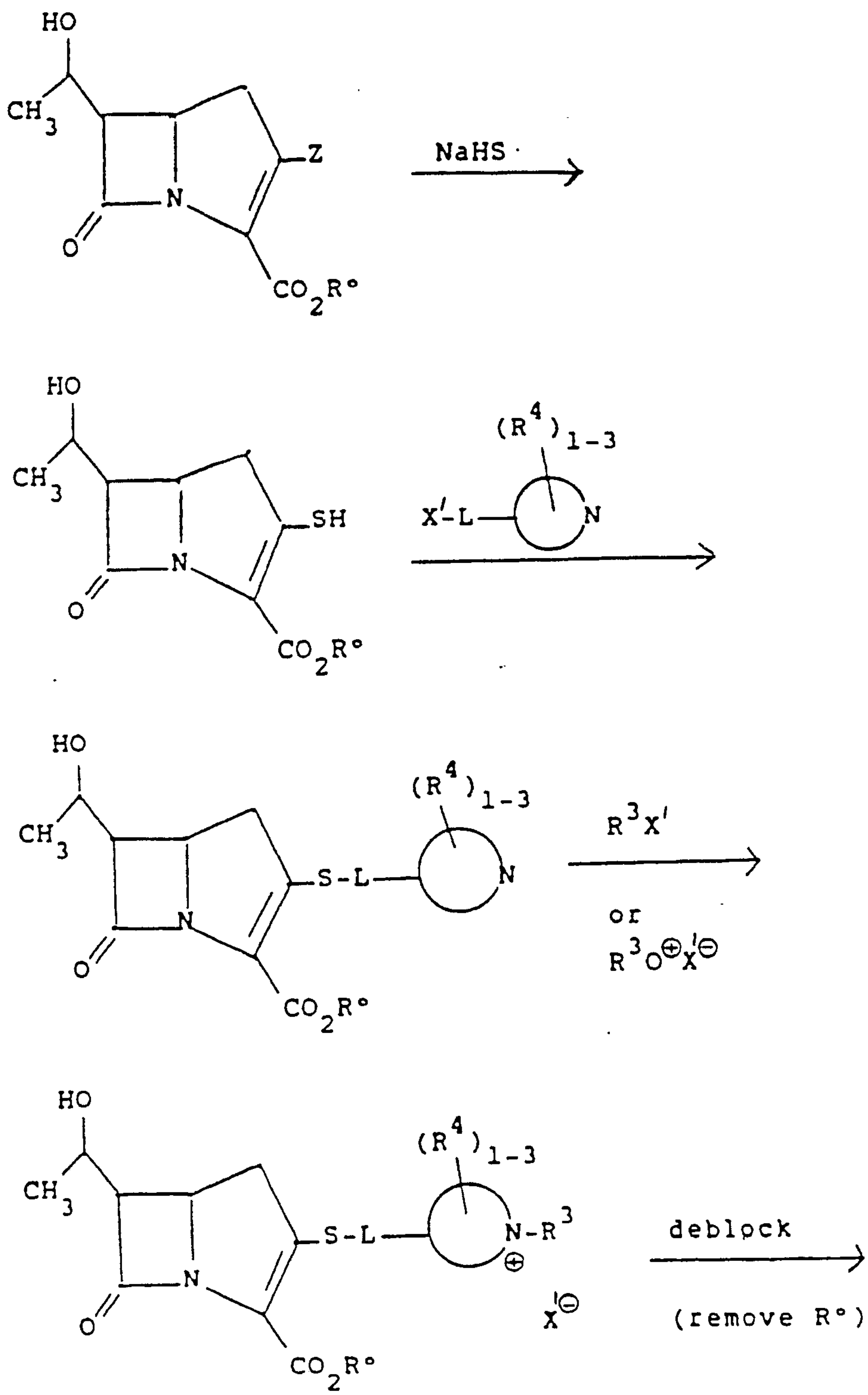

NaHS
R3X'
or
R3O⊕X'⊖
deblock
(remove R°)


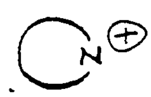

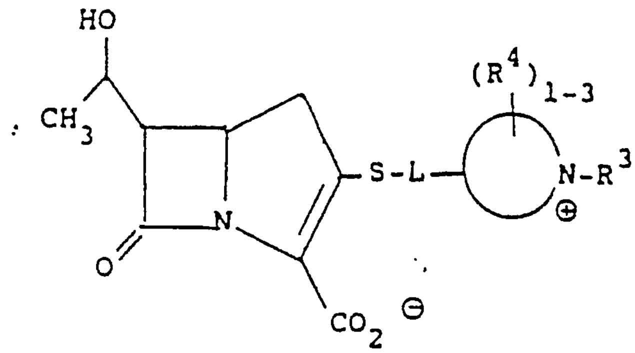

I

wherein Z, X', L, R° R³ and R⁴ are as previously defined.

In this case the 2-mercapto intermediate is generated from the activated carbapenem upon exposure to sodium hydrosulfide in dimethylformamide or dimethylacetamide at a temperature of from -50° C to -20° C for a period of five minutes to one hour. The sulfur atom is alkylated in a solvent such as acetonitrile, dimethylformamide or dimethylacetamide in the presence of a base such as N,N-diisopropylethylamine, triethylamine, or 4-dimethylaminopyridine at a temperature of from -40° C to 25° C for a period of from ten minutes to eight hours. The side chain alkylation, removal of R° and purification of I are conducted as described above.

D . A fourth process is illustrated by the following set of equations:

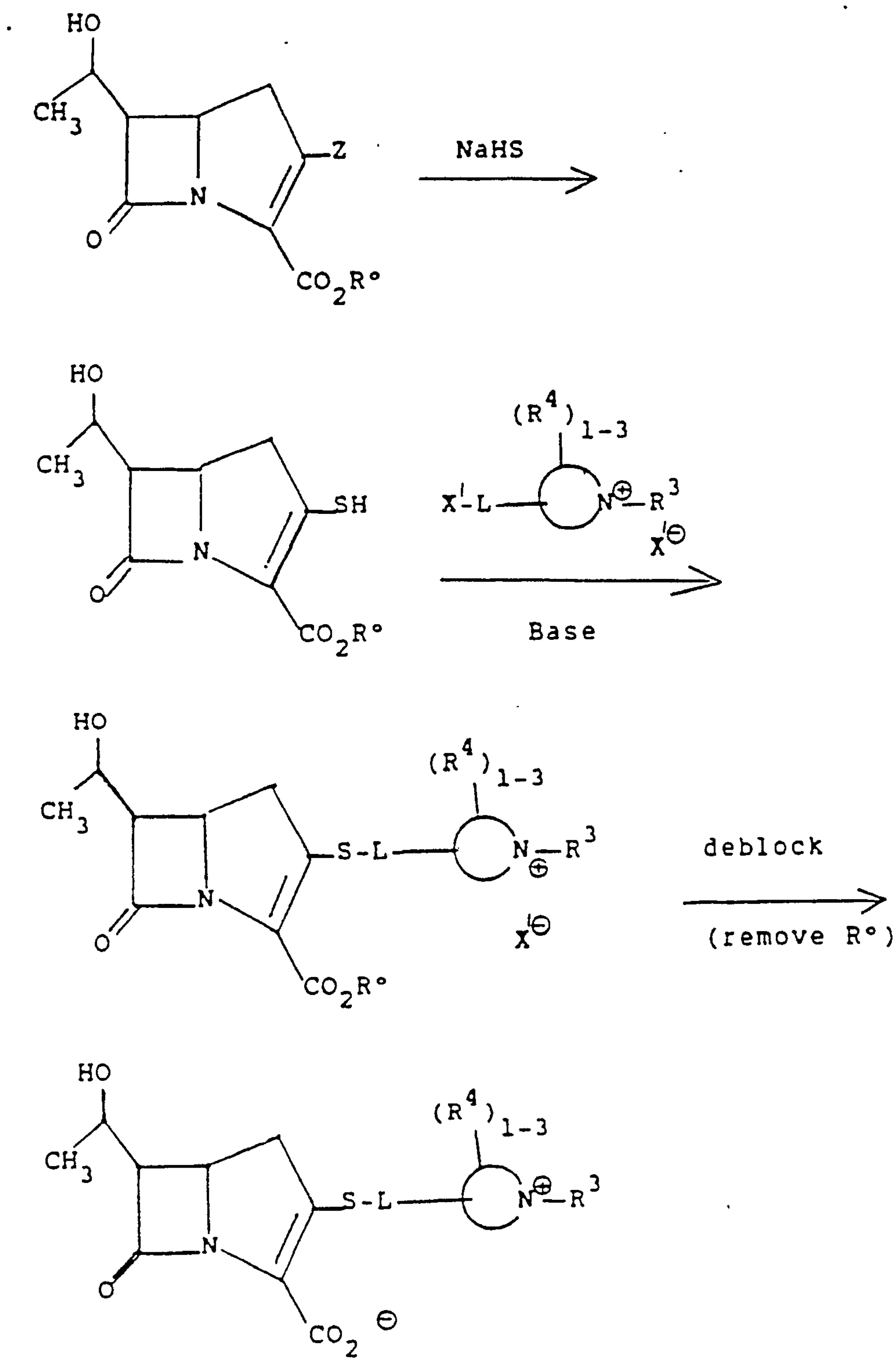


I

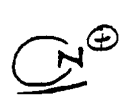

wherein Z, X', R°, $R^3$, and $R^4$ are as previously defined.

The difference between this process and that described in process C is that the side chain moiety is alkylated with the group $R^3$ prior to addition to the carbapenem nucleus. The side chain addition step and the deblocking are conducted as described above.

The following examples illustrate the preparation of compounds of Formula I. The temperature is in

degrees Celsius unless otherwise indicated.

EXAMPLE 1

Step A.

Preparation of p-Nitrobenzyl (5R,6S)-6-(1(R)-hydroxy-ethyl)-2-(2-pyridylmethylthio)-carbapen-2-em-3-carboxylate 2.

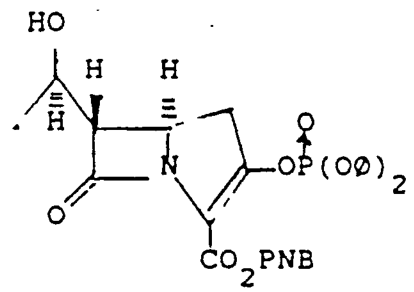

1

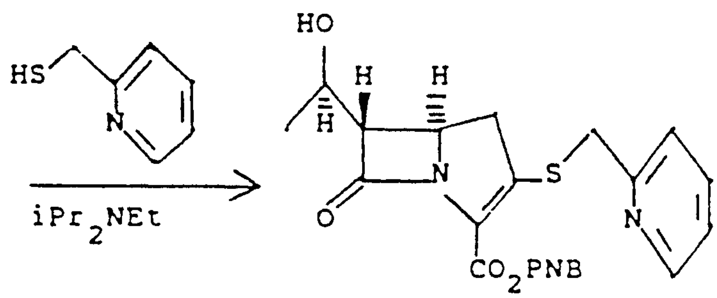


2

A solution of vinyl phosphate 1 (2.32 g, 4 mmoles) in anhydrous acetonitrile is cooled to -20° (ice-methanol) under a nitrogen atmosphere and treated with 2-mercaptomethylpyridine (0.554 ml, 5.0 mmoles) and diisopropylethylamine (0.871 ml, 5.0 mmoles). The resulting mixture is stirred at -20 to -15° for 60 minutes during which time a precipitate formed. The mixture is diluted with ethyl acetate (16 ml) and aged at -15° to -5° for 30 minutes. The precipitate is collected, washed with ice-cold ethyl acetate and dried in vacuo to give the product (1.095 g) as a white solid.

The filtrate and washings are diluted with ethyl acetate, washed with 0.1M pH 7 phosphate buffer two times and brine, dried over magnesium sulfate, filtered and concentrated to a semisolid. This material is triturated with ethyl acetate and ether two times and dried in vacuo to yield an additional 0.498 g of product. Total yield of 2 is 1.593 g, 87%.

Step B.

Preparation of (5R,6S)-6-(1(R)-hydroxyethyl)-2-(1-methyl-2-pyridinium)methylthio-carbapen-2-em-3-carboxylate 4.

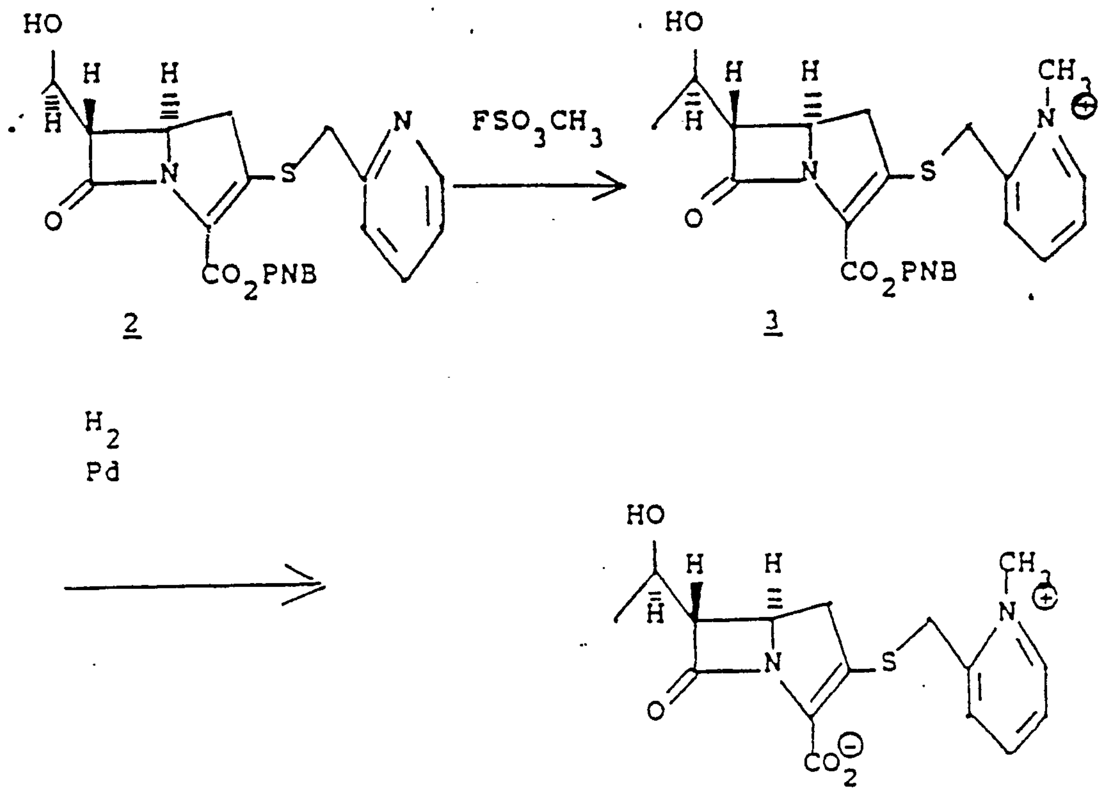


2 3

4

To a magnetically stirred solution of 2 (0.853 g, 1.87 mmole) in 15.3 ml of dichloromethane is added methyl fluorosulfonate (0.160 ml, 1.97 mmole) at room temperature. The reaction is followed by UV assay of removed aliquots of the solution. After two hours, a yellow oil separates and the UV absorbance at 318 nm of the dichloromethane layer drops to 4% of original. The upper layer is decanted and the lower layer washed two times with 5 ml portions of dichloromethane. The residual oil is assayed by UV for 3 then pumped to a foam in vacuo. The crude 3 thus produced is dissolved in a mixture of 6 ml of N,N-dimethylacetamide, 37 ml n-butanol, 19 ml ethyl acetate and 37 ml 0.5N N-methylmorpholine-HCl pH 7.0 buffer and 280 mg of 20% palladium hydroxide on carbon added. The mixture is hydrogenated at 2.9 bar (42 psi) with shaking for 70 minutes. At the end of this period, the mixture is removed, filtered through a prewashed celite bed with 5 to 10 ml water and the organic phase discarded. The aqueous phase is washed with 2 X 50 ml of dichloromethane and concentrated in vacuo to a volume of 23 ml. The solution of crude product is charged onto a 2.8 X 38 cm column of Dowex® 50-X4 ($Na^+$ cycle) at 5° and eluted with water. The eluate is monitored by UV and fractions containing the desired product are pooled, concentrated to 150 ml and lyophilized to yield 272 mg of final product 4. It is also possible to reverse the steps of alkylation and deprotection of the carboxy group.

Step C.

Preparation of 1-methyl-2-mercaptomethyl-pyridinium perchlorate 7.

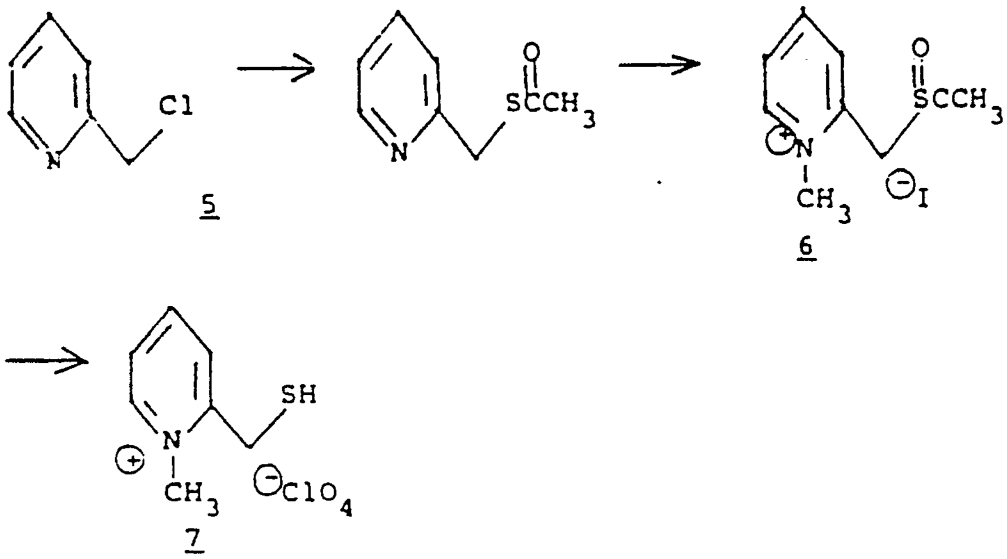

5 6 7

To a solution of 2-picolyl chloride hydrochloride (5.00 g 30.5 mmol) and potassium thio acetate (4.18 g, 36.6 mmol) in 50 ml N,N-dimethyl-formamide, triethylamine (4.25 ml, 30.5 mmol) is added slowly to yield a pink solution. The mixture is heated to 80° and held there for 2 hours, after which time the solvent is removed in vacuo to yield a brown oil. The residue is taken up in ethyl acetate, washed with water and brine, dried over magnesium sulfate, filtered and concentrated to yield crude 5 as a dark oil (5.0 g).

Thioester 5 (2.0 g, 12 mmol) is dissolved in 10 ml N,N-dimethylformamide under nitrogen and methyl iodide (7.5 ml, 120 mmol) is added dropwise. The mixture is stirred 20 hours at room temperature, then the solvent is removed in vacuo to yield a brown powder which is triturated with dichloromethane, filtered and dried in vacuo to yield 2.21 g of 6 as a tan powder. Additional material (1.07 g) is obtained by concentration of an aqueous extract of the dichloromethane wash from the trituration step. (nmr ($D_2O$)δ 2.49 (s): 4.42 (s); 7.9 to 9.0 (m).

Pyridinium thioester 6 (102.7 mg, 0.33 mmol) is suspended in 0.63 ml 2N methanolic perchloric acid and stirred at room temperature under nitrogen for 66 hours. The resulting tan suspension is filtered and the solid washed with ether to give 51.7 mg of thiol 7 as a tan powder (nmr ($D_2O$)δ 4.23 (s): 4.43 (s), 7.8 to 8.6 (m).

Step D

Preparation of (5R,6S)-6-(1(R)-hydroxyethyl)-2-(1-methyl-2-pyridinium)methylthio-carbapen-2-em-3-carboxylate 4.

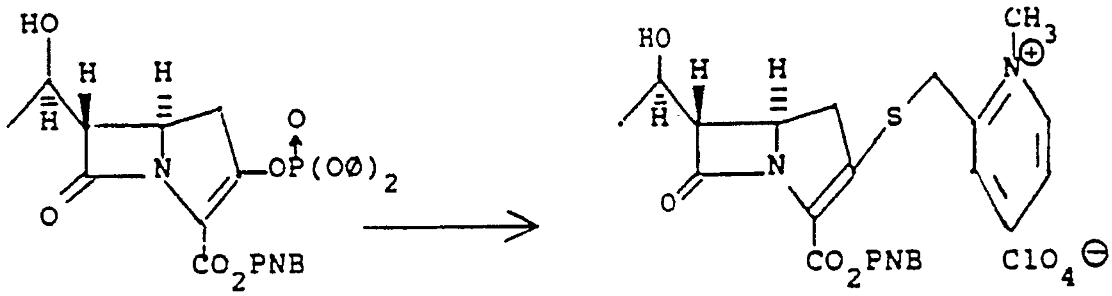

1 3

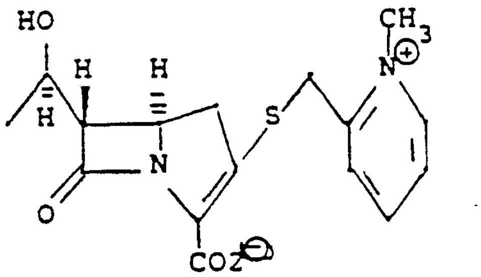

4

To a solution of vinyl phosphate 1 (58 mg, 0.1 mmol) and thiol 7 (34.9 mg) in 0.32 ml of N,N-dimethylacetamide at -20° under nitrogen is added N,N-diisopropylethylamine (34.8 µl, 0.2 mmol). The mixture is aged 25 minutes at -20° then transferred directly to a hydrogenation vessel with 1.9 ml i-propanol, 1.0 ml ethyl acetate and 1.9 ml water. Phosphate buffer (pH 7, 0.1M, 1.0 ml) and 20% palladium hydroxide on carbon (15 mg) is added and the mixture hydrogenated at 3.17 bar (46 psi) for two hours. The catalyst is removed by filtration and the filtrate diluted with 5 ml ethyl acetate and 5 ml water. The aqueous phase is separated, washed with ethyl acetate, concentrated to ca. 1 ml in vacuo and the product purified by chromatography on Dowex® 50 -X4 ($Na^+$) as described above to yield 6.3 mg of product 4.

The starting material 1 can be prepared by the following procedure:

A solution of p-nitrobenzyl (5R-6S)-6[1(R)-hydroxyethyl]-2-oxocarbapenam-3(R)-carboxylate (200 mg, 0.574 mmol) in acetonitrile (0.60 ml) was treated at ice-water bath temperature with diphenylchlorophosphate (0.125 ml, 0.603 mmol) and N,N-diisopropyl-ethylamine (0.119 ml, 0.683 mmol) and stirred at 0° for 25 minutes. I

EXAMPLE 2

Step A: Preparation of p-Nitrobenzyl (5R,6S)-6[1(R)-hydroxyethyl]-2-[(1-methyl-5-imidazolyl)methylthio]carbapen-2-em-3-carboxylate (8)

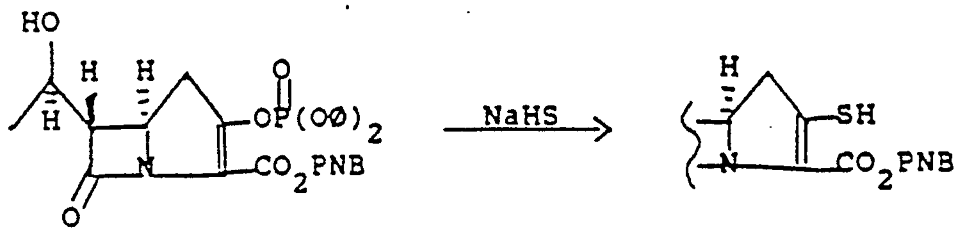


1

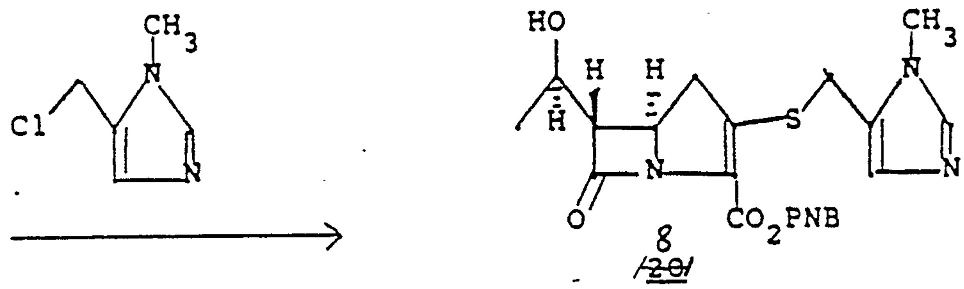

8

A solution of vinyl phosphate 1 (674 mg, 1.16 mmol) in anhydrous N,N-dimethylformamide (DMF, 3.9 ml) was cooled in a dry ice-acetonitrile bath (-40° C) under a $N_2$ atmosphere and treated dropwise over 2 minutes with a solution of sodium hydrogen sulfide (68.4 mg, 1.22 mmol) in DMF (2 ml). The reaction mixture was treated with N,N-diisopropyl-ethylamine (0.647 ml, 3.72 mmol), stirred at -40° C for 20 minutes, then treated dropwise with a solution of 1-methyl-4-chloromethylimidazole hydrochloride (203.6 mg, 1.22 mmol) in DMF (2.4 ml). After stirring an additional 20 minutes at -40° C, the reaction mixture was diluted with ethyl acetate (100 ml), washed with water (4 x 100 ml) and brine, dried with $MgSO_4$, filtered, and evaporated in vacuo to give a yellow-brown solid (367 mg). This material was triturated with 1:1 ethyl acetate-ether and dried in vacuo to afford the title compound 8 (250 mg. 47%) as a yellow-brown solid. IR (Nujol) $\vartheta$ max 1769, 1690, 1517, 1333 $cm^{-1}$; UV (dioxane) $\lambda$ max 319 nm ($\epsilon$12,600), 267 (11,900); NHR ($CDCl_3$)$\delta$ 1.37 (d, J=6.3Hz, C H 3 CHOH), 3.14 (dd, J=17.8 and 8.6Hz, CHC Ha Hb), 3.21 (dd, J=2.7 and 6.7Hz, H6), 3.42 (dd, j=17.8 and 9.7Hz, CHCHa Hb ), 3.68 (s, $NCH_3$), 4.03, 4.12 (ABq, J=14.6Hz, $SCH_2$), 4.25 (m, H5 and $CH_3$C H OH), 5.23, 5.50 (ABq, J=13.8Hz, $CH_2$Ar), 7.00 (s, imidazole-H), 7.47 (s, imidazole-H), 7.65 (d, J=8.7Hz, 2 ArH), 8.22 (d, J=8.7Hz, 2ArH).

Step B: Preparation of p-Nitrobenzyl (5R.6S)-6[1(R)-hydroxyethyl]-2-[(l,3=dimethyl-4-imidazolium)-methylthio]carbapen-2-em-3-carboxylate fluorosulfate (9)

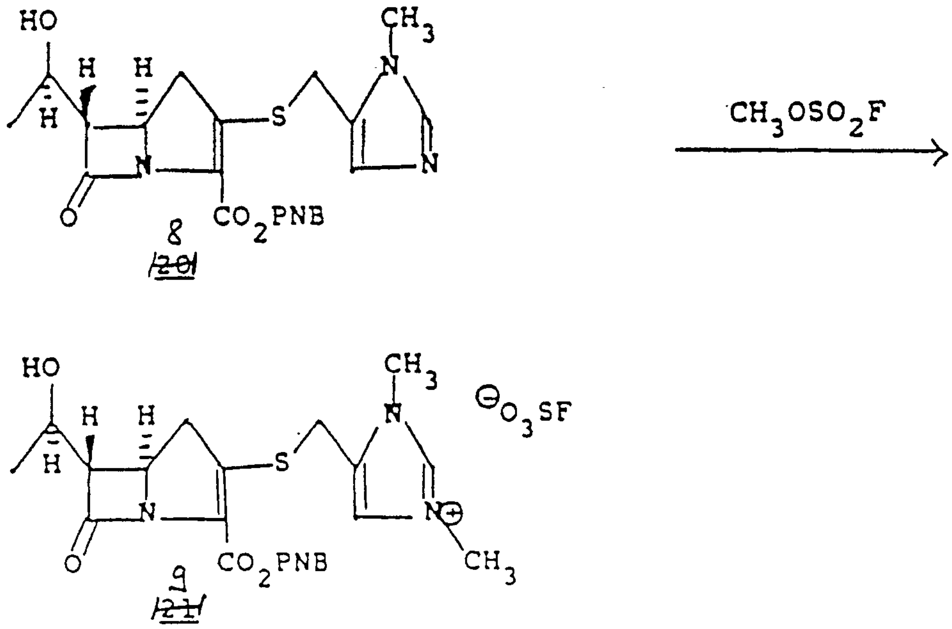


8 ~~20~~

9 ~~21~~

A solution of carbapenem derivative 8 (223.6 mg, 0.49 mmol) in anhydrous methylene chloride (10 ml) was cooled in an ice-bath and stirred under a nitrogen atmosphere while a solution of methyl fluorosulfate (0.042 ml, 0.52 mmol) in methylene chloride (2 ml) was added dropwise over 5 minutes. A gummy precipitate formed which, on continued stirring at 0° C, gave way to a fine, cream colored solid. After 30 minutes, the solid was collected, washed with methylene chloride (2 x 10 ml), and dried *in vacuo* to give 9 (228 mg) as a cream colored powder. IR (Nujol)$\vartheta$ max 1767, 1691, 1620, 1290 $cm^{-1}$; UV (10:1 dioxane-water) $\lambda$ max 316 nm ($\epsilon$12,500), 271 (11,300).

Step C: Preparation of (5R,6S)-6[1(R)-hydroxyethyl]-2-[(1,3-dimethyl-4-imidazolium)methylthio]-carbapen-2-em-3-carboxylate (10)

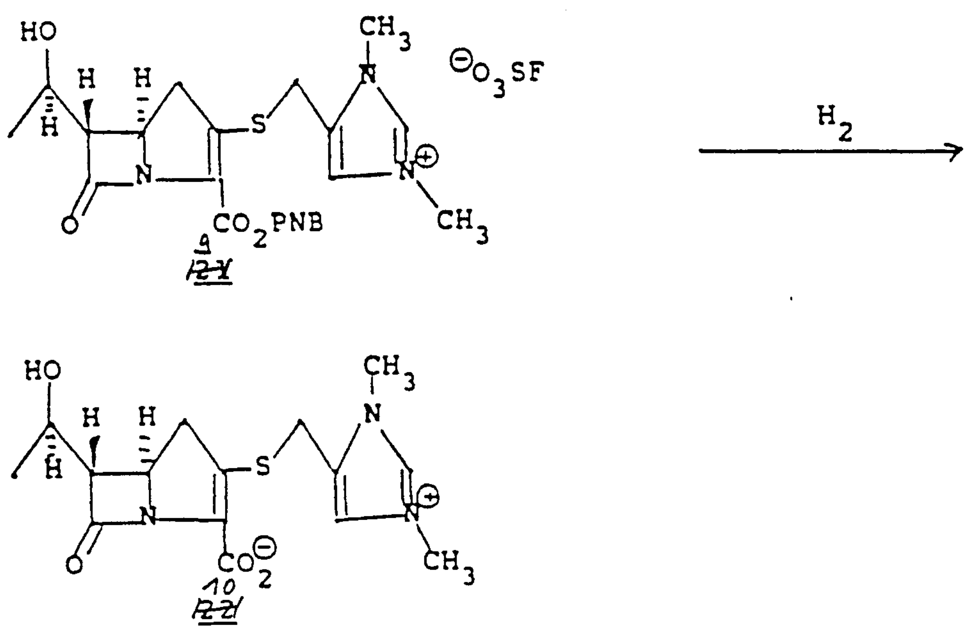


9 ~~21~~

10 ~~22~~

The imidazolium salt 9 (221 mg, 0.386 mmol) was taken up in a mixture of n-butanol (20 ml), ethyl acetate (10 ml), water (20 ml), and 0.5M pH 6.8 N-methylmorpholine-hydrochloric acid buffer (10 ml), treated with 20% palladium hydroxide on carbon (100 mg), and hydrogenated at 3,10 bar (45 psi) for one hour. The mixture was filtered through a celite pad to remove the catalyst which was washed with additional water. The aqueous portion of the filtrate was washed three times with methylene chloride, concentrated in

vacuo to ca. 3 ml, and loaded onto a column of Dowex®50W-X4 resin (sodium form, 0,074 - 0,037 mm (200-400 mesh). 2.5 x 34 cm) which was eluted with de-ionized water in a cold room at 400 drop fractions every 5.1 minutes. The product containing fractions (23-32, 242 ml) were located by uv, concentrated in vacuo, and lyophilized to yield the title compound 10 (69 mg) as a white, amorphous solid. IR (Nujol) $\vartheta$ max 3400 (br), 1750, 1590 $cm^{-1}$; uv (0.05M pH 7.0 MOPS) $\lambda$ max 297 nm (96% $NH_2OH$ extinguished, $\epsilon$ ext. 7,900): NMR ($D_2O$)$\delta$ 1.28 (d, J=6.4Hz, C H $_3$CHOH), 3.09 (dd, J=8.6 and 17.5Hz, CHC H aHb), 3.24 (dd, J=9.5 and 17.4Hz, CHCHaC H b), 3.42 (dd, J=2.6 and 6.0Hz, H6), 3.85 (s, $NCH_3$), 3.86 (s, $NCH_3$), 4.07, 4.21 (ABq, J=15.5Hz, $SCH_2$), 4.18 (m, H5), 4.23 (pentet, J=6.4Hz, $CH_3$C H OH), 7.43 (brs, imidazole-H), 8.66 (brs. imidazole-H).

EXAMPLE 3

Utilizing the procedures of Examples 1 and 2 the following compounds are prepared:

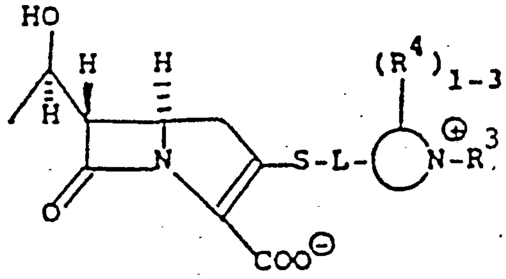

| Compound No. | L | 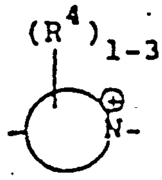 | $R^3$ |
|---|---|---|---|
| 1 | $-CH_2-$ | 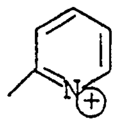 | $CH_2CO_2H$ |
| 2 | " | " | $CH_2\overset{\overset{O}{\uparrow}}{P}(OH)OCH_3$ |
| 3 | " | " | $CH_2SO_3H$ |
| 4 | " | " | 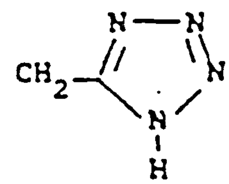 |
| 5 | " | 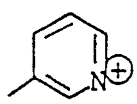 | $CH_2CO_2H$ |
| 6 | " | " | $CH_2\overset{\overset{O}{\uparrow}}{P}(OH)OCH_3$ |
| 7 | " | " | $CH_2SO_3H$ |
| 8 | " | 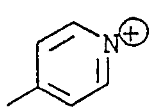 | $CH_2CO_2H$ |
| 9 | " | " | $CH_2\overset{\overset{O}{\uparrow}}{P}(OH)OCH_3$ |
| 10 | " | " | $CH_2SO_3H$ |

| | | | |
|---|---|---|---|
| 11 | $-CH_2-$ | 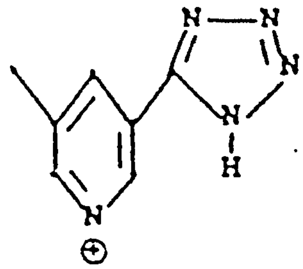 | $CH_3$ |
| 12 | " | 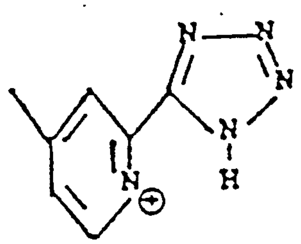 | " |
| 13 | " | 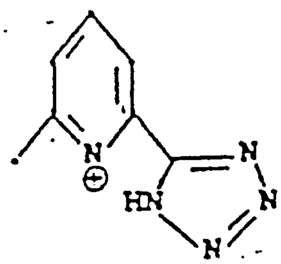 | " |
| 14 | " | 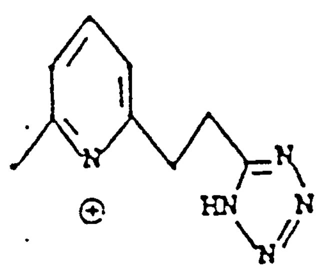 | " |
| 15 | bond | 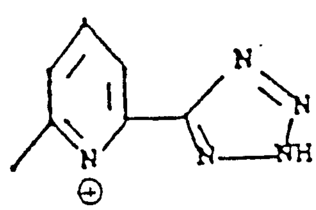 | " |

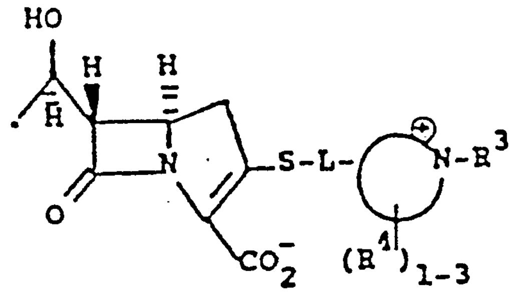

| Compound No. | L | 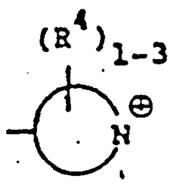 | $R^3$ | $R^4$ |
|---|---|---|---|---|
| 1 | $-CH_2-$ | 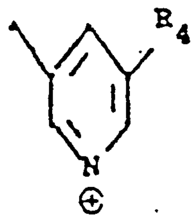 | $CH_3$ | $CO_2H$ |
| 2 | " | " | " | $SO_3H$ |
| 3 | " | " | " | $CH_2CO_2H$ |
| 4 | " | " | " | 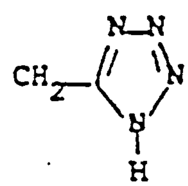 |
| 5 | " | " | " | $CH_2CH_2CO_2H$ |
| 6 | " | " | " | $CH_2SO_3H$ |
| 7 | " | " | " | 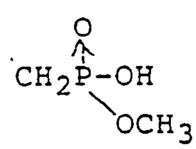 |
| 8 | " | " | " | $CH_2CH_2SO_3H$ |
| 9 | " | 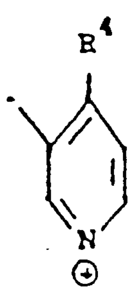 | " | $CO_2H$ |

| | | | | |
|---|---|---|---|---|
| 10 | " | " | " | $SO_3H$ |
| 11 | " | " | " | $CH_2CO_2H$ |
| 12 | " | " | " | 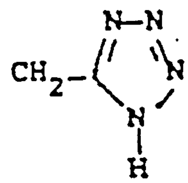 |
| 13 | " | " | " | $CH_2CH_2CO_2H$ |
| 14 | " | " | " | $CH_2SO_3H$ |
| 15 | " | " | " | 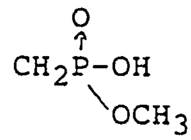 |
| 16 | " | " | " | $CH_2CH_2SO_3H$ |
| 17 | " | 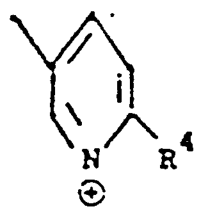 | " | $CO_2H$ |
| 18 | " | " | " | $SO_3H$ |
| 19 | " | " | " | $CH_2CO_2H$ |
| 20 | " | " | " | 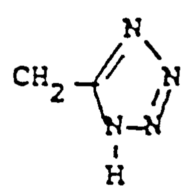 |
| 21 | " | " | " | $CH_2CH_2CO_2H$ |
| 22 | " | " | " | $CH_2SO_3H$ |

| | | | | |
|---|---|---|---|---|
| 23 | " | " | " | 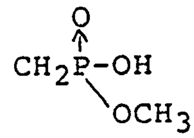 |
| 24 | " | " | " | $CH_2CH_2SO_3H$ |
| 25 | " | " | " | 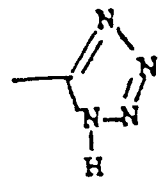 |
| 26 | " | 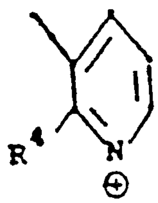 | " | $CO_2H$ |
| 27 | " | " | " | $SO_3H$ |
| 28 | " | " | " | $CH_2CO_2H$ |
| 29 | " | " | " | 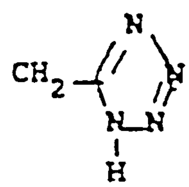 |
| 30 | " | " | " | $CH_2CH_2CO_2H$ |
| 31 | " | " | " | $CH_2SO_3H$ |
| 32 | " | " | " | 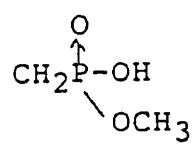 |
| 33 | " | " | " | $CH_2CH_2SO_3H$ |

| | | | | |
|---|---|---|---|---|
| 34 | " | " | " | 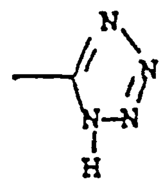 |
| 35 | " | 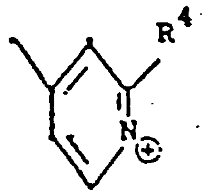 | " | $CO_2H$ |
| 36 | " | " | " | $SO_3H$ |
| 37 | " | " | " | $CH_2CO_2H$ |
| 38 | " | " | " | 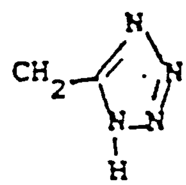 |
| 39 | " | " | " | $CH_2CH_2CO_2H$ |
| 40 | " | " | " | $CH_2SO_3H$ |
| 41 | " | " | " | 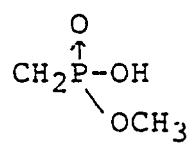 |
| 42 | " | " | " | $CH_2CH_2SO_3H$ |
| 43 | " | " | " | 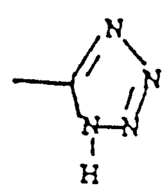 |
| 44 | " | 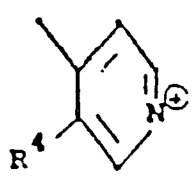 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 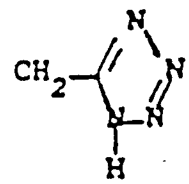 |
| 48 | " | " | " | $CH_2CH_2CO_2H$ |
| 49 | " | " | " | $CH_2SO_3H$ |
| 50 | " | " | " | 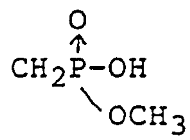 |
| 51 | " | " | " | $CH_2CH_2SO_3H$ |
| 52 | " | " | " | 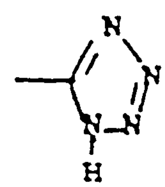 |
| 53 | " | 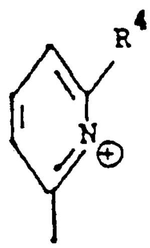 | " | $CO_2H$ |
| 54 | " | " | " | $SO_3H$ |
| 55 | " | " | " | $CH_2CO_2H$ |
| 56 | " | " | " | 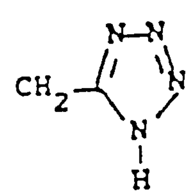 |

| | | | | |
|---|---|---|---|---|
| 57 | " | " | " | $CH_2CH_2CO_2H$ |
| 58 | " | " | " | $CH_2SO_3H$ |
| 59 | " | " | " | 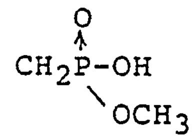 |
| 60 | " | " | " | $CH_2CH_2SO_3H$ |
| 61 | " | 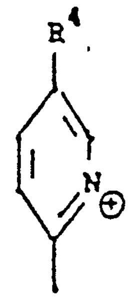 | " | $CO_2H$ |
| 62 | " | " | " | $SO_3H$ |
| 63 | " | " | " | $CH_2CO_2H$ |
| 64 | " | " | " | 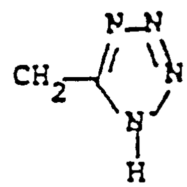 |
| 65 | " | " | " | $CH_2CH_2CO_2H$ |
| 66 | " | " | " | $CH_2SO_3H$ |
| 67 | " | " | " | 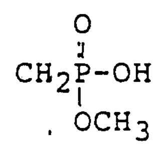 |
| 68 | " | " | " | $CH_2CH_2SO_3H$ |
| 69 | " | 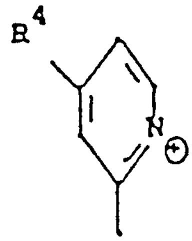 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 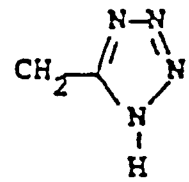 |
| 73 | " | " | " | $CH_2CH_2CO_2H$ |
| 74 | " | " | " | $CH_2SO_3H$ |
| 75 | " | " | " | 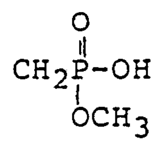 |
| 76 | " | " | " | $CH_2CH_2SO_3H$ |
| 77 | " | 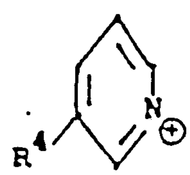 | " | $CO_2H$ |
| 78 | " | " | " | $SO_3H$ |
| 79 | " | " | " | $CH_2CO_2H$ |
| 80 | " | " | " | 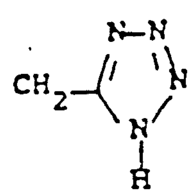 |
| 81 | " | " | " | $CH_2CH_2CO_2H$ |
| 82 | " | " | " | $CH_2SO_3H$ |

| | | | | |
|---|---|---|---|---|
| 83 | " | " | " | 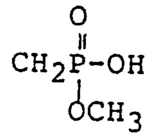 |
| 84 | " | " | " | $CH_2CH_2SO_3H$ |

## Claims

1. A compound having the formula:

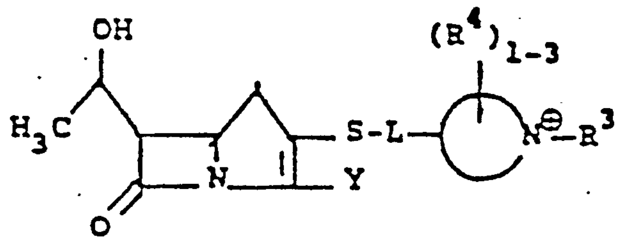

I

wherein:

L is a covalent bond or a bridging group selected from $-(CH_2)_{1-4}S-$; $-(CH_2)_{1-4}-O-$; $-(CH_2)_{1-4}-X''-(CH_2)_{1-4}$ where X" is O, or S; substituted or unsubstituted $C_1$-$C_4$ straight, $C_1$-$C_6$ branched alkyl or $C_3$-$C_7$ cycloalkyl groups wherein the substituents are selected from $C_1$-$C_6$ alkyl, O-$C_1$-$C_6$ alkyl, S-$C_1$-$C_6$ alkyl, halo, OH, $CF_3$, CN, $NK_2$, NH($C_1$-$C_6$ alkyl), N($C_1$-$C_6$ alkyl$)_2$, $CO_2H$,

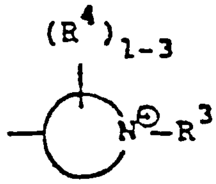 is a pyridinium group

$CONH_2$, CONH($C_1$-$C_6$ alkyl), and CON($C_1$-$C_6$ alkyl$)_2$;

wherein at least one of $R^3$ and $R^4$ is:

an acidic side-chain of the structure $-(CH_2)_n-X(CH_2)_m-Y'-A$ which in the case of $R^4$ may also be simply -A where:

n = 0-4

m = 0-4 -A is a member selected from the group consisting essentially of carboxy($CO_2H$), phosphono[$P=O(OH)_2$], alkylphosphono {$P=O(OH)[O(C_1$-$C_4$-alkyl)]}, alkylphosphinyl [P=O-(OH) $C_1$-$C_4$ alkyl)], substituted phosphoramido [P=O(OH)NH($C_1$-$C_4$ alkyl) and P=O(OH)-$NHR^x$] sulfino ($SO_2H$), sulfo ($SO_3H$), 5-tetra-zolyl ($CN_4H$), arylsulfonamido ($SO_2NHR^x$) and acylsulfonamides represented by the structures $CONHSO_2$($C_1$-$C_4$ alkyl), $CONHSO_2N$($C_1$-$C_4$ alkyl$)_2$, $SO_2NHCO$($C_1$-$C_4$ alkyl) and $SO_2NHCOR^x$ wherein $R^x$ is a heteroaryl group containing from 5 to 11 ring atoms of which up to five are heteroatoms; x = $CHR^s$, CH=CH, phenylene (-$C_6H_4$-), NH, N($C_1$-$C_4$ alkyl), O, S, S=O, C=O, $SO_2$, $SO_2NH$, $CO_2$, CONH, $OCO_2$, OC=O,

NHC=O; $R^s$ = H, O($C_1$-$C_4$ alkyl), $NH_2$, NH($C_1$-$C_4$ alkyl), N($C_1$-$C_4$ alkyl)$_2$, CN, $CONH_2$, CON-($C_1$-$C_4$ alkyl)$_2$, $CO_2H$, $SO_2NH_2$, $SO_2NH$($C_1$-$C_4$ alkyl);
$Y^1$ = single bond, NH, N($C_1$-$C_4$ alkyl), O, S;
wherein $R^3$ optionally is:

1) an unsubstituted or substituted $C_1$-$C_6$ alkyl radical;
2) an unsubstituted or substituted $C_1$-$C_6$ alkenyl radical;
3) an unsubstituted or substituted $C_1$-$C_6$ alkynyl radical;
4) a $C_3$-$C_7$ cycloalkyl radical in which the ring is substituted or unsubstituted and one or more atoms may be replaced by a heteroatom;
5) a $C_3$-$C_7$ cycloalkyl methyl radical in which the ring may be substituted and one or more atoms may be replaced by a heteroatom;
6) an unsubstituted or substituted $C_5$-$C_7$ cycloalkenyl radical;
7) an unsubstituted or substituted bivalent $C_2$-$C_6$ alkylidene radical, optionally interrupted by a heteroatom, and joined to the heteroarylium group to form a ring which is carbocyclic or in which one or more atoms is replaced by a heteroatom. The new ring may contain one or more double bonds;
8) an unsubstituted or substituted phenyl radical;
9) an unsubstituted or substituted phenyl ($C_1$-$C_4$ alkyl) radical;
10) a cyano ($C_1$-$C_4$ alkyl) radical;
11) a carbamoyl ($C_1$-$C_4$ alkyl) radical; 12) a hydroxy ($C_1$-$C_4$ alkyl) radical; or
13) an amino ($C_1$-$C_4$ alkyl) radical in which the nitrogen atom is unsubstituted or substituted with one to three $C_1$-$C_4$ alkyl groups; wherein the substituents in the above definitions of $R^3$ are independently selected from the group consisting of the definitions of $R^4$ set out below:

and wherein $R^4$ optionally is independently selected from:

a) a trifluoromethyl group;
b) a halogen atom;
c) an unsubstituted $C_1$-$C_4$ alkoxyl radical;
d) a hydroxy group;
e) an unsubstituted ($C_1$-$C_6$ alkyl) carbonoxy radical;
f) a carbamoyloxy radical which is unsubstituted or substituted on nitrogen with one or two $C_1$-$C_4$ alkyl groups;
g) a $C_1$-$C_6$ alkylthio radical, $C_1$-$C_6$ alkylsulfinyl radical or $C_1$-$C_6$ alkylsulfonyl radical, each of which is unsubstituted on the alkyl group;
h) a sulfamoyl group which is unsubstituted or substituted on nitrogen by one or two $C_1$-$C_4$ alkyl groups;
i) an amino group;
j) a mono ($C_1$-$C_4$ alkyl) amino or di($C_1$-$C_4$ alkyl)amino group, each of which is unsubstituted on the alkyl group;
k) a formlamino group;
l) an unsubstituted ($C_1$-$C_6$ alkyl)carbonylamino radical;
m) a ($C_1$-$C_4$ alkoxy) carbonylamino radical;
n) a ureido group in which the terminal nitrogen is unsubstituted or substituted with one or two $C_1$-$C_4$ alkyl groups;
o) a ($C_1$-$C_6$ alkyl) sulfonamido group;
p) a cyano group;
q) a formyl or acetalized formyl radical;
r) an unsubstituted ($C_1$-$C_6$ alkyl)carbonyl radical wherein the carbonyl is free or acetalized
s) an unsubstituted phenylcarbonyl radical;
t) a hydroximinomethyl radical in which the oxygen or carbon atom is optionally substituted by a $C_1$-$C_4$ alkyl group;
u) a ($C_1$-$C_6$ alkoxy)carbonyl radical;
v) a carbamoyl radical which is unsubstituted or substituted on nitrogen by one or two $C_1$-$C_4$ alkyl groups;
w) an N-hydroxycarbamoyl or N($C_1$-$C_4$ alkoxy)carbamoyl radical in which the nitrogen atom may be additionally substituted by a $C_1$-$C_4$ alkyl group;
x) a thiocarbamoyl group; where $R^5$, $R^6$ and $R^7$ are independently hydrogen, $C_1$-$C_4$ alkyl or wherein two of the alkyl groups together form a $C_2$-$C_6$alkylidene radical optionally interrupted by a heteroatom and joined together to form a ring:

aa) a guanidinyl group where $R^5$ in y) above is $NR^8R^9$ and $R^8$ and $R^9$ are as defined for $R^5$ through $R^7$ above.
ab) hydrogen;
ac) an unsubstituted $C_1$-$C_6$ alkyl radical;
ad) an unsubstituted $C_1$-$C_6$ alkenyl radical;
ae) an unsubstituted $C_1$-$C_6$ alkynyl radical;
af) a $C_3$-$C_7$ cycloalkyl radical in which the ring is unsubstituted and one or more atoms may be replaced by a heteroatom;
ag) $C_3$-$C_7$ cycloalkyl methyl radical in which one or more atoms may be replaced by a heteroatom;
ah) an unsubstituted $C_5$-$C_7$ cycloalkenyl radical;
ai) an unsubstituted phenyl radical; and
aj) an unsubstituted phenyl ($C_1$-$C_4$ alkyl) radical; and wherein heteroatom in the above definitions of A, $R^3$ and $R^4$ means nitrogen, oxygen or sulfur, independently selected where more than one heteroatom is involved; and
Y is selected from: i) COOH or a pharmaceutically acceptable. ester or salt thereof,
ii) $COOR^1$ wherein $R^1$ is a removable carboxy protecting group.
iii) COOM wherein M is an alkali metal, or
iv) $COO^{\ominus}$; provided that when Y is other than iv) a counterion $z^{\ominus}$ is provided;

**2.** A compound of Claim 1 wherein L is substituted or unsubstituted branched or linear $C_1$-$C_4$ alkyl.

**3.** A compound of Claim 2 wherein $R^3$ is an unsubstituted or substituted $C_1$-$C_4$ alkyl group.

**4.** A compound of Claim 3 wherein L is $-CH_2-$, $-CH(CH_3)-$ or $-(CH_2)_2-$.

**5.** A compound of Claim 1 wherein the compound is a member selected from the group consisting of:

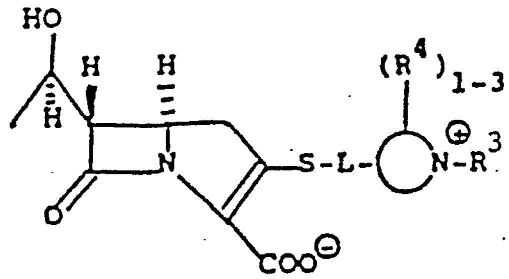

| Compound No. | L | 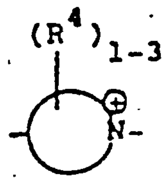 | $R^3$ |
|---|---|---|---|
| 1 | $-CH_2-$ | 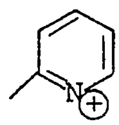 | $CH_2CO_2H$ |
| 2 | " | " | $CH_2\overset{O \atop \uparrow}{P}(OH)OCH_3$ |
| 3 | " | " | $CH_2SO_3H$ |
| 4 | " | " | 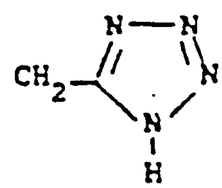 |
| 5 | " | 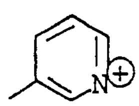 | $CH_2CO_2H$ |
| 6 | " | " | $CH_2\overset{O \atop \uparrow}{P}(OH)OCH_3$ |
| 7 | " | " | $CH_2SO_3H$ |
| 8 | " | 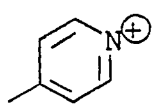 | $CH_2CO_2H$ |
| 9 | " | " | $CH_2\overset{O \atop \uparrow}{P}(OH)OCH_3$ |
| 10 | " | " | $CH_2SO_3H$ |

| | | | |
|---|---|---|---|
| 11 | $-CH_2-$ | 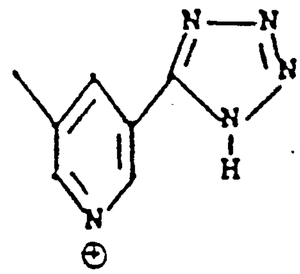 | $CH_3$ |
| 12 | " | 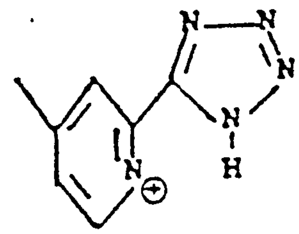 | " |
| 13 | " | 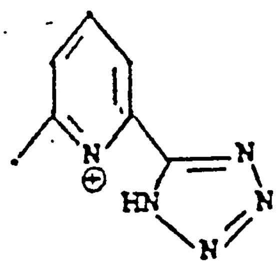 | " |
| 14 | " | 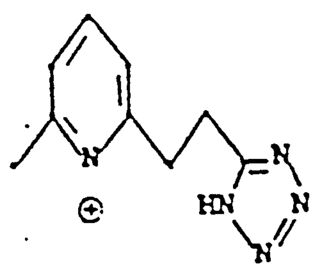 | " |
| 15 | bond | 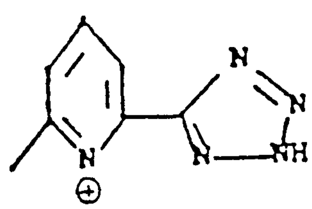 | " |

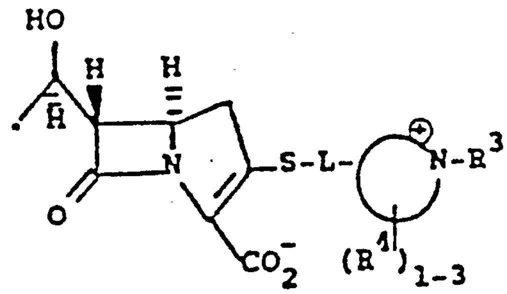

| Compound No. | L | 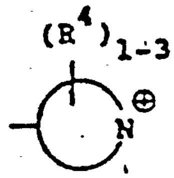 | $R^3$ | $R^4$ |
|---|---|---|---|---|
| 1 | $-CH_2-$ | 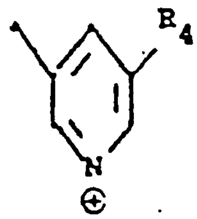 | $CH_3$ | $CO_2H$ |
| 2 | " | " | " | $SO_3H$ |
| 3 | " | " | " | $CH_2CO_2H$ |
| 4 | " | " | " | 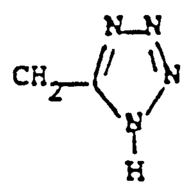 |
| 5 | " | " | " | $CH_2CH_2CO_2H$ |
| 6 | " | " | " | $CH_2SO_3H$ |
| 7 | " | " | " | 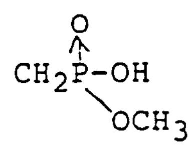 |
| 8 | " | " | " | $CH_2CH_2SO_3H$ |
| 9 | " | 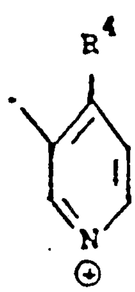 | " | $CO_2H$ |

| | | | | |
|---|---|---|---|---|
| 10 | " | " | " | $SO_3H$ |
| 11 | " | " | " | $CH_2CO_2H$ |
| 12 | " | " | " | 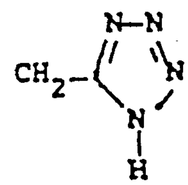 |
| 13 | " | " | " | $CH_2CH_2CO_2H$ |
| 14 | " | " | " | $CH_2SO_3H$ |
| 15 | " | " | " | 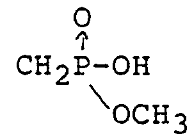 |
| 16 | " | " | " | $CH_2CH_2SO_3H$ |
| 17 | " | 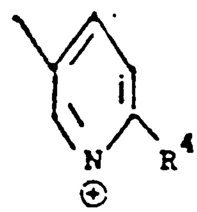 | " | $CO_2H$ |
| 18 | " | " | " | $SO_3H$ |
| 19 | " | " | " | $CH_2CO_2H$ |
| 20 | " | " | " | 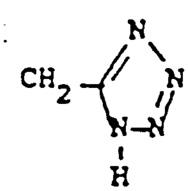 |
| 21 | " | " | " | $CH_2CH_2CO_2H$ |
| 22 | " | " | " | $CH_2SO_3H$ |

| | | | | |
|---|---|---|---|---|
| 23 | " | " | " | 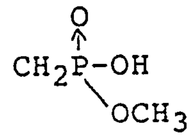 |
| 24 | " | " | " | $CH_2CH_2SO_3H$ |
| 25 | " | " | " | 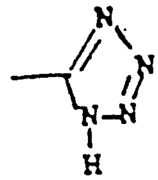 |
| 26 | " | 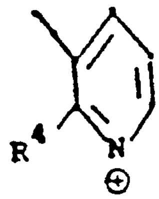 | " | $CO_2H$ |
| 27 | " | " | " | $SO_3H$ |
| 28 | " | " | " | $CH_2CO_2H$ |
| 29 | " | " | " | 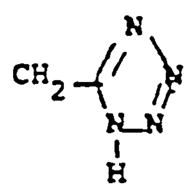 |
| 30 | " | " | " | $CH_2CH_2CO_2H$ |
| 31 | " | " | " | $CH_2SO_3H$ |
| 32 | " | " | " | 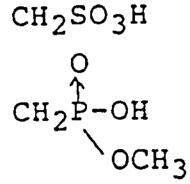 |
| 33 | " | " | " | $CH_2CH_2SO_3H$ |

| | | | | |
|---|---|---|---|---|
| 34 | " | " | " | 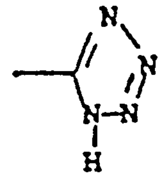 |
| 35 | " | 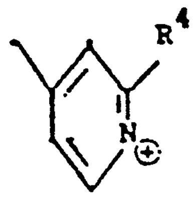 | " | $CO_2H$ |
| 36 | " | " | " | $SO_3H$ |
| 37 | " | " | " | $CH_2CO_2H$ |
| 38 | " | " | " | 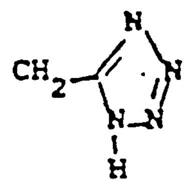 |
| 39 | " | " | " | $CH_2CH_2CO_2H$ |
| 40 | " | " | " | $CH_2SO_3H$ |
| 41 | " | " | " | $CH_2\overset{\overset{O}{\uparrow}}{P}(-OH)\backslash OCH_3$ |
| 42 | " | " | " | $CH_2CH_2SO_3H$ |
| 43 | " | " | " | 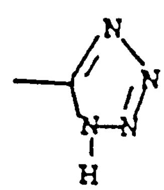 |
| 44 | " | 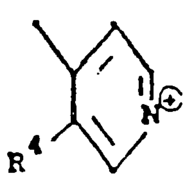 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 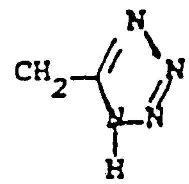 |
| 48 | " | " | " | $CH_2CH_2CO_2H$ |
| 49 | " | " | " | $CH_2SO_3H$ |
| 50 | " | " | " | 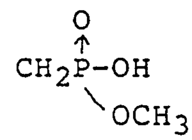 |
| 51 | " | " | " | $CH_2CH_2SO_3H$ |
| 52 | " | " | " | 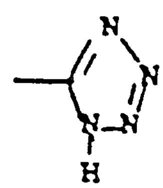 |
| 53 | " | 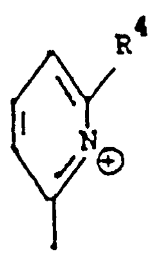 | " | $CO_2H$ |
| 54 | " | " | " | $SO_3H$ |
| 55 | " | " | " | $CH_2CO_2H$ |
| 56 | " | " | " | 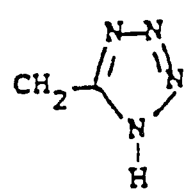 |

| | | | | |
|---|---|---|---|---|
| 57 | " | " | " | $CH_2CH_2CO_2H$ |
| 58 | " | " | " | $CH_2SO_3H$ |
| 59 | " | " | " | 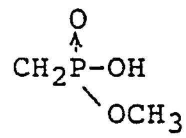 |
| 60 | " | " | " | $CH_2CH_2SO_3H$ |
| 61 | " | 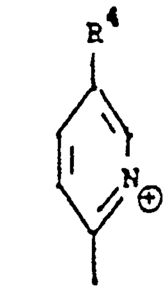 | " | $CO_2H$ |
| 62 | " | " | " | $SO_3H$ |
| 63 | " | " | " | $CH_2CO_2H$ |
| 64 | " | " | " | 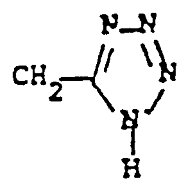 |
| 65 | " | " | " | $CH_2CH_2CO_2H$ |
| 66 | " | " | " | $CH_2SO_3H$ |
| 67 | " | " | " | 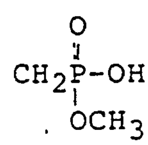 |
| 68 | " | " | " | $CH_2CH_2SO_3H$ |
| 69 | " | 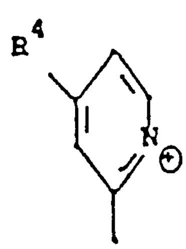 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 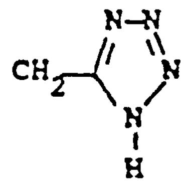 |
| 73 | " | " | " | $CH_2CH_2CO_2H$ |
| 74 | " | " | " | $CH_2SO_3H$ |
| 75 | " | " | " | $CH_2\underset{\overset{\mid}{OCH_3}}{\overset{\overset{O}{\|}}{P}}-OH$ |
| 76 | " | " | " | $CH_2CH_2SO_3H$ |
| 77 | " | 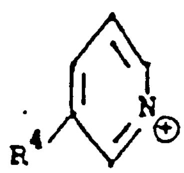 | " | $CO_2H$ |
| 78 | " | " | " | $SO_3H$ |
| 79 | " | " | " | $CH_2CO_2H$ |
| 80 | " | " | " | 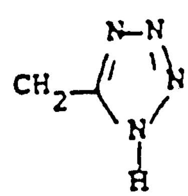 |
| 81 | " | " | " | $CH_2CH_2CO_2H$ |
| 82 | " | " | " | $CH_2SO_3H$ |

| | | | | |
|---|---|---|---|---|
| 83 | " | " | " | 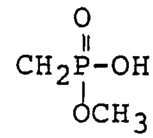 |
| 84 | " | " | " | $CH_2CH_2SO_3H$ |

6. The combination of a compound of Claim 1 and a DHP inhibitor.

7. A combination of Claim 6 wherein the DHP inhibitor is 7-(L-2-amino-2-carboxyethylthio)-2-(2,2-dimethylcyclopropanecarboxamide)-2-heptenoic acid.

8. A pharmaceutical composition for antibiotic use comprising an antibacterially effective amount of a compound of Claim 1, an inhibitorily effective amount of a DHP inhibitor, and, optionally, a pharmaceutically acceptable carrier.

9. A pharmaceutical composition according to Claim 8 wherein the DHP inhibitor is 7-(L-2-amino-2-carboxyethylthio)-2-(2,2-dimethylcyclopropanecarboxamide)-2-heptenoic acid.

10. A compound of Claim 1 of the structure:

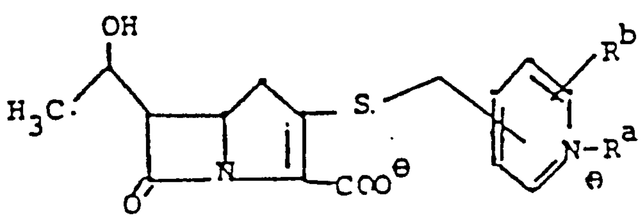

wherein
$R^a$ is $C_{1-4}$ alkyl; or an acidic side-chain of the structure $-(CH_2)n-X-(CH_2)m-Y'-A$ where:
n = 0-4
m = 0-4
X = $CHR^s$, CH=CH, phenylene ($-C_6H_4-$), NH, N($C_1$-$C_4$ alkyl), O, S, S=O, C=O, $SO_2$, $SO_2NH$, $CO_2$, CONH, $OCO_2$, OC=O, NHC=O;

$R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S;

A is as defined in claim 1;

Rb is hydrogen; cyano; or an acidic side-chain of the structure -A or $(CH_2)_n$-$X(CH_2)_mY'$-A where:
n = 0-4
m = 0-4
x = $CHR^s$, CH=CH, phenylene ($-C_6H_4-$), NH, N($C_1$-$C_4$ alkyl), O, S, S=O, C=O, $SO_2$, $SO_2NH$, $CO_2$, CONH, $OCO_2$, OC=O, NHC=O; $R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S;

A is as defined in Claim 1; provided that Ra or Rb must be an acidic side-chain.

**11.** A pharmaceutical composition for antibiotic use comprising an antibacterially effective amount of a o compound of Claim 10, an inhibitorily effective amount of a DHP inhibitor, and, optionally, a pharmaceutical carrier. CLAIMS FOR THE CONTRACTING STATE: AT

**1.** A process for preparing a compound having the formula:

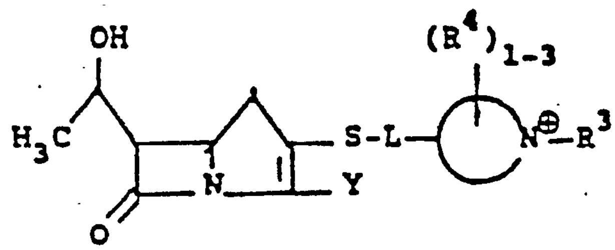

I

wherein:

L is a covalent bond or a bridging group selected from $-(CH_2)_{1-4}S-$; $-(CH_2)_{1-4}-O-$; $-(CH_2)_{1-4}-X''-(CH_2)_{1-4}$ where X" is O, or S; substituted or unsubstituted $C_1$-$C_4$ straight, $C_1$-$C_6$ branched alkyl $C_3$-$C_7$ cycloalkyl groups wherein the substituents are selected from $C_1$-$C_6$ alkyl, O-$C_1$-$C_6$ alkyl, S-$C_1$-$C_6$ alkyl, halo, OH, $CF_3$, CN, $NH_2$, NH($C_1$-$C_6$ alkyl), N($C_1$-$C_6$ alkyl$)_2$, $CO_2H$,

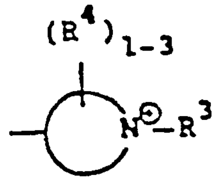 is a pyridinium group

$CONH_2$, CONH($C_1$-$C_6$ alkyl), and CON($C_1$-$C_6$ alkyl$)_2$;
wherein at least one of $R^3$ and $R^4$ is:
an acidic side-chain of the structure $(CH_2)_n$-X-$(CH_2)_m$-Y'-A which in the case of $R^4$ may also be simply -A where:
n = 0-4
m = 0-4 -A is a member selected from the group consisting essentially of carboxy($CO_2H$), phosphono[$P=O(OH)_2$], alkylphosphono {P=O(OH)[O($C_1$-$C_4$-alkyl)]}, alkylphosphinyl [P=O-(OH) ($C_1$-$C_4$ alkyl)], substituted phosphoramido [P=O(OH)NH($C_1$-$C_4$ alkyl) and P=O(OH)-$NHR^x$], sulfino ($SO_2H$), sulfo ($SO_3H$), 5-tetra-zolyl ($CN_4H$), arylsulfonamido ($SO_2NHR^x$) and acylsulfonamides represented by the structures $CONHSO_2$($C_1$-$C_4$ alkyl), $CONHSO_2N$($C_1$-$C_4$ alkyl$)_2$, $SO_2NHCO$($C_1$-$C_4$ alkyl) and $SO_2NHCOR^x$ wherein $R^x$ is a heteroaryl group containing from 5 to 11 ring atoms of which up to five are heteroatoms; X = $CHR^s$ CH=CH, phenylene ($-C_6H_4-$), NH, N($C_1$-$C_4$ alkyl), O, S, S=O, C=O, $SO_2$, $SO_2NH$, $CO_2$, CONH, $OCO_2$, OC=O, NHC=O; $R^s$ = H, O($C_1$-$C_4$ alkyl), $NH_2$, NH($C_1$-$C_4$ alkyl), N($C_1$-$C_4$ alkyl$)_2$, CN, $CONH_2$, CON-($C_1$-$C_4$ alkyl$)_2$, $CO_2H$, $SO_2NH_2$, $SO_2NH$($C_1$-$C_4$ alkyl);
Y' = single bond, NH, N($C_1$-$C_4$ alkyl), O, S; wherein $R^3$ optionally is:

1) an unsubstituted or substituted $C_1-C_6$ alkyl radical;
2) an unsubstituted or substituted $C_1-C_6$ alkenyl radical;
3) an unsubstituted or substituted $C_1-C_6$ alkynyl radical;
4) a $C_3-C_7$ cycloalkyl radical in which the ring is substituted or unsubstituted and one or more atoms may be replaced by a heteroatom;
5) a $C_3-C_7$ cycloalkyl methyl radical in which the ring may be substituted and one or more atoms may be replaced by a heteroatom;
6) an unsubstituted or substituted $C_5-C_7$ cycloalkenyl radical;
7) an unsubstituted or substituted bivalent $C_2-C_6$ alklidene radical, optionally interrupted by a heteroatom, and joined to the heteroarylium group to form a ring which is carbocyclic or in which one

or more atoms is replaced by a heteroatom. The new ring may contain one or more double bonds;
8) an unsubstituted or substituted phenyl radical;
9) an unsubstituted or substituted phenyl ($C_1$-$C_4$ alkyl) radical;
10) a cyano ($C_1$-$C_4$ alkyl) radical;
11) a carbamoyl ($C_1$-$C_4$ alkyl) radical;
12) a hydroxy ($C_1$-$C_4$ alkyl) radical; or
13) an amino ($C_1$-$C_4$ alkyl) radical in which the nitrogen atom is unsubstituted or substituted with one to three $C_1$-$C_4$ alkyl groups; wherein the substituents in the above definitions of $R^3$ are independently selected from the group consisting of the definitions of $R^4$ set out below; and wherein $R^4$ optionally is independently selected from:
a) a trifluoromethyl group;
b) a halogen atom;
c) an unsubstituted $C_1$-$C_4$ alkoxyl radical;
d) a hydroxy group;
e) an unsubstituted ($C_1$-$C_6$ alkyl) carbonyloxy radical;
f) a carbamoyloxy radical which is unsubstituted or substituted
on nitrogen with one or two $C_1$-$C_4$ alkyl groups;
g) a $C_1$-$C_6$ alkylthio radical, $C_1$-$C_6$ alkylsulfinyl radical or $C_1$-$C_6$ alkylsulfonyl radical, each of which is unsubstituted on the alkyl group;
h) a sulfamoyl group which is unsubstituted or substituted on nitrogen by one or two $C_1$-$C_4$ alkyl groups;
i) an amino group;
j) a mono ($C_1$-$C_4$ alkyl) amino or di($C_1$-$C_4$ alkyl)amino group, each of which is unsubstituted on the alkyl group;
k) a formylamino group;
l) an unsubstituted ($C_1$-$C_6$ alkyl)carbonylamino radical;
m) a ($C_1$-$C_4$ alkoxy) carbonylamino radical;
n) a ureido group in which the terminal nitrogen is unsubstituted with or substituted with one or two $C_1$-$C_4$ alkyl groups;
o) a ($C_1$-$C_6$ alkyl) sulfonamido group;
p) a cyano group;
q) a formyl or acetalized formyl radical;
r) an unsubstituted ($C_1$-$C_6$ alkyl)carbonyl radical wherein the carbonyl is free or acetalized;
s)an unsubstituted phenylcarbonyl radical;
t) a hydroximinomethyl radical in which the oxygen or carbon atom is optionally substituted by a $C_1$-$C_4$ alkyl group;
u) a ($C_1$-$C_6$ alkoxy)carbonyl radical;
v) a carbamoyl radical which is unsubstituted or substituted on nitrogen by one or two $C_1$-$C_4$ alkyl groups;
w) an N-hydroxycarbamoyl or N($C_1$-$C_4$ alkoxy)carbamoyl radical in which the nitrogen atom may be additionally substituted by a $C_1$-$C_4$ alkyl group;
x) a thiocarbamoyl group;

y) an amidino group 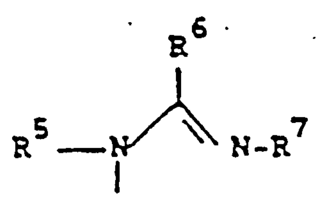 ,

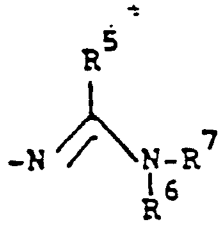

z) a carboxamidino group 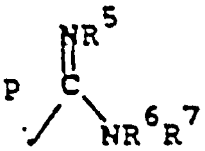 , where $R^5$, $R^6$ and $R^7$ are as defined above;

aa) a guanidinyl group where $R^6$ in y) above is $NR^8R^9$ and $R^8$ and $R^9$ are as defined for $R^5$ through $R^7$ above.

ab) hydrogen;

ac) an unsubstituted $C_1$-$C_6$ alkyl radical;

ad) an unsubstituted $C_1$-$C_6$ alkenyl radical;

ae) an unsubstituted $C_1$-$C_6$ alkynyl radical;

af) a $C_3$-$C_7$ cycloalkyl radical in which the ring is unsubstituted and one or more atoms may be replaced by a heteroatom;

ag) a $C_3$-$C_7$ cycloalkyl methyl radical in which one or more atoms may be replaced by a heteroatom;

ah) an unsubstituted $C_5$-$C_7$ cycloalkenyl radical;

ai) an unsubstituted phenyl radical; and

aj) an unsubstituted phenyl ($C_1$-$C_4$ alkyl) radical; and wherein heteroatom in the above definitions of A, $R^3$ and $R^4$ means nitrogen, oxygen or sulfur, independently selected where more than one heteroatom is involved; and

Y is selected from :
i) COOH or a pharmaceutically acceptable ester or salt thereof.
ii) COOR ' wherein R' is a removable carboxy protecting group,
iii) COOM wherein M is an alkali metal, or
iv) $COO^{\ominus}$;provided that when Y is other than iv) a counterion $Z^{\ominus}$ is provided:

which comprises

A. reaction of a compound of formula:

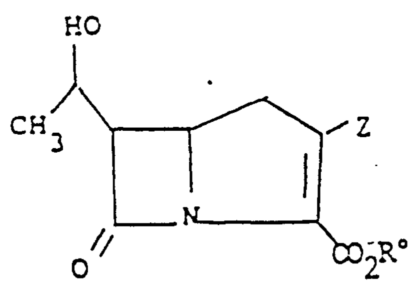

with a compound of formula:

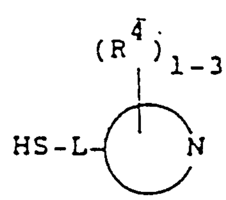

in the presence of a base,
treating the obtained compound of formula:

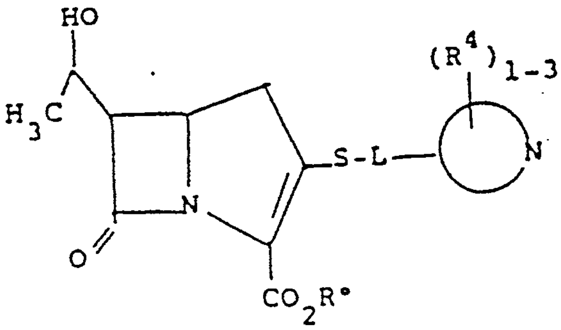 with $R^3X'$ or $R^3O^{\oplus}X'^{\ominus}$

and optionally deblocking the obtained compound of formula:

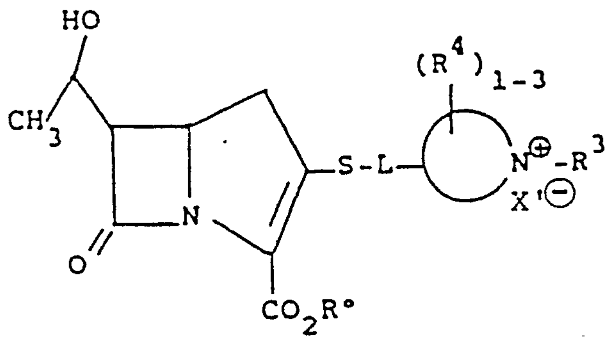

to obtain:

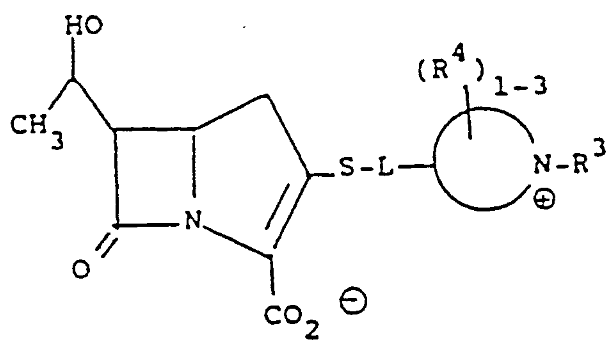

I

wherein Z and X' are leaving groups or X' is an anionic group; and R° is a protecting group and $R^3$, L,

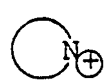

and $R^4$ are as defined above.

B. reaction of a compound of formula: with a compound of formula:

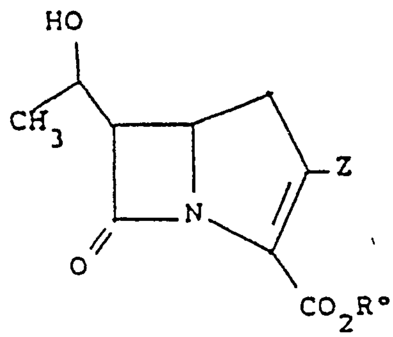

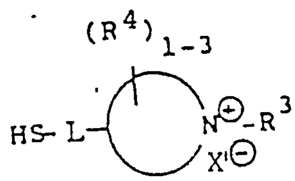

in the presence of a base and optionally deblocking the obtained compound of formula:

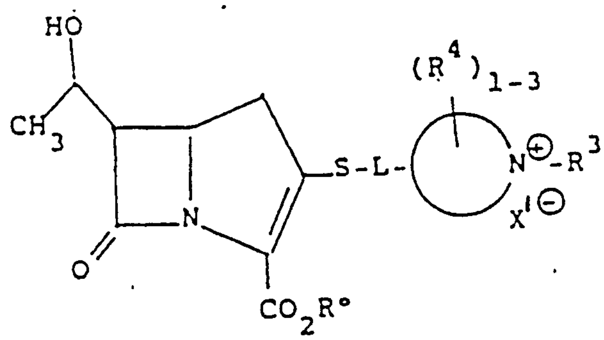

to obtain:

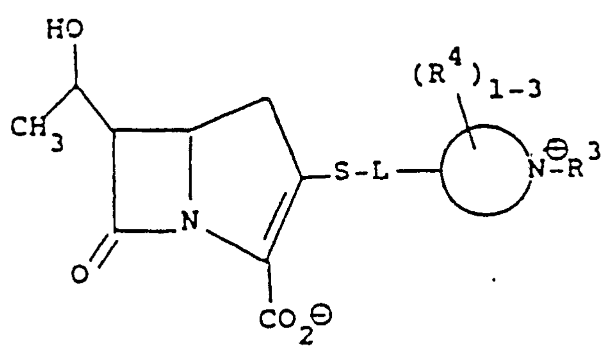

wherein Z, X', L, R°, $R^3$,

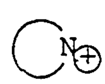

and $R^4$ are as defined above,

C. reaction of a compound of formula:

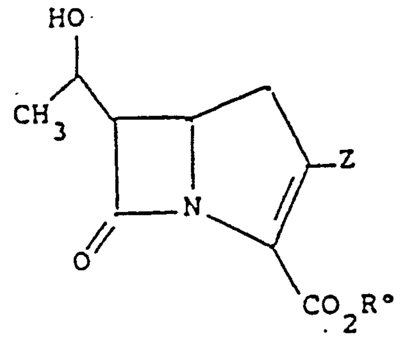

with NaHS

treating the obtained compound of formula:

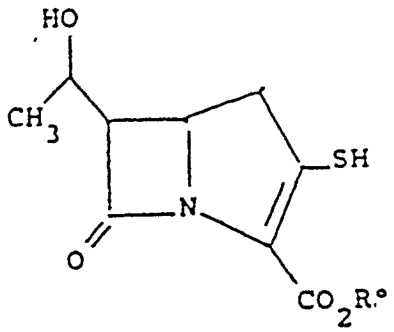

with

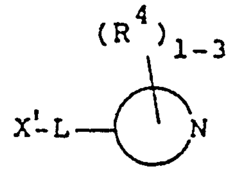

treating the obtained compound of formula:

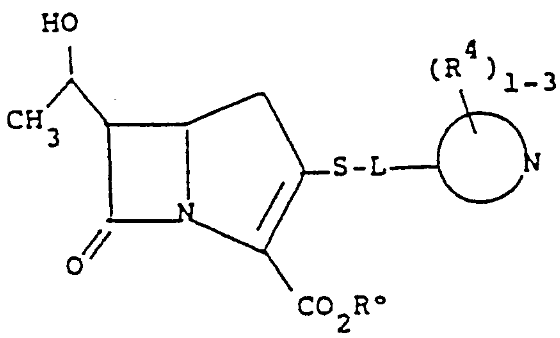

with $R^3X'$ or $R^3O^{\oplus}X'^{\ominus}$

and optionally deblocking the obtained compound of formula:

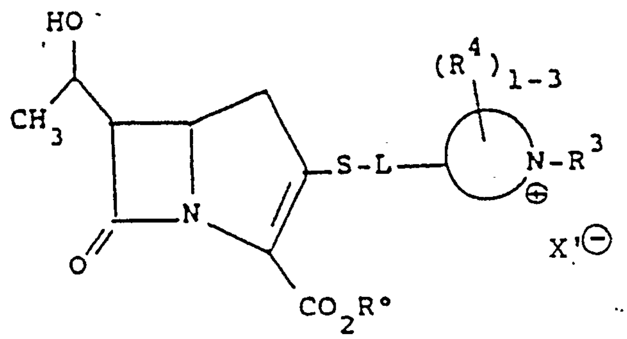

to obtain:

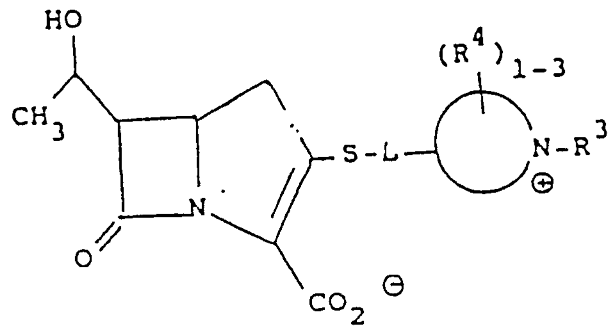

I

wherein Z, X', L, R°, $R^3$,

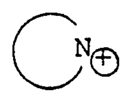

and $R^4$ are as previously defined,

D. reaction of a compound of formula:

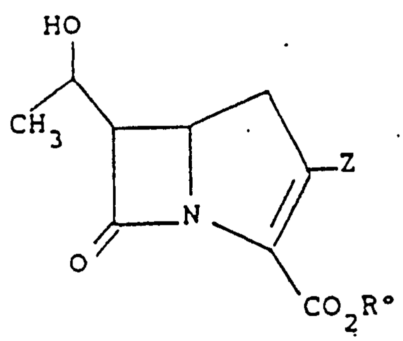

with NaHS ,

treating the obtained compound of formula:

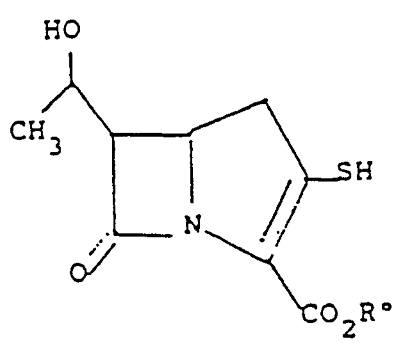

with

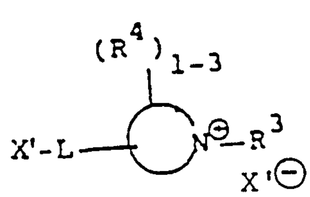

in the presence of a base, and optionally deblocking the obtained compound of formula:

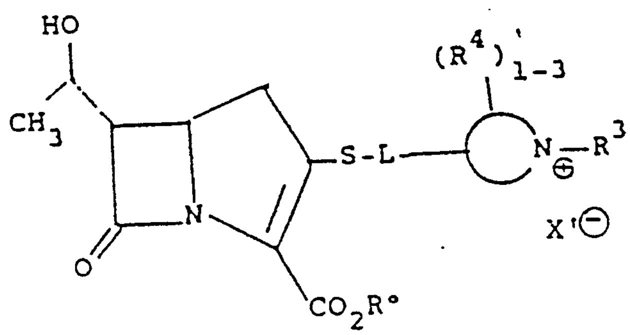

to obtain:

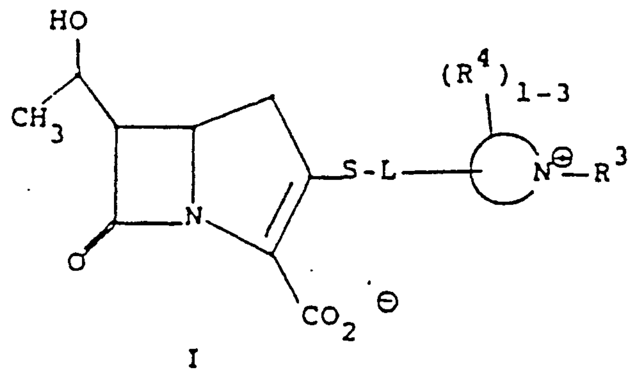

I

wherein Z, X', R°, $R^3$,L,

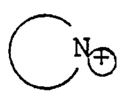

and $R^4$ are as previously defined.

2. A process of Claim 1 for preparing a compound wherein L is substituted or unsubstituted branched or linear $C_1$-$C_4$ alkyl.

3. A process of Claim 2 for preparing a compound wherein $R^3$ is an unsubstituted or substituted $C_1$-$C_4$ alkyl group.

4. A process of Claim 3 for preparing a compound wherein L is
$-CH_2-$,
$-CH(CH_3)-$ or $-(CH_2)_2-$.

5. A process of Claim 1 for preparing a compound wherein the compound is a member selected from the group consisting of:

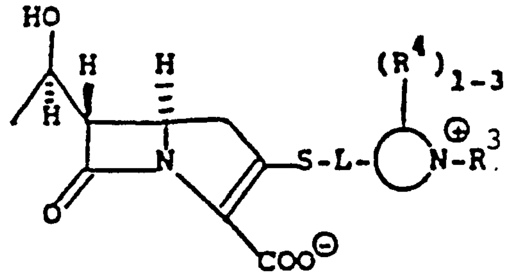

| Compound No. | L | 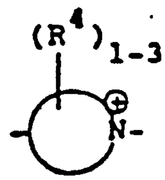 | $R^3$ |
|---|---|---|---|
| 1 | $-CH_2-$ | 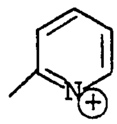 | $CH_2CO_2H$ |
| 2 | " | " | $CH_2\overset{O}{\uparrow}{P}(OH)OCH_3$ |
| 3 | " | " | $CH_2SO_3H$ |
| 4 | " | " | 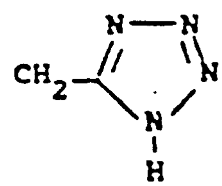 |
| 5 | " | 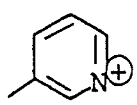 | $CH_2CO_2H$ |
| 6 | " | " | $CH_2\overset{O}{\uparrow}{P}(OH)OCH_3$ |
| 7 | " | " | $CH_2SO_3H$ |
| 8 | " | 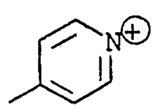 | $CH_2CO_2H$ |
| 9 | " | " | $CH_2\overset{O}{\uparrow}{P}(OH)OCH_3$ |
| 10 | " | " | $CH_2SO_3H$ |

| | | | |
|---|---|---|---|
| 11 | $-CH_2-$ | 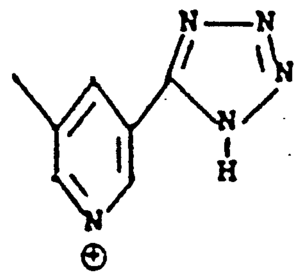 | $CH_3$ |
| 12 | " | 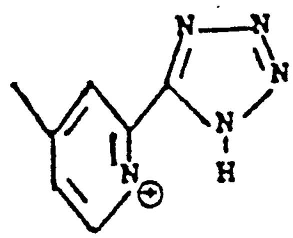 | " |
| 13 | " | 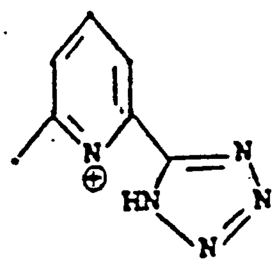 | " |
| 14 | " | 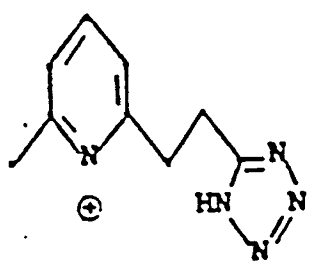 | " |
| 15 | bond | 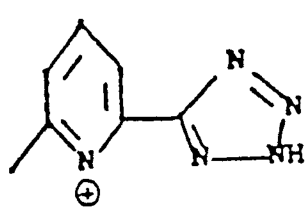 | " |

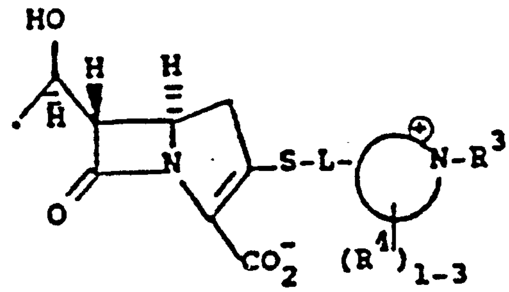

| Compound No. | L | 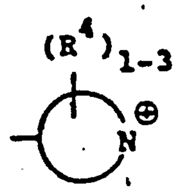 | $R^3$ | $R^4$ |
|---|---|---|---|---|
| 1 | $-CH_2-$ | 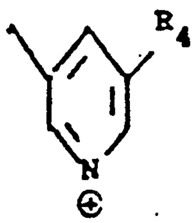 | $CH_3$ | $CO_2H$ |
| 2 | " | " | " | $SO_3H$ |
| 3 | " | " | " | $CH_2CO_2H$ |
| 4 | " | " | " | 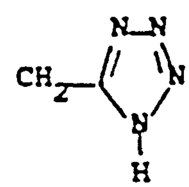 |
| 5 | " | " | " | $CH_2CH_2CO_2H$ |
| 6 | " | " | " | $CH_2SO_3H$ |
| 7 | " | " | " | 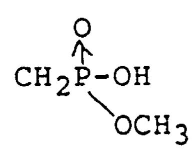 |
| 8 | " | " | " | $CH_2CH_2SO_3H$ |
| 9 | " | 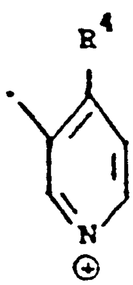 | " | $CO_2H$ |

| | | | | |
|---|---|---|---|---|
| 10 | " | " | " | $SO_3H$ |
| 11 | " | " | " | $CH_2CO_2H$ |
| 12 | " | " | " | 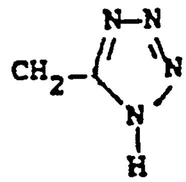 |
| 13 | " | " | " | $CH_2CH_2CO_2H$ |
| 14 | " | " | " | $CH_2SO_3H$ |
| 15 | " | " | " | 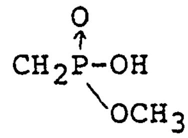 |
| 16 | " | " | " | $CH_2CH_2SO_3H$ |
| 17 | " | 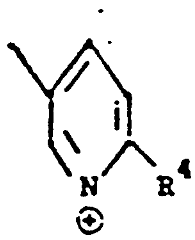 | " | $CO_2H$ |
| 18 | " | " | " | $SO_3H$ |
| 19 | " | " | " | $CH_2CO_2H$ |
| 20 | " | " | " | 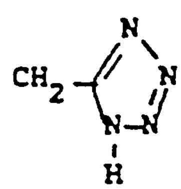 |
| 21 | " | " | " | $CH_2CH_2CO_2H$ |
| 22 | " | " | " | $CH_2SO_3H$ |

| | | | | |
|---|---|---|---|---|
| 23 | " | " | " | 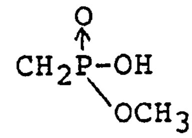 |
| 24 | " | " | " | $CH_2CH_2SO_3H$ |
| 25 | " | " | " | 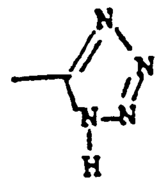 |
| 26 | " | 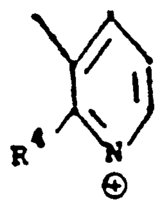 | " | $CO_2H$ |
| 27 | " | " | " | $SO_3H$ |
| 28 | " | " | " | $CH_2CO_2H$ |
| 29 | " | " | " | 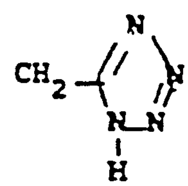 |
| 30 | " | " | " | $CH_2CH_2CO_2H$ |
| 31 | " | " | " | $CH_2SO_3H$ |
| 32 | " | " | " | 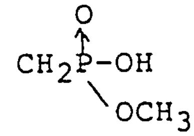 |
| 33 | " | " | " | $CH_2CH_2SO_3H$ |

| | | | | |
|---|---|---|---|---|
| 34 | " | " | " | 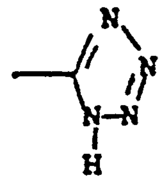 |
| 35 | " | 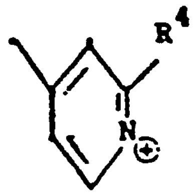 | " | $CO_2H$ |
| 36 | " | " | " | $SO_3H$ |
| 37 | " | " | " | $CH_2CO_2H$ |
| 38 | " | " | " | 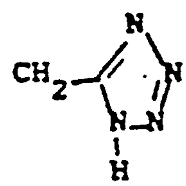 |
| 39 | " | " | " | $CH_2CH_2CO_2H$ |
| 40 | " | " | " | $CH_2SO_3H$ |
| 41 | " | " | " | $CH_2\overset{O}{\overset{\uparrow}{P}}(OH)OCH_3$ |
| 42 | " | " | " | $CH_2CH_2SO_3H$ |
| 43 | " | " | " | 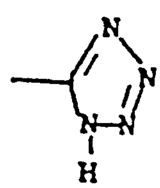 |
| 44 | " | 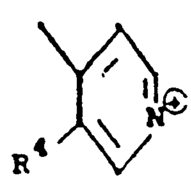 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 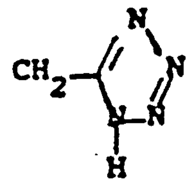 |
| 48 | " | " | " | $CH_2CH_2CO_2H$ |
| 49 | " | " | " | $CH_2SO_3H$ |
| 50 | " | " | " | 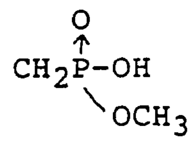 |
| 51 | " | " | " | $CH_2CH_2SO_3H$ |
| 52 | " | " | " | 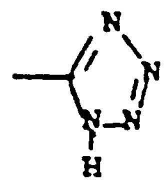 |
| 53 | " | 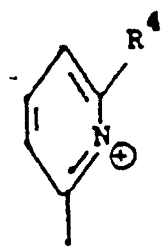 | " | $CO_2H$ |
| 54 | " | " | " | $SO_3H$ |
| 55 | " | " | " | $CH_2CO_2H$ |
| 56 | " | " | " | 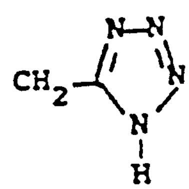 |

| | | | | |
|---|---|---|---|---|
| 57 | " | " | " | $CH_2CH_2CO_2H$ |
| 58 | " | " | " | $CH_2SO_3H$ |
| 59 | " | " | " | 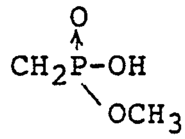 |
| 60 | " | " | " | $CH_2CH_2SO_3H$ |
| 61 | " | 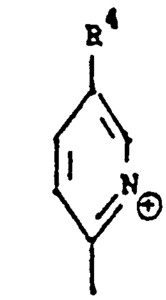 | " | $CO_2H$ |
| 62 | " | " | " | $SO_3H$ |
| 63 | " | " | " | $CH_2CO_2H$ |
| 64 | " | " | " | 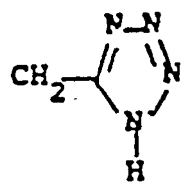 |
| 65 | " | " | " | $CH_2CH_2CO_2H$ |
| 66 | " | " | " | $CH_2SO_3H$ |
| 67 | " | " | " | 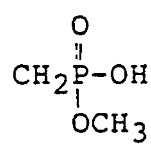 |
| 68 | " | " | " | $CH_2CH_2SO_3H$ |
| 69 | " | 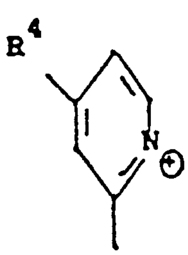 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 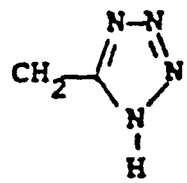 |
| 73 | " | " | " | $CH_2CH_2CO_2H$ |
| 74 | " | " | " | $CH_2SO_3H$ |
| 75 | " | " | " | $CH_2\overset{O}{\overset{\parallel}{P}}(OCH_3)-OH$ |
| 76 | " | " | " | $CH_2CH_2SO_3H$ |
| 77 | " | 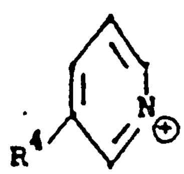 | " | $CO_2H$ |
| 78 | " | " | " | $SO_3H$ |
| 79 | " | " | " | $CH_2CO_2H$ |
| 80 | " | " | " | 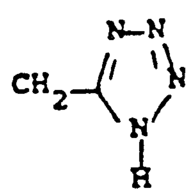 |
| 81 | " | " | " | $CH_2CH_2CO_2H$ |
| 82 | " | " | " | $CH_2SO_3H$ |

| | | | | |
|---|---|---|---|---|
| 83 | " | " | " | 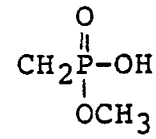 |
| 84 | " | " | " | $CH_2CH_2SO_3H$ |

6. A process according to Claim 1 wherein the compound obtained is additionally combined with a DHP inhibitor.

7. The process according to Claim 6 wherein the DHP inhibitor is 7-(L-2-amino-2-carboxyethylthio)-2-(2,2-dimethylcyclopropanecarboxamide)-2-heptenoic acid.

8. A process according to Claim 1 wherein an antibacterially effective amount of the compound obtained is additionally combined with an inhibitorily effective amount of a DHP inhibitor, and, optionally, a pharmaceutically acceptable carrier.

9. The process according to Claim 8 wherein the DHP inhibitor is 7-(L-2-amino-2-carboxyethylthio)-2-(2,2-dimethylcyclopropanecarboxamide)-2-heptenoic acid.

10. A process of Claim 1 for preparing a compound of the structure:

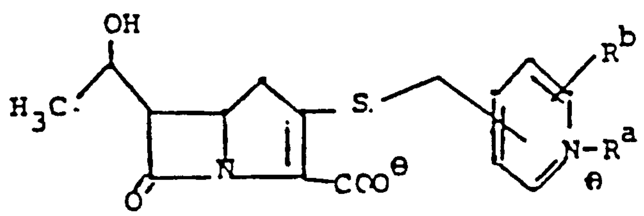

wherein
$R^a$ is $C_{1-4}$ alkyl; or an acidic side-chain of the structure $(CH_2)_n$-X-$(CH_2)_m$-Y'-A where:
n = 0-4
m = 0-4
X = $CHR^s$, CH=CH, phenylene (-$C_6H_4$-), NH, N($C_1$-$C_4$ alkyl), O, S, S=O, C=O, $SO_2$, $SO_2NH$, $CO_2$, CONH, $OCO_2$, OC=O, NHC=O; $R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S;

A is as defined in Claim 1;

$R^b$ is hydrogen; cyano; or an acidic side-chain of the structure -A or -$(CH_2)_n$-X-$(CH_2)_m$-Y'-A where:
n = 0-4
m = 0-4
X = $CHR^s$, CH=CH, phenylene (-$C_6H_4$-), NH, N($C_1$-$C_4$ alkyl), O, S, S=O, C=O, $SO_2$, $SO_2NH$, $CO_2$, CONH, $OCO_2$, OC=O, NHC=O; $R^s$ = H, O($C_1$-$C_4$ alkyl), $NH_2$, NH($C_1$-$C_4$ alkyl), N($C_1$-$C_4$ alkyl)$_2$, CN, $CONH_2$, CON($C_1$-$C_4$ alkyl)$_2$, $CO_2H$, $SO_2NH_2$, $SO_2NH$($C_1$-$C_4$ alkyl);

Y' = single bond, NH, N($C_1$-$C_4$ alkyl), O, S;

A is as defined in claim 1; provided that $R^a$ or $R^b$ must be an acidic side-chain.

11. A process according to Claim 10 wherein an antibacterially effective amount of the compound obtained is additionally combined with an inhibitorily effective amount of a DHP inhibitor, and, optionally, a pharmaceutical carrier.

## Revendications

1. Composé répondant à la for

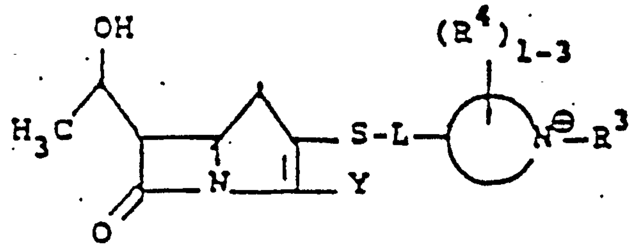

I

dans laquelle:

L est une liaison covalente ou un groupe de pontage choisi parmi $-(CH_2)_{1-4}-S-$ ; $-(CH_2)_{1-4}-O-$; $-CH_2)_{1-4}-X''-(CH_2)_{1-4}-$ où X" est O ou S des groupes alkyles droits en $C_1-C_4$, ramifiés en $C_1-C_6$ ou cycloalkyles en $C_3-C_7$ substitués ou non -dont les substituants sont choisis parmi alkyle en $C_1-C_6$, O-alkyle en $C_1-C_6$, S-alkyle en $C_1-C_6$, halogéno, OH, $CF_3$, CH, $NH_2$, NH-(alkyle en $C_1-C_6$), N(alkyle en $C_1-C_6)_2$, $CO_2H$, $CONH_2$, CONH(alkyle en $C_1-C_6$) et CON-(alkyle en $C_1-C_6-)_2$;

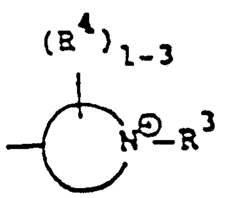 est un groupe pyridinium

où au moins un de $R^3$ et $R^4$ est:

une chaîne latérale acide de structure $-(CH_2)_n-X-(CH_2)_m-Y'-A$, auquel cas $R^4$ peut également être simplement -A où :

n = 0-4

m = 0-4

-A est un composant du groupe constitué essentiellement par carboxy ($CO_2H$), phosphono [$P=O(OH)_2$], alkylphosphono {$P=O(OH)$[O-(alkyle en $C_1-C_4$)]}, alkylphosphinyle [P=O-(OH)(alkyle en $C_1-C_4$)], phosphoramido substitué [P=O(OH)NH(alkyle en $C_1-C_4$) et P=O-(OH)$NHR^X$], sulfino ($SO_2H$), sulfo ($SO_3H$), 5-tétrazolyle ($CN_4H$), arylsulfonamido ($SO_2NHR^X$) et les acylsulfonamides représentés par les structures $CONHSO_2$(alkyle en $C_1-C_4$), CONH-$SO_2N$(alkyle en $C_1-C_4)_2$, $SO_2NHCO$(alkyle en $C_1-C_4$) et $SO_2NHCOR^X$ où $R^X$ est un groupe hétéroaryle contenant de 5 à 11 atomes dont jusqu'à 5 sont des hétéroatomes; X est $CHR^s$, CH=CH, phénylène ($-C_6H_4-$), NH, N(alkyle en $C_1-C_4$), O,S, S=O, C=O, $SO_2$, $SO_2NH$, $CO_2$, CONH, $OCO_2$, OC=O, NHC=O ; $R^s$ est H, O(alkyle en $C_1-C_4$), $NH_2$, NH(alkyle en $C_1-C_4$), N(alkyle en $C_1-C_4)_2$, CN, $CONH_2$, CON(alkyle en $C_1-C_4)_2$, $CO_2H$, $SO_2NH_2$, $SO_2NH$(alkyle en $C_1-C_4$);

Y' est une simple liaison, NH, N(alkyle en $C_1-C_4$), O, S ;

où $R^3$ est facultativement :

1) un radical alkyle en $C_1-C_6$ non substitué ou substitué ;

2) un radical alcényle en $C_1-C_6$ non substitué ou substitué

3) un radical alcynyle en $C_1-C_6$ non substitué ou substitué

4) un radical cycloalkyle en $C_3-C_7$ dont le cycle est substitué ou non substitué et un ou plusieurs atomes peuvent être remplacés par un hétéroatome ;

5) un radical (cycloalkyl en $C_3$-$C_7$)méthyle dont le cycle peut être substitué et un ou plusieurs atomes peuvent être remplacés par un hétéroatome ;
6) un radical cycloalcényle en $C_5$-$C_7$ non substitué ou substitué ;
7) un radical alkylidène bivalent en $C_2$-$C_6$ non substitué ou substitué, facultativement interrompu par un hétéroatome et uni au groupe hétéroarylium pour former un cycle qui est carbocyclique ou dans lequel un ou plusieurs atomes sont remplacés par un hétéroatome; le nouveau cycle peut contenir une ou plusieurs doubles liaisons ;
8) un radical phényle non substitué ou substitué
9) un radical phényl(alkyle en $C_1$-$C_4$) non substitué ou substitué;
10) un radical cyano(alkyle en $C_1$-$C_4$);
11) un radical carbamoyl(alkyle en $C_1$-$C_4$) ;
12) un radical hydroxy(alkyle en $C_1$-$C_4$) ; ou
13) un radical amino(alkyle en $C_1$-$C_4$) dans lequel l'atome d'azote est non substitué ou substitué par 1 à 3 groupes alkyles en $C_1$-$C_4$ ; où les substituants dans les définitions ci-dessus de $R^3$ sont indépendamment choisis dans le groupe constitué par les définitions de $R^4$ indiquées ci-après ;
et où $R^4$ facultativement est choisi indépendamment parmi :
a) un groupe trifluorométhyle ;
b) un atome d'halogène ;
c) un radical alcoxyle en $C_1$-$C_4$ non substitué ;
d) un groupe hydroxy ;
e) un radical (alkyl en $C_1$-$C_6$)carbonyloxy non substitué ;
f) un radical carbamoyloxy qui est non substitué ou substitué sur l'azote par un ou deux groupes alkyles en $C_1$-$C_4$ ;
g) un radical alkylthio en $C_1$-$C_6$, un radical alkylsulfinyle en $C_1$-$C_6$ ou un radical alkylsulfonyle en $C_1$-$C_6$, qui sont chacun non substitués sur le groupe alkyle ;
h) un groupe sulfamoyle qui est non substitué ou substitué sur l'azote par un ou deux groupes alkyles en $C_1$-$C_4$ ;
i) un groupe amino ;
j) un groupe mono(alkyl en $C_1$-$C_4$)amino ou di(alkyl en $C_1$-$C_4$) amino, chacun non substitué sur le groupe alkyle ;
k) un groupe formylamino ;
l) un radical (alkyl en $C_1$-$C_6$)carbonylamino non substitué ;
n) un radical (alcoxy en $C_1$-$C_4$)carbonylamino ;
n) un groupe uréido dont l'azote terminal est non substitué ou substitué par un ou deux groupes alkyles en $C_1$-$C_4$ ;
o) un groupe (alkyl en $C_1$-$C_6$)sulfonamido ;
p) un groupe cyano ;
q) un radical formyle ou formyle acétalisé ;
r) un radical (alkyl en $C_1$-$C_6$)carbonyle non substitué dont le carbonyle est libre ou acétalisé .
s) un radical phénylcarbonyle non substitué ;
t) un radical hydroxyiminométhyle dont l'atome d'oxygène ou de carbone est facultativement substitué par un groupe alkyle en $C_1$-$C_4$ ;
u) un radical (alcoxy en $C_1$-$C_6$)carbonyle ;
v) un radical carbamoyle qui est non substitué ou substitué sur l'azote par un ou deux groupes alkyles en $C_1$-$C_4$ ;
w) un radical N-hydroxycarbamoyle ou N(alcoxy en $C_1$-$C_4$) carbamoyle dont l'atome d'azote peut de plus être substitué par un groupe alkyle en $C_1$-$C_4$ ;
x) un groupe thiocarbamoyle ;

y) un groupe amidino 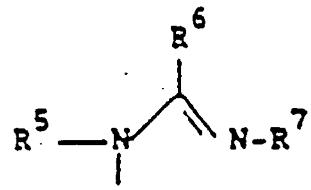

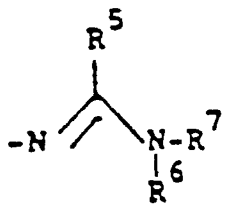

où $R^5$, $R^6$ et $R^7$ sont indépendamment un hydrogène, un alkyle en $C_1$-$C_4$ ou deux des groupes alkyles forment ensemble un radical alylidène en $C_2$-$C_6$ facultativement interrompu par un hétéroatome et réunis pour former un cycle ;

z) un groupe carboxamidino 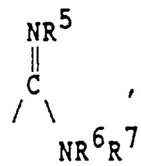 , dans lequel $R^5$, $R^6$ et $R^7$ sont définis comme ci-dessus ;

aa) un groupe guanidinyle où $R^6$ dans Y) ci-dessus est $NR^8R^9$ et $R^8$ et $R^9$ sont définis comme pour $R^5$ à $R^7$ ci-dessus ;
ab) un hydrogène ;
ac) un radical alkyle en $C_1$-$C_6$ non substitué ;
ad) un radical alcényle en $C_1$-$C_6$ non substitué ;
ae) un radical alcynyle en $C_1$-$C_6$ non substitué ;
af) un radical cycloalyle en $C_3$-$C_7$ dont le cycle est non substitué et un ou plusieurs atomes peuvent être remplacés par un hétéroatome ;
ag) un radical (cycloalkyl en $C_3$-$C_7$)méthyle dont un ou plusieurs atomes peuvent être remplacés par un hétéroatome ;
ah) un radical cycloalcényle en $C_5$-$C_7$ non substitué ;
ai) un radical phényle non substitué ; et
aj) un radical phényl (alkyle en $C_1$-$C_4$) non substitué ; et un hétéroatome dans les définitions ci-dessus de A, $R^3$ et $R^4$ est un atome d'azote, d'oxygène ou de soufre choisi indépendamment dans le cas où il y a plusieurs hétéroatomes ; et

Y est choisi parmi : i) COOH ou un ester ou sel pharmaceutiquement acceptable de celui-ci,
ii) COOR' où R' est un groupe carboxy-protecteur éliminable,
iii) COOM où M est un métal alcalin, ou
iv) $COO^{\ominus}$ sous réserve que, lorsque Y est autre que iv), il existe un ion opposé $Z^{\oplus}$.

2. Composé selon la revendication 1, dans lequel L est un alkyle en $C_1$-$C_4$ ramifié ou linéaire substitué ou non substitué.

3. Composé selon la revendication 2, dans lequel $R^3$ est un groupe alkyle en $C_1$-$C_4$ non substitué ou substitué.

4. Composé selon la revendication 3, dans lequel L est $-CH_2-$, $-CH(CH_3)-$ ou $-(CH_2)_2-$.

5. Composé selon la revendication 1 qui appartient au groupe constitué par :

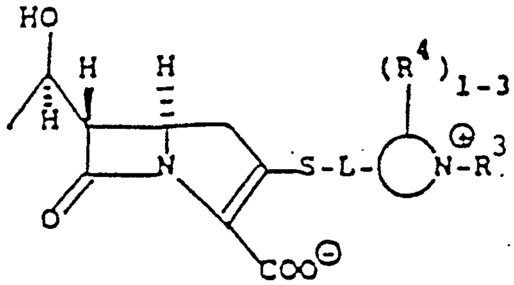

| Composé n° | L | 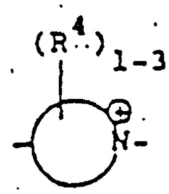 | $R^3$ |
|---|---|---|---|
| 1 | $-CH_2-$ | 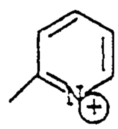 | $CH_2CO_2H$ |
| 2 | " | " | $CH_2\overset{O}{\overset{\uparrow}{P}}(OH)OCH_3$ |
| 3 | " | " | $CH_2SO_3H$ |
| 4 | " | " | 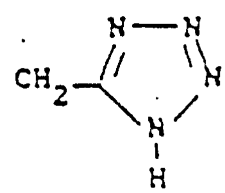 |
| 5 | " | 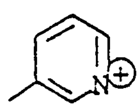 | $CH_2CO_2H$ |
| 6 | " | " | $CH_2\overset{O}{\overset{\uparrow}{P}}(OH)OCH_3$ |
| 7 | " | " | $CH_2SO_3H$ |
| 8 | " | 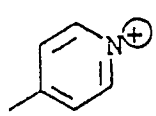 | $CH_2CO_2H$ |
| 9 | " | " | $CH_2\overset{O}{\overset{\uparrow}{P}}(OH)CCH_3$ |
| 10 | " | " | $CH_2SO_3H$ |

| | | | |
|---|---|---|---|
| 11 | $-CH_2-$ | 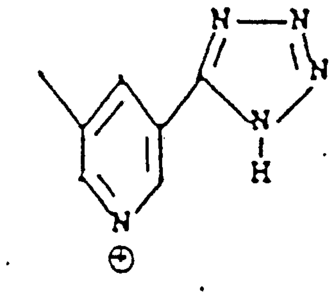 | $CH_3$ |
| 12 | " | 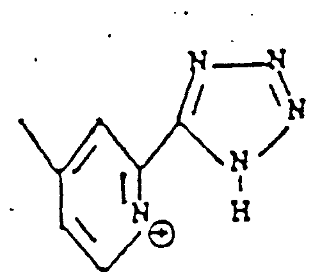 | " |
| 13 | " | 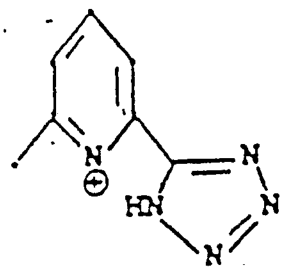 | " |
| 14 | " | 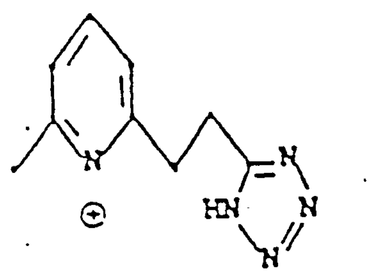 | " |
| 15 | bond | 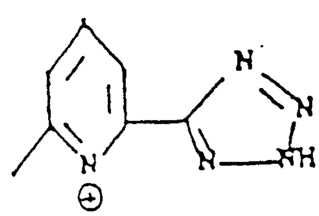 | " |

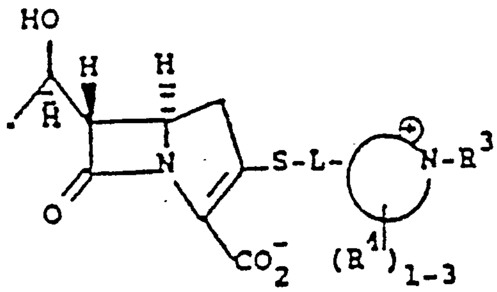

| Composé n° | L | 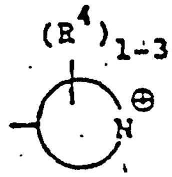 | $R^3$ | $R^4$ |
|---|---|---|---|---|
| 1 | $-CH_2-$ | 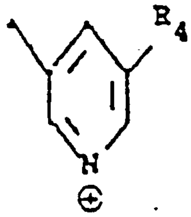 | $CH_3$ | $CO_2H$ |
| 2 | " | " | " | $SO_3H$ |
| 3 | " | " | " | $CH_2CO_2H$ |
| 4 | " | " | " | 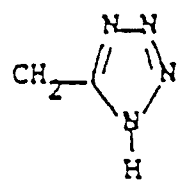 |
| 5 | " | " | " | $CH_2CH_2CO_2H$ |
| 6 | " | " | " | $CH_2SO_3H$ |
| 7 | " | " | " | 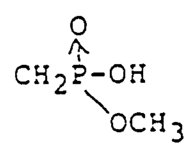 |
| 8 | " | " | " | $CH_2CH_2SO_3H$ |
| 9 | " | 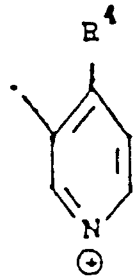 | " | $CO_2H$ |

| | | | | |
|---|---|---|---|---|
| 10 | " | " | " | $SO_3H$ |
| 11 | " | " | " | $CH_2CO_2H$ |
| 12 | " | " | " | 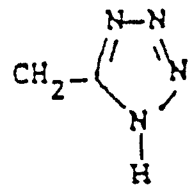 |
| 13 | " | " | " | $CH_2CH_2CO_2H$ |
| 14 | " | " | " | $CH_2SO_3H$ |
| 15 | " | " | " | 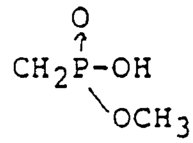 |
| 16 | " | " | " | $CH_2CH_2SO_3H$ |
| 17 | " | 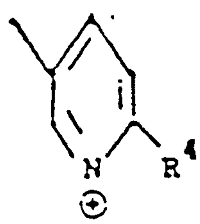 | " | $CO_2H$ |
| 18 | " | " | " | $SO_3H$ |
| 19 | " | " | " | $CH_2CO_2H$ |
| 20 | " | " | " | 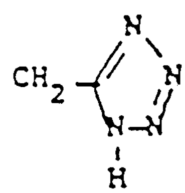 |
| 21 | " | " | " | $CH_2CH_2CO_2H$ |
| 22 | " | " | " | $CH_2SO_3H$ |

| | | | | |
|---|---|---|---|---|
| 23 | " | " | " | 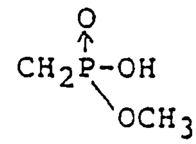 |
| 24 | " | " | " | $CH_2CH_2SO_3H$ |
| 25 | " | " | " | 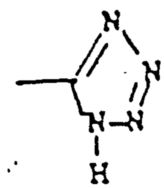 |
| 26 | " | 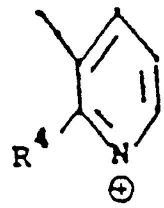 | " | $CO_2H$ |
| 27 | " | " | " | $SO_3H$ |
| 28 | " | " | " | $CH_2CO_2H$ |
| 29 | " | " | " | 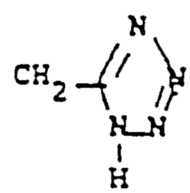 |
| 30 | " | " | " | $CH_2CH_2CO_2H$ |
| 31 | " | " | " | $CH_2SO_3H$ |
| 32 | " | " | " | 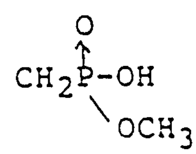 |
| 33 | " | " | " | $CH_2CH_2SO_3H$ |

| | | | | |
|---|---|---|---|---|
| 34 | " | " | " | 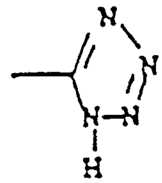 |
| 35 | " | 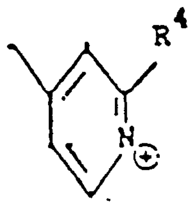 | " | $CO_2H$ |
| 36 | " | " | " | $SO_3H$ |
| 37 | " | " | " | $CH_2CO_2H$ |
| 38 | " | " | " | 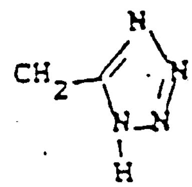 |
| 39 | " | " | " | $CH_2CH_2CO_2H$ |
| 40 | " | " | " | $CH_2SO_3H$ |
| 41 | " | " | " | 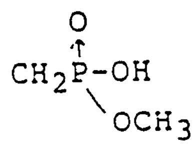 |
| 42 | " | " | " | $CH_2CH_2SO_3H$ |
| 43 | " | " | " | 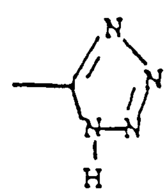 |
| 44 | " | 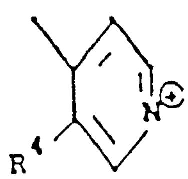 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 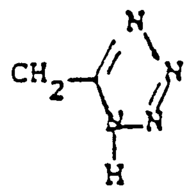 |
| 48 | " | " | " | $CH_2CH_2CO_2H$ |
| 49 | " | " | " | $CH_2SO_3H$ |
| 50 | " | " | " | 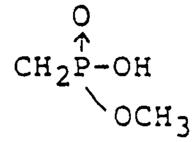 |
| 51 | " | " | " | $CH_2CH_2SO_3H$ |
| 52 | " | " | " | 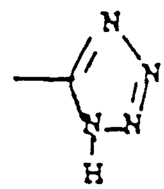 |
| 53 | " | 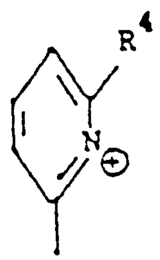 | " | $CO_2H$ |
| 54 | " | " | " | $SO_3H$ |
| 55 | " | " | " | $CH_2CO_2H$ |
| 56 | " | " | " | 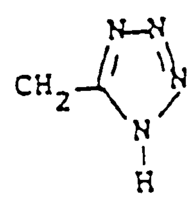 |

| | | | | |
|---|---|---|---|---|
| 57 | " | " | " | $CH_2CH_2CO_2H$ |
| 58 | " | " | " | $CH_2SO_3H$ |
| 59 | " | " | " | 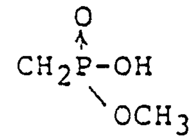 |
| 60 | " | " | " | $CH_2CH_2SO_3H$ |
| 61 | " | 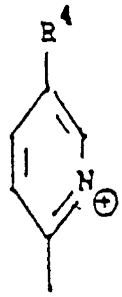 | " | $CO_2H$ |
| 62 | " | " | " | $SO_3H$ |
| 63 | " | " | " | $CH_2CO_2H$ |
| 64 | " | " | " | 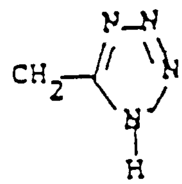 |
| 65 | " | " | " | $CH_2CH_2CO_2H$ |
| 66 | " | " | " | $CH_2SO_3H$ |
| 67 | " | " | " | 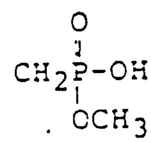 |
| 68 | " | " | " | $CH_2CH_2SO_3H$ |
| 69 | " | 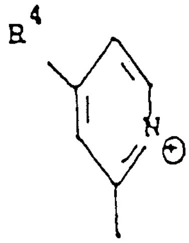 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 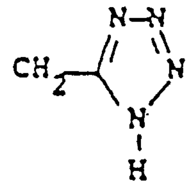 |
| 73 | " | " | " | $CH_2CH_2CO_2H$ |
| 74 | " | " | " | $CH_2SO_3H$ |
| 75 | " | " | " | 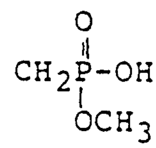 |
| 76 | " | " | " | $CH_2CH_2SO_3H$ |
| 77 | " | 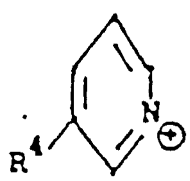 | " | $CO_2H$ |
| 78 | " | " | " | $SO_3H$ |
| 79 | " | " | " | $CH_2CO_2H$ |

| | | | | |
|---|---|---|---|---|
| 80 | " | " | " | 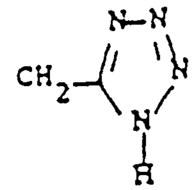 |
| 81 | " | " | " | $CH_2CH_2CO_2H$ |
| 82 | " | " | " | $CH_2SO_3H$ |
| 83 | " | " | " | 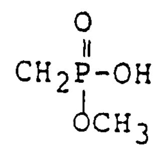 |
| 84 | " | " | " | $CH_2CH_2SO_3H$ |

6. Combinaison d'un composé selon la revendication 1 et d'un inhibiteur de la DHP.

7. Combinaison selon la revendication 6, dans laquelle l'inhibiteur de la DHP est l'acide 7-(L-2-amino-2-carboxyéthylthio)-2-(2,2-diméthylcyclopropanecarboxamide)-2-hepténoique.

8. Composition pharmaceutique utile comme antibiotique comprenant une quantité à effet antibactérien d'un composé selon la revendication 1, une quantité à effet inhibiteur d'un inhibiteur de la DHP et, facultativement, un véhicule pharmaceutiquement acceptable.

9. Composition pharmaceutique selon la revendication 8, dans laquelle l'inhibiteur de la DHP est l'acide 7-(L-2-amino-2-carboxyéthylthio)-2-(2,2-diméthylcyclopropanecarboxamide)-2-hepténoïque.

10. Composé selon la revendication 1 de structure :

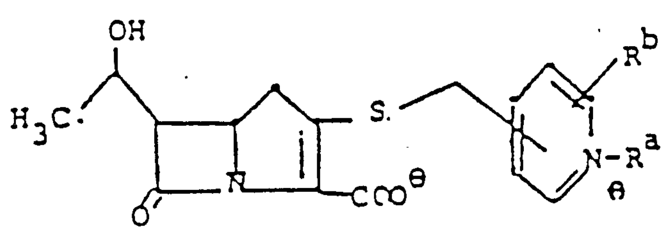

dans laquelle :
$R^a$ est un alkyle en $C_1$-$C_4$ ; ou une chaîne latérale acide de structure $-(CH_2)_n-X-(CH_2)_m-Y'-A$ dans laquelle:
n a une valeur de 0 à 4
m a une valeur de 0 à 4
X est $CHR^s$, CH=CH, phénylène ($-C_6H_4-$), NH, N(alkyle en $C_1$-$C_4$), O, S, S=O, C=O, $SO_2$, $SO_2NH$, $CO_2$, CONR, $OCO_2$, OC=O, NRC=O ; $R^s$ est H, O(alkyle en $C_1$-$C_4$), $NH_2$, NH(alkyle en $C_1$-$C_4$), N(alkyle en $C_1$-$C_4$)$_2$, CN, $CONH_2$, COH(alkyle en $C_1$-$C_4$)$_2$, $CO_2H$, $SO_2NR_2$, $SO_2NH$(alkyle en $C_1$-$C_4$) ;
Y' est une simple liaison, NH, N(alkyle en $C_1$-$C_4$), O, S ;

A est comme défini dans la revendication 1 ;
$R^b$ est un hydrogène ; un cyano ; ou une chaîne latérale acide de structure -A ou $-(CH_2)_n-X-(CH_2)_m-Y'-A$ dans laquelle :
n a une valeur de 0 à 4
m a une valeur de 0 à 4
X est $CHR^s$, CH=CH, phénylène ($-C_6H_4-$), NH, N(alkyle en $C_1$-$C_4$), O, S, S=O, C=O, $SO_2$, $SO_2NH$, $CO_2$, CONH, $OCO_2$, OC=O, NHC=O ; $R^s$ est H, O(alkyle en $C_1$-$C_4$), $NH_2$, NH(alkyle en $C_1$-$C_4$), N(alkyle en $C_1$-$C_4$)$_2$, CN, $CONH_2$, CON(alkyle en $C_1$-$C_4$)$_2$, $CO_2H$, $SO_2NH_2$, $SO_2NH$(alkyle en $C_1$-$C_4$) ;
Y' est une simple liaison, NH, N(alkyle en $C_1$-$C_4$), O, S ;
A est comme défini dans la revendication 1;
sous réserve que $R^a$ ou $R^b$ soit une chaîne latérale acide.

**11.** Composition pharmaceutique utile comme antibiotique comprenant une quantité à effet antibactérien d'un composé selon la revendication 10, une quantité à effet inhibiteur d'un inhibiteur de la DHP et facultativement un véhicule pharmaceutique.

REVENDICATIONS POUR L'ETAT CONTRACTANT: AT

**1.** Procédé pour préparer un composé répondant à la formule :

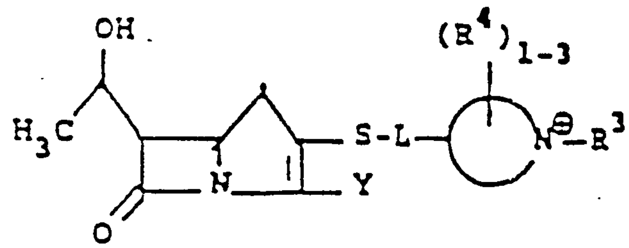

I

dans laquelle :

L est une liaison covalente ou un groupe de pontage choisi parmi $-(CH_2)_{1-4}-S-$ ; $-(CH_2)_{1-4}-O-$ ; $-(CH_2)_{1-4}-X''-(CH_2)_{1-4}-$ où X" est O ou S ; des groupes alkyles droits en $C_1$-$C_4$, ramifiés en $C_1$-$C_6$ ou cycloalkyles en $C_3$-$C_7$ substitués ou non substitués dont les substituants sont choisis parmi alkyle en $C_1$-$C_6$, O-alkyle en $C_1$-$C_6$, S-alkyle en $C_1$-$C_6$, halogéno, OH, $CF_3$, CN, $NR_2$, NH(alkyle en $C_1$-$C_6$), N(alkyle en $C_1$-$C_6$)$_2$, $CO_2H$, $CONH_2$, CONH(alkyle en $C_7$-$C_6$) et CON(alkyle en $C_1$-$C_6$)$_2$ ;

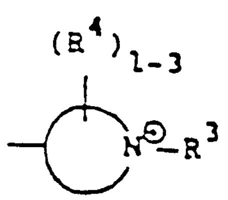 est un groupe pyridinium

où au moins un de $R^3$ et $R^4$ est :
une chaîne latérale acide de structure $-(CH_2)_n-X-(CH_2)_m-Y'-A$, auquel cas $R^4$ peut également être simplement -A où :
n = 0-4
m = 0-4
-A est un composant du groupe constitué essentiellement par carboxy ($CO_2H$), phosphono [$P=O(OH)_2$], alkylphosphono {P=O(OH)[O-(alkyle en $C_1$-$C_4$)]}, alkylphosphinyle [P=O(OH)(alkyle en $C_1$-$C_4$)], phosphoramido substitué [P=O(OH)NH(alkyle en $C_1$-$C_4$) et P=O(OR)-$NHR^X$], sulfino ($SO_2H$), sulfo $SO_3H$), 5-tétrazolyle ($CN_4H$), arylsulfonamido ($SO_2NHR^X$) et les acylsulfonamides représentés par les structures $CONHSO_2$(alkyle en $C_1$-$C_4$), $CONHSO_2N$-(alkyle en $C_1$-$C_4$)$_2$, $SO_2NHCO$(alkyle en $C_1$-$C_4$) et $SO_2NHCOR^X$ où $R^X$ est un groupe hétéroaryle contenant de 5 à 11 atomes dont jusqu'à 5 sont des hétéroatomes

X est $CHR^s$, CH = CH, phénylène ($-C_6H_4-$), NH, N(alkyle en $C_1$-$C_4$), O, S, S = O C = O, $SO_2$, $SO_2NH$, $CO_2$, CONH, $OCO_2$, OC = O, NHC = O ;

$R^s$ est H, O(alkyle en $C_1$-$C_4$), $NH_2$, NH(alkyle en $C_1$-$C_4$), N(alkyle en $C_1$-$C_4$)$_2$, CN, $CONH_2$, CON(alkyle en $C_1$-$C_4$)$_2$, $CO_2H$, $SO_2NH_2$, $SO_2NH$(alkyle en $C_1$-$C_4$) ;

Y' est une simple liaison, NH, N(alkyle en $C_1$-$C_4$), O, S ;

où $R^3$ est facultativement :

1) un radical alkyle en $C_1$-$C_6$ non substitué ou substitué ;

2) un radical alcényle en $C_1$-$C_6$ non substitué ou substitué ;

3) un radical alcynyle en $C_1$-$C_6$ non substitué ou substitué ;

4) un radical cycloalkyle en $C_3$-$C_7$ dont le cycle est substitué ou non substitué et un ou plusieurs atomes peuvent être remplacés par un hétéroatome ;

5) un radical (cycloalkyl en $C_3$-$C_7$)méthyle dont le cycle peut être substitué et un ou plusieurs atomes peuvent être remplacés par un hétéroatome ;

6) un radical cycloalcényle en $C_5$-$C_7$ non substitué ou substitué ;

7) un radical alkylidène bivalent en $C_2$-$C_6$ non substitué ou substitué, facultativement interrompu par un hétéroatome et uni au groupe hétéroarylium pour former un cycle qui est carbocyclique ou dans lequel un ou plusieurs atomes sont remplacés par un hétéroatome ; le nouveau cycle peut contenir une ou plusieurs doubles liaisons ;

8) un radical phényle non substitué ou substitué ;

9) un radical phényl(alkyle en $C_1$-$C_4$) non substitué ou substitué ;

10) un radical cyano(alkyle en $C_1$-$C_4$) ;

11) un radical carbamoyl(alkyle en $C_1$-$C_4$) ;

12) un radical hydroxy(alkyle en $C_1$-$C_4$) ; ou

13) un radical amino(alkyle en $C_1$-$C_4$) dans lequel l'atome d'azote est non substitué ou substitué par 1 à 3 groupes alkyles en $C_1$-$C_4$ ; où les substituants dans les définitions ci-dessus de $R^3$ sont indépendamment choisis dans le groupe constitué par les définitions de $R^4$ indiquées ci-après ;

et où $R^4$ facultativement est choisi indépendamment parmi :

a) un groupe trifluorométhyle ;

b) un atome d'halogène ;

c) un radical alcoxyle en $C_1$-$C_4$ non substitué ;

d) un groupe hydroxy ;

e) un radical (alkyl en $C_1$-$C_6$)carbonyloxy non substitué ;

f) un radical carbamoyloxy qui est non substitué ou substitué sur l'azote par un ou deux groupes alkyles en $C_1$-$C_4$ ;

g) un radical alkylthio en $C_1$-$C_6$, un radical alkylsulfinyle en $C_1$-$C_6$ ou un radical alylsulfonyle en $C_1$-$C_6$, qui sont chacun non substitués sur le groupe alkyle ;

h) un groupe sulfamoyle qui est non substitué ou substitué sur l'azote par un ou deux groupes alkyles en $C_1$-$C_4$ ;

i) un groupe amino ;

j) un groupe mono(alkyl en $C_1$-$C_4$)amino ou di(alkyl en $C_1$-$C_4$) amino, chacun non substitué sur le groupe alkyle ;

k) un groupe formylamino ;

l) un radical (alkyl en $C_1$-$C_6$)carbonylamino non substitué ;

m) un radical (alcoxy en $C_1$-$C_4$)carbonylamino ;

n) un groupe uréido dont l'azote terminal est non substitué ou substitué par un ou deux groupes alkyles en $C_1$-$C_4$ ;

o) un groupe (alkyl en $C_1$-$C_6$)sulfonamido ;

p) un groupe cyano ;

q) un radical formyle ou formyle acétalisé ;

r) un radical (alkyl en $C_1$-$C_6$)carbonyle non substitué dont le carbonyle est libre ou acétalisé ;

s) un radical phénylcarbonyle non substitué ;

t) un radical hydroxyiminométhyle dont l'atome d'oxygène ou de carbone est facultativement substitué par un groupe alkyle en $C_1$-$C_4$ ;

u) un radical (alcoxy en $C_1$-$C_6$)carbonyle ;

v) un radical carbamoyle qui est non substitué ou substitué sur l'azote par un ou deux groupes alkyles en $C_1$-$C_4$ ;

w) un radical N-hydroxycarbamoyle ou N(alcoxy en $C_1$-$C_4$) carbamoyle dont l'atome d'azote peut de plus être substitué par un groupe alkyle en $C_1$-$C_4$ ;

y) un groupe amidino 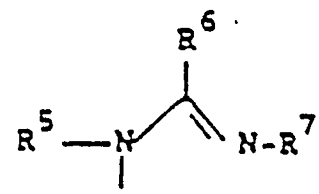

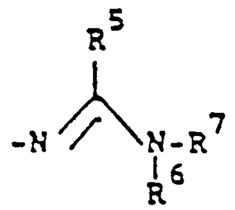

x) un groupe thiocarbamoyle ; où $R^5$, $R^6$ et $R^7$ sont indépendamment un hydrogène, un alkyle en $C_1$-$C_4$, ou deux des groupes alkyles forment ensemble un radical alkylidène en $C_2$-$C_6$ facultativement interrompu par un hétéroatome et réunis pour former un cycle ;

z) un groupe carboxamidino 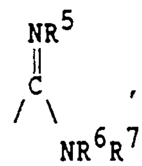 , dans lequel $R^5$, $R^6$ et $R^7$ sont définis comme ci-dessus ;

aa) un groupe guanidinyle où $R^6$ dans Y) ci-dessus est $NR^8R^9$ et $R^8$ et $R^9$ sont définis comme pour $R^5$ à $R^7$ ci-dessus ;
ab) un hydrogène ;
ac) un radical alkyle en $C_1$-$C_6$ non substitué ;
ad) un radical alcényle en $C_1$-$C_6$ non substitué ;
ae) un radical alcynyle en $C_1$-$C_6$ non substitué ;
af) un radical cycloalkyle en $C_3$-$C_7$ dont le cycle est non substitué et un ou plusieurs atomes peuvent être remplacés par un hétéroatome ;
ag) un radical (cycloalkyl en $C_3$-$C_7$)méthyle dont un ou plusieurs atomes peuvent être remplacés par un hétéroatome ;
ah) un radical cycloalcényle en $C_5$-$C_7$ non substitué ;
ai) un radical phényle non substitué ; et
aj) un radical phényl(alkyle en $C_1$-$C_4$) non substitué ; et un hétéroatome dans les définitions ci-dessus de A, $R^3$ et $R^4$ est un atome d'azote, d'oxygène ou de soufre choisi indépendamment dans le cas où il y a plusieurs hétéroatomes ; et

Y est choisi parmi :
i) COOH ou un ester ou sel pharmaceutiquement acceptable de celui-ci,
ii) $COOR^1$ où $R^1$ est un groupe carboxy-protecteur éliminable,
iii) COOM où M est un métal alcalin, ou
iv) $COO^{\ominus}$ ; sous réserve que, lorsque Y est autre que iv), il existe un ion opposé $z^{\ominus}$.

qui comprend

A . la réaction d'un composé de formule :

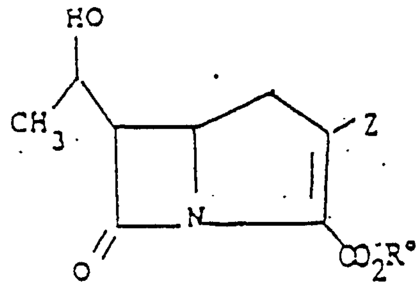

avec un composé de formule :

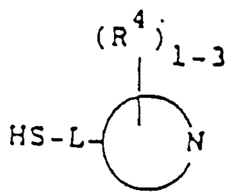

en présence d'une base,
le traitement du composé obtenu de formule :

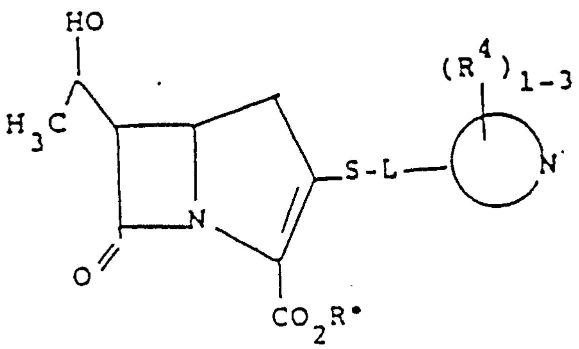 with $R^3X'$ or $R^3O^{\oplus}X'^{\ominus}$

avec $R^3X'$ ou $R^3O^{\oplus}X'^{\ominus}$

et facultativement le déblocage du composé obtenu de formule :

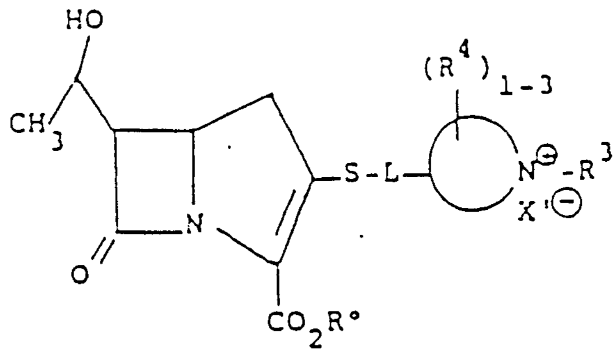

pour obtenir :

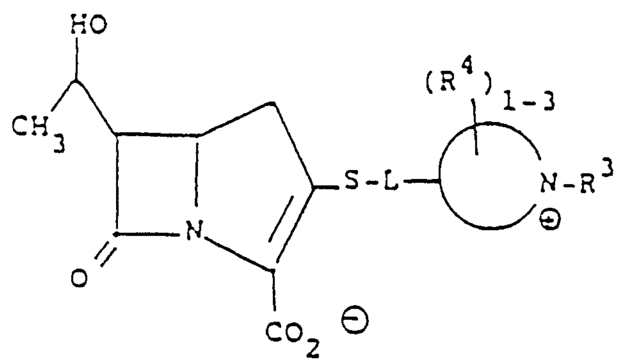

I

où Z et X' sont des groupes labiles ou X' est un groupe anionique ; et R° est un groupe protecteur et $R^3$, L,

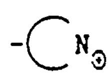

et $R^4$ sont comme défini ci-dessus,

B . la réaction d'un composé de formule :

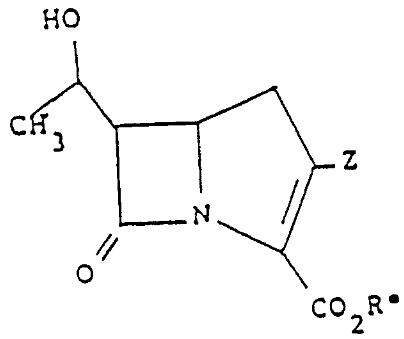

avec un composé de formule :

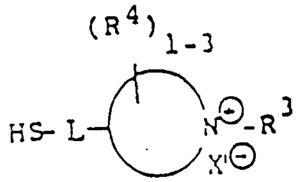

en présence d'une base et facultativement le déblocage du composé obtenu de formule:

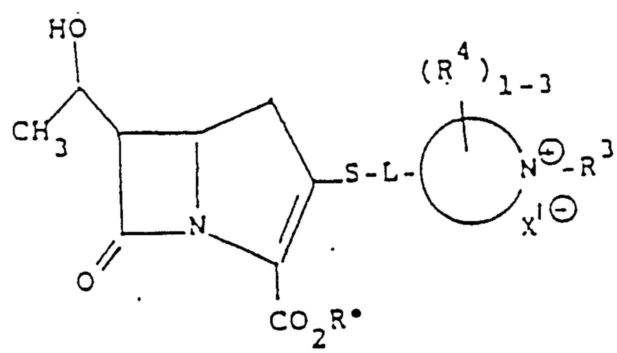

pour obtenir :

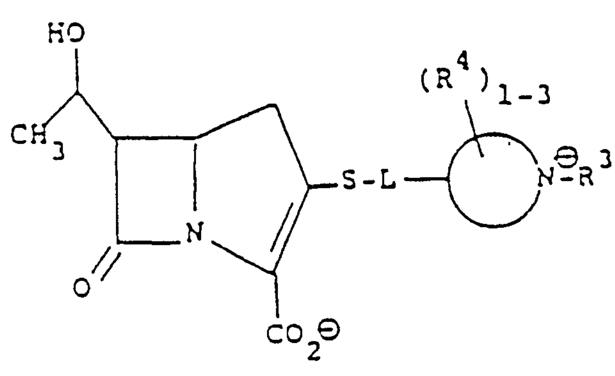

où Z, X', L, R•, $R^3$,

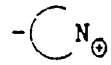

et $R^4$ sont comme défini ci-dessus,

C . la réaction d'un composé de formule

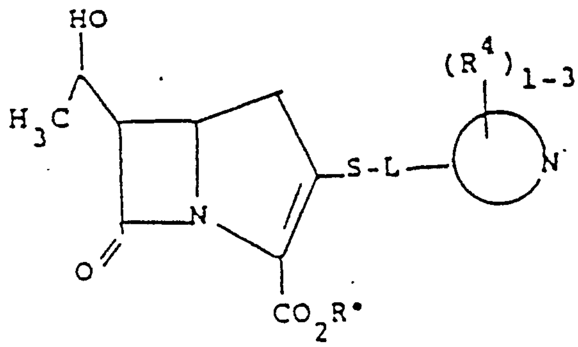

with $R^3X'$ or $R^3O^{\oplus}X'^{\ominus}$

avec $R^3X'$ ou $R^3O^{\oplus}$◊$X'^{\ominus}$
et facultativement le déblocage du composé obtenu de formule :

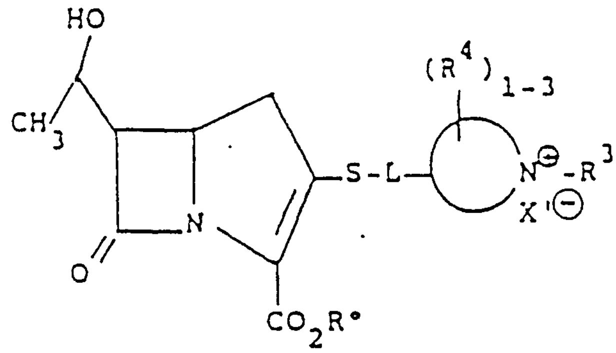

pour obtenir :

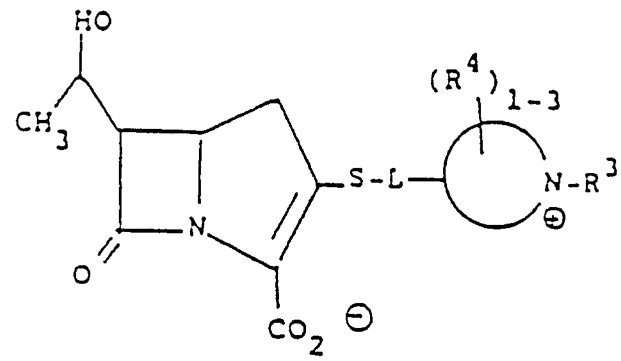

I

où Z et X' sont des groupes labiles ou X' est un groupe anionique ; et R• est un groupe protecteur et $R^3$ L,

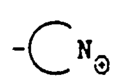

et $R^4$ sont comme défini ci-dessus,
B . la réaction d'un composé de formule :

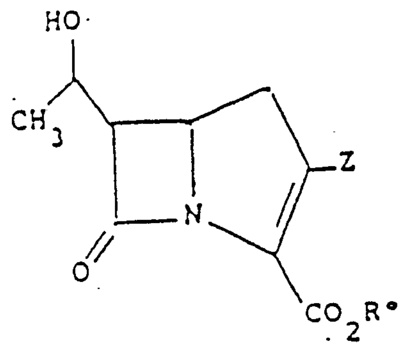

avec NaHS,
le traitement du composé obtenu de formule :

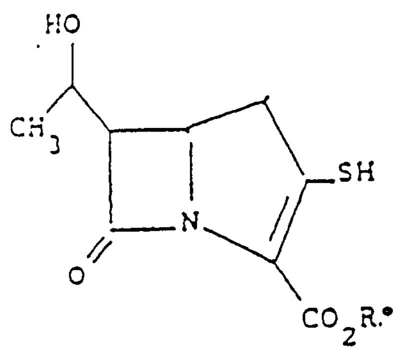

avec

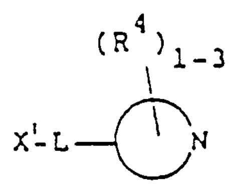

le traitement du composé obtenu de formule :

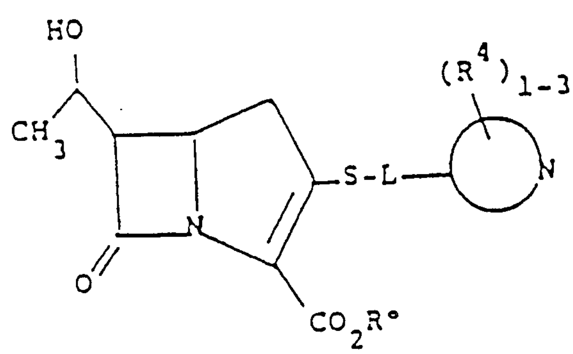

avec $R^3X'$ ou $R^3O^{\oplus}X'^{\ominus}$
et facultativement le déblocage du composé obtenu de formule :

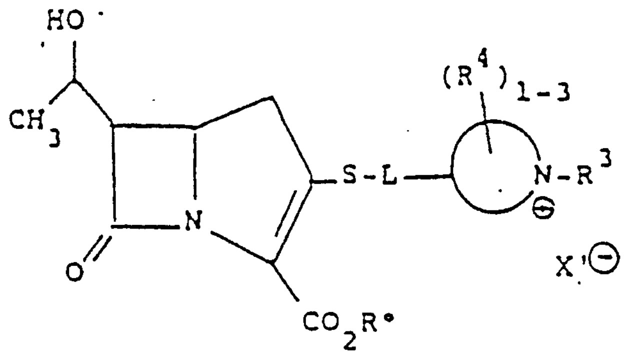

pour obtenir :

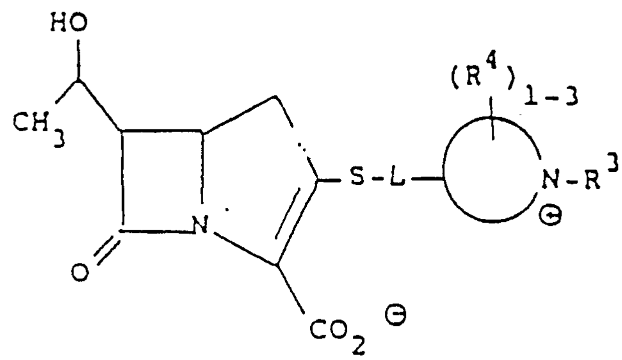

où Z, X', L, R•, R³,

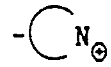

et R⁴ sont comme précédemment défini,

D . la réaction d'un composé de formule : avec NaHS,

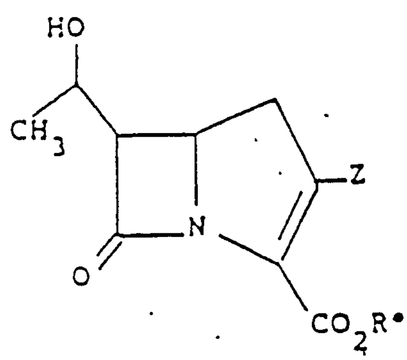

le traitement du composé obtenu de formule :

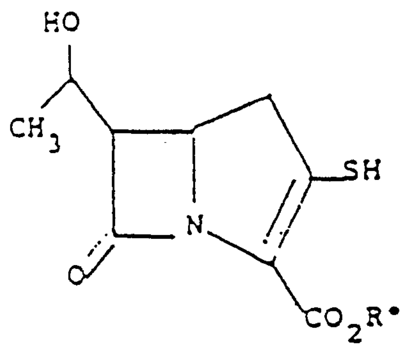

avec 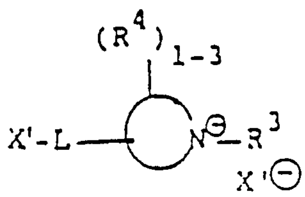

en présence d'une base et facultativement le déblocage du composé obtenu de formule :

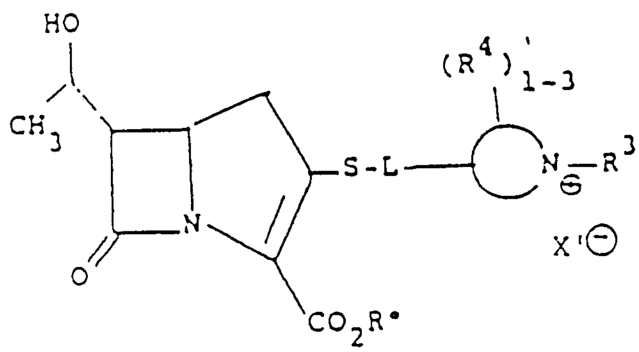

pour obtenir :

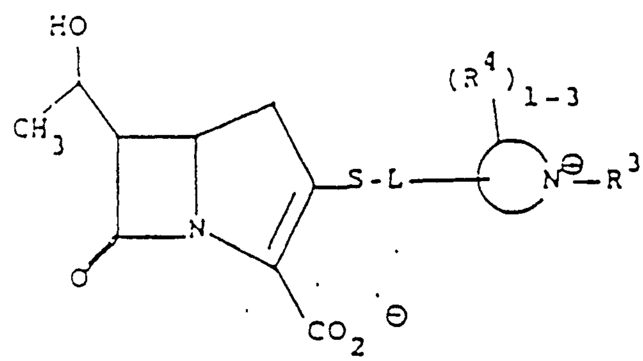

I

où Z, X', R•, $R^3$, L,

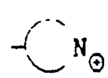

et $R^4$ sont comme précédemment défini.

2. Procédé selon la revendication 1 pour préparer un composé dans lequel L est un alkyle en $C_1$-$C_4$ ramifié ou linéaire, substitué ou non substitué.

3. Procédé selon la revendication 2 pour préparer un composé dans lequel $R^3$ est un groupe alkyle en $C_1$-$C_4$ non substitué ou substitué.

4. Procédé selon la revendication 3 pour préparer un composé dans lequel L est $-CH_2-$, $-CH(CH_3)-$ ou --$(CH_2)_2-$.

5. Procédé selon la revendication 1 pour préparer un composé qui fait partie du groupe constitué par :

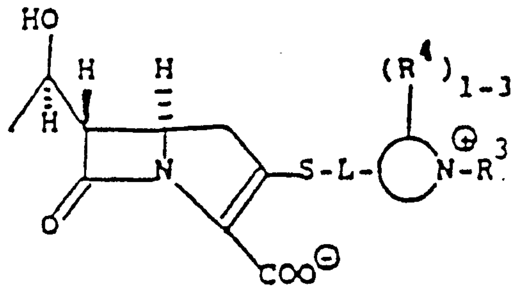

| Composé n° | L | 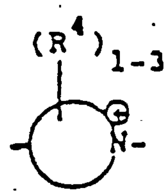 | $R^3$ |
|---|---|---|---|
| 1 | $-CH_2-$ | 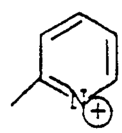 | $CH_2CO_2H$ |
| 2 | " | " | $CH_2\overset{O \uparrow}{P}(OH)OCH_3$ |
| 3 | " | " | $CH_2SO_3H$ |
| 4 | " | " | 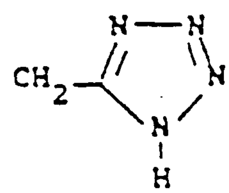 |
| 5 | " | 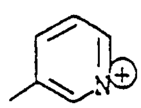 | $CH_2CO_2H$ |
| 6 | " | " | $CH_2\overset{O \uparrow}{P}(OH)OCH_3$ |
| 7 | " | " | $CH_2SO_3H$ |
| 8 | " | 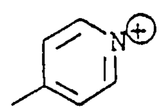 | $CH_2CO_2H$ |
| 9 | " | " | $CH_2\overset{O \uparrow}{P}(OH)OCH_3$ |
| 10 | " | " | $CH_2SO_3H$ |

| | | | |
|---|---|---|---|
| 11 | $-CH_2-$ | 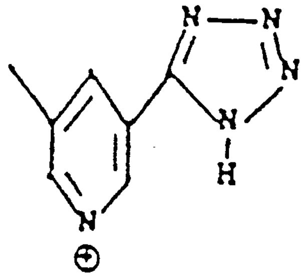 | $CH_3$ |
| 12 | " | 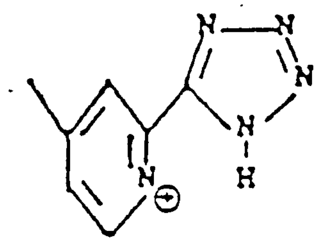 | " |
| 13 | " | 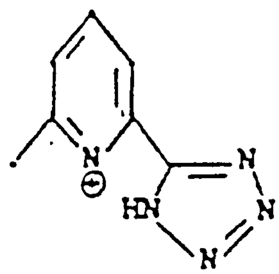 | " |
| 14 | " | 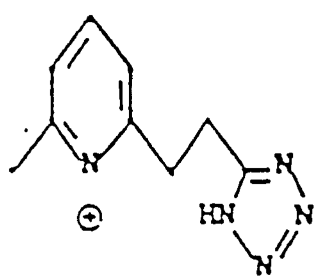 | " |
| 15 | bond | 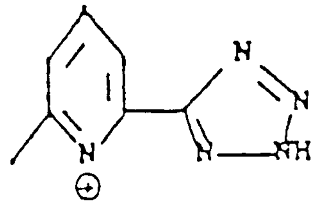 | " |

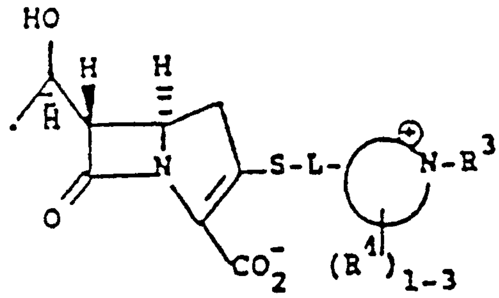

| Composé n° | L | 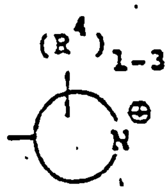 | $R^3$ | $R^4$ |
|---|---|---|---|---|
| 1 | $-CH_2-$ | 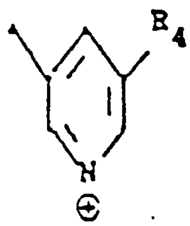 | $CH_3$ | $CO_2H$ |
| 2 | " | " | " | $SO_3H$ |
| 3 | " | " | " | $CH_2CO_2H$ |
| 4 | " | " | " | 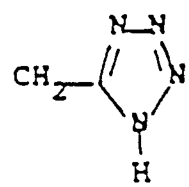 |
| 5 | " | " | " | $CH_2CH_2CO_2H$ |
| 6 | " | " | " | $CH_2SO_3H$ |
| 7 | " | " | " | 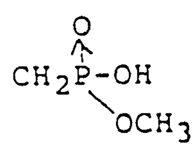 |
| 8 | " | " | " | $CH_2CH_2SO_3H$ |
| 9 | " | 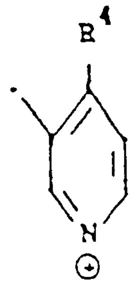 | " | $CO_2H$ |

| | | | | |
|---|---|---|---|---|
| 10 | " | " | " | $SO_3H$ |
| 11 | " | " | " | $CH_2CO_2H$ |
| 12 | " | " | " | 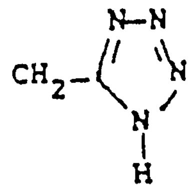 |
| 13 | " | " | " | $CH_2CH_2CO_2H$ |
| 14 | " | " | " | $CH_2SO_3H$ |
| 15 | " | " | " | 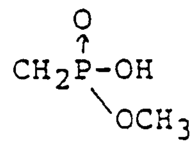 |
| 16 | " | " | " | $CH_2CH_2SO_3H$ |
| 17 | " | 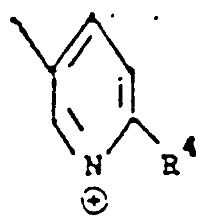 | " | $CO_2H$ |
| 18 | " | " | " | $SO_3H$ |
| 19 | " | " | " | $CH_2CO_2H$ |
| 20 | " | " | " | 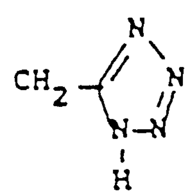 |
| 21 | " | " | " | $CH_2CH_2CO_2H$ |
| 22 | " | " | " | $CH_2SO_3H$ |

| | | | | |
|---|---|---|---|---|
| 23 | " | " | " | 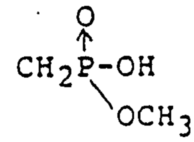 |
| 24 | " | " | " | $CH_2CH_2SO_3H$ |
| 25 | " | " | " | 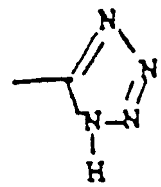 |
| 26 | " | 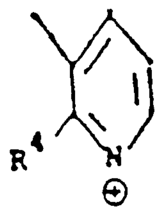 | " | $CO_2H$ |
| 27 | " | " | " | $SO_3H$ |
| 28 | " | " | " | $CH_2CO_2H$ |
| 29 | " | " | " | 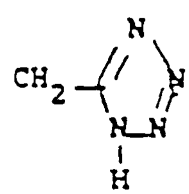 |
| 30 | " | " | " | $CH_2CH_2CO_2H$ |
| 31 | " | " | " | $CH_2SO_3H$ |
| 32 | " | " | " | 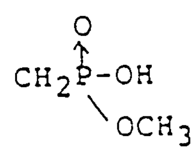 |
| 33 | " | " | " | $CH_2CH_2SO_3H$ |

| | | | | |
|---|---|---|---|---|
| 34 | " | " | " | 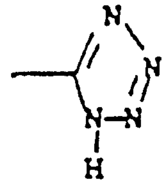 |
| 35 | " | 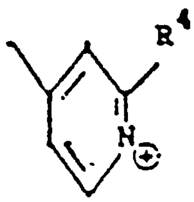 | " | $CO_2H$ |
| 36 | " | " | " | $SO_3H$ |
| 37 | " | " | " | $CH_2CO_2H$ |
| 38 | " | " | " | 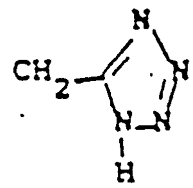 |
| 39 | " | " | " | $CH_2CH_2CO_2H$ |
| 40 | " | " | " | $CH_2SO_3H$ |
| 41 | " | " | " | 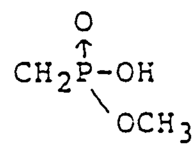 |
| 42 | " | " | " | $CH_2CH_2SO_3H$ |
| 43 | " | " | " | 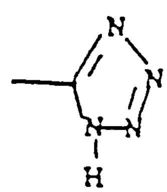 |
| 44 | " | 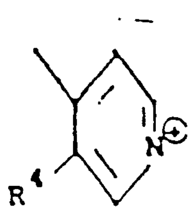 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 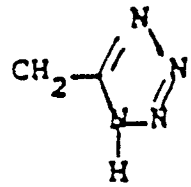 |
| 48 | " | " | " | $CH_2CH_2CO_2H$ |
| 49 | " | " | " | $CH_2SO_3H$ |
| 50 | " | " | " | $CH_2\overset{O}{\overset{\uparrow}{P}}(OH)OCH_3$ |
| 51 | " | " | " | $CH_2CH_2SO_3H$ |
| 52 | " | " | " | 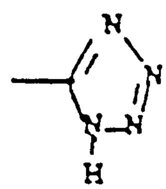 |
| 53 | " | 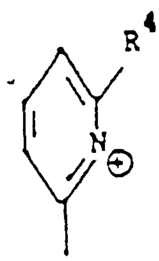 | " | $CO_2H$ |
| 54 | " | " | " | $SO_3H$ |
| 55 | " | " | " | $CH_2CO_2H$ |
| 56 | " | " | " | 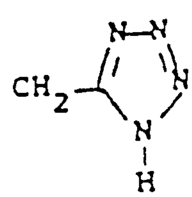 |

| | | | | |
|---|---|---|---|---|
| 57 | " | " | " | $CH_2CH_2CO_2H$ |
| 58 | " | " | " | $CH_2SO_3H$ |
| 59 | " | " | " | 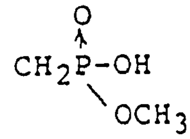 |
| 60 | " | " | " | $CH_2CH_2SO_3H$ |
| 61 | " | 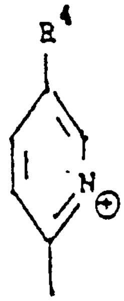 | " | $CO_2H$ |
| 62 | " | " | " | $SO_3H$ |
| 63 | " | " | " | $CH_2CO_2H$ |
| 64 | " | " | " | 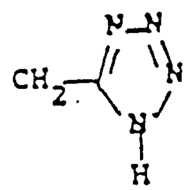 |
| 65 | " | " | " | $CH_2CH_2CO_2H$ |
| 66 | " | " | " | $CH_2SO_3H$ |
| 67 | " | " | " | 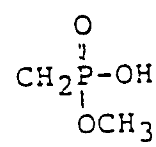 |
| 68 | " | " | " | $CH_2CH_2SO_3H$ |
| 69 | " | 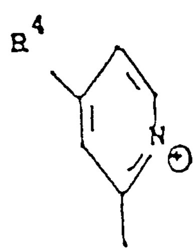 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 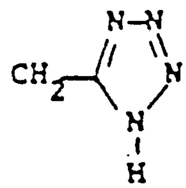 |
| 73 | " | " | " | $CH_2CH_2CO_2H$ |
| 74 | " | " | " | $CH_2SO_3H$ |
| 75 | " | " | " | $CH_2\overset{O}{\overset{\Vert}{P}}(OCH_3)-OH$ |
| 76 | " | " | " | $CH_2CH_2SO_3H$ |
| 77 | " | 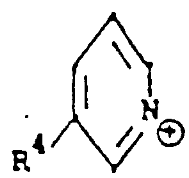 | " | $CO_2H$ |
| 78 | " | " | " | $SO_3H$ |
| 79 | " | " | " | $CH_2CO_2H$ |
| 80 | " | " | " | 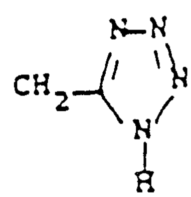 |
| 81 | " | " | " | $CH_2CH_2CO_2H$ |
| 82 | " | " | " | $CH_2SO_3H$ |

| | | | | |
|---|---|---|---|---|
| 83 | " | " | " | 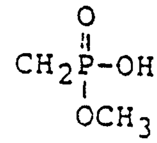 |
| 84 | " | " | " | $CH_2CH_2SO_3H$ |

**6.** Procédé selon la revendication 1, dans lequel le composé obtenu est de plus combiné avec un inhibiteur de la DHP.

**7.** Procédé selon la revendication 6, dans lequel l'inhibiteur de la DHP est l'acide 7-(L-2-amino-2-carboxyéthylthio)-2-(2,2-diméthylcyclopropanecarboxamide)-2-hepténoïque.

**8.** Procédé selon la revendication 1, dans lequel une quantité à effet antibactérien du composé obtenu est de plus combinée avec une quantité à effet inhibiteur d'un inhibiteur de la DHP et, facultativement, un véhicule pharmaceutiquement acceptable.

**9.** Procédé selon la revendication 8, dans lequel l'inhibiteur de la DRP est l'acide 7-(L-2-amino-2-carboxyéthylthio)-2-(2,2-diméthylcyclopropanecarboxamide)-2-hepténoïque.

**10.** Procédé selon la revendication 1 pour préparer un composé de structure :

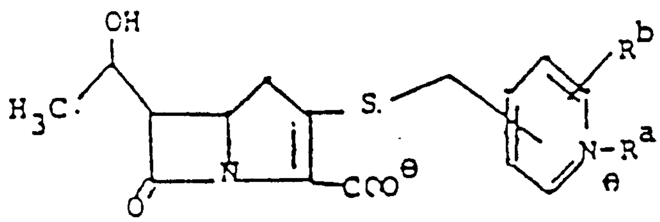

dans laquelle :
$R^a$ est un alkyle en $C_1$-$C_4$ ; ou une chaîne latérale acide de structure $-CH_2)_n-X-(CH_2)_m-Y'-A$ dans laquelle :
n a une valeur de 0 à 4
m a une valeur de 0 à 4
X est $CHR^s$, CH = CH, phénylène ($-C_6H_4-$), NH, N(alkyle en $C_1$-$C_4$), O, S, S = O, C = O, $SO_2$, $SO_2NH$, $CO_2$, CONH, $OCO_2$, OC = O, NHC = O ;
$R^s$ est H, O(alkyle en $C_1$-$C_4$), $NH_2$, NH(alkyle en $C_1$-$C_4$), N(alkyle en $C_1$-$C_4$)$_2$, CN, $CONH_2$, CON(alkyle en $C_1$-$C_4$)$_2$, $CO_2H$, $SO_2NH_2$, $SO_2NH$(alkyle en $C_1$-$C_4$) ; Y' est une simple liaison, NH, N(alkyle en $C_1$-$C_4$), O, S ;
A est comme défini dans la revendication 1;
$R^b$ est un hydrogène ; un cyano ; ou une chaîne latérale acide de structure -A ou $-(CH_2)_n-X-(CH_2)_m-Y'-A$ dans laquelle :
n a une valeur de 0 à 4
m a une valeur de 0 à 4
X est $CHR^s$, CH = CH, phénylène ($-C_6H_4-$), NH, N(alkyle en $C_1$-$C_4$), O, S, S = O, C = O, $SO_2$, $SO_2NH$, $CO_2$, CONH, $OCO_2$, OC = O, NHC = O ;
$R^s$ est H, O(alkyle en $C_1$-$C_4$), $NH_2$, NH(alkyle en $C_1$-$C_4$ ), N(alkyle en $C_1$-$C_4$)$_2$, CN, $CONH_2$, CON(alkyle en $C_1$-$C_4$)$_2$, $CO_2H$, $SO_2NH_2$, $SO_2NH$(alkyle en $C_1$-$C_4$);
Y' est une simple liaison, NH, N(alkyle en $C_1$-$C_4$), O, S ;
A est comme défini dans la revendication 1 ;
sous réserve que $R^a$ ou $R^b$ soit une chaîne latérale acide.

11. Procédé selon la revendication 10, dans lequel une quantité à effet antibactérien du composé obtenu est de plus combinée à une quantité à effet inhibiteur d'un inhibiteur de la DHP et facultativement à un véhicule pharmaceutique.

**Ansprüche**

1. Verbindung mit der Formel:

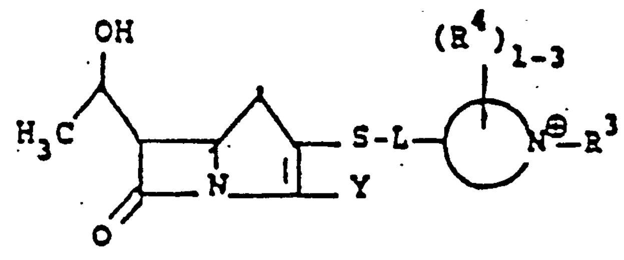

I

worin:

L eine covalente Bindung oder eine überbrückende Gruppe ist, ausgewählt unter-$(CH_2)_{1-4}S$-; --$(CH_2)_{1-4}$-0-; -$(CH_2)_{1-4}$-X"-$(CH_2)_{1-4}$' worin X" für O oder S steht; substituierten oder unsubstituierten linearen $C_1$-$C_4$- veizweigten $C_1$-$C_6$-Alkyl- oder $C_3$-$C_7$-Cycloalkylgruppen, worin die Substituenten unter $C_1$-$C_6$-Alkyl, O-$C_1$-$C_6$-Alkyl, S-$C_1$-$C_6$-Alkyl, Halogen, OH, $CF_3$, CN, $NH_2$, NH($C_1$-$C_6$-Alkyl), N($C_1$-$C_6$-Alkyl)$_2$, $CO_2H$, $CONH_2$, CONH($C_1$-$C_6$-Alkyl) und CON($C_1$-$C_6$-Alkyl)$_2$ ausgewählt sind;

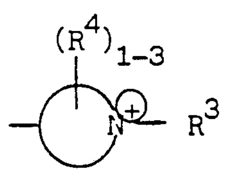

eine Pyridiniumgruppe ist,

worin mindestens ein Substituent $R^3$ und $R^4$
eine saure Seitenkette der Struktur -$(CH_2)_n$-X-$(CH_2)_m$-Y'-A ist, welche im Fall von $R^4$ auch einfach -A sein kann, worin:
n = 0-4
m = 0-4
welches -A ein Rest ist, welcher aus der Gruppe ausgewählt ist, die im wesentlichen aus Carboxy($CO_2H$), Phosphono[P=O(OH)$_2$] , Alkyl-phosphono{P=O(OH) [O($C_1$-$C_4$-Alkyl)]} , Alkylphosphyl[P=O(OH)($C_1$-$C_4$-alkyl)], substituiertem Phosphoramido[P=O(OH)NH($C_1$-$C_4$-alkyl) und P=O(OH)NHR$^{x}$] Sulfino($SO_2H$), sulfo($SO_3H$), 5-Tetrazolyl($CN_4H$), Arylsulfonamido-($SO_2NHR^x$) und Acylsulfonamiden, dargestellt durch die Strukturen $CONHSO_2$($C_1$-$C_4$-alkyl ), $CONHSO_2N$($C_1$-$C_4$-alkyl)$_2$ $SO_2NHCO$($C_1$-$C_4$-alkyl) und $SO_2NHCOR^x$, worin $R^x$ eine Heterarylgruppe mit 5 bis 11 Ringatomen ist, wovon bis zu fünf Heteroatome sind, besteht;

X = $CHR^s$, CH=CH, Phenylen(-$C_6H_4$-), NH, N($C_1$-$C_4$-Alkyl), O, S, S=O, C=O, $SO_2$, $SO_2NH$, $CO_2$, CONH, $OCO_2$, OC=O, NHC=O;
$R^s$ = H, O($C_1$-$C_4$-Alkyl), $NH_2$, NH($C_1$-$C_4$-Alkyl), N($C_1$-$C_4$-Alkyl)$_2$ CN, $CONH_2$, CON($C_1$-$C_4$-Alkyl)-$_2$, $CO_2H$, $SO_2NH_2$, $SO_2NH$($C_1$-$C_4$-Alkyl);

Y' = eine Einfachbindung, NH N($C_1$-$C_4$-Alkyl), O, S; worin $R^3$ gegebenenfalls:
1) ein unsubstituierter oder substituierter $C_1$-$C_6$-Alkylrest;
2) ein unsubstituierter oder substituierter $C_1$-$C_6$-Alkenylrest;
3) ein unsubstituierter oder substituierter $C_1$-$C_6$-Alkinylrest;
4) ein $C_3$-$C_7$-cloalkylrest, worin der Ring substituiert oder unsubstituiert ist und ein oder mehrere

Atome durch ein Heteroatom ersetzt sein können;
5) ein $C_3$-$C_7$-Cycloalkylmethylrest, worin der Ring substituiert sein kann und ein oder mehrere Atome durch ein Heteroatom ersetzt sein können;
6) ein unsubstituierter oder substituierter $C_5$-$C_7$-Cycloalkenylrest;
7) ein unsubstituierter oder substituierter zweirtiger $C_2$-$C_6$-Alkylidenrest, welcher gegebenenfalls durch ein Heteroatom unterbrochen ist und welcher an die Heteroaryliumgruppe gebunden ist, um einen Ring auszubilden, welcher carbocyclisch ist oder in welchem ein oder mehrere Atome durch ein Heteroatom ersetzt sind; der neue Ring kann eine oder mehrere Doppelbindungen enthalten;
8) ein unsubstituierter oder substituierter Phenylrest;
9) ein unsubstituierter oder substituierter Phenyl($C_1$-$C_4$-alkyl)-Rest;
10) ein Cyano($C_1$-$C_4$-alkyl)-Rest;
11) ein Carbamoyl($C_1$-$C_4$-alkyl)-Rest;
12) ein Hydroxy($C_1$-$C_4$-alkyl)-Rest; oder 13) ein Amino($C_1$-$C_4$-alkyl)-Rest, worin das Stickstoffatom unsubstituiert oder mit ein bis drei $C_1$-$C_4$-Alkylgruppen substituiert ist, ist;
worin die Substituenten in den obigen Definitionen von $R^3$ unabhängig voneinander aus der Gruppe ausgewählt sind, welche aus den Definitionen von $R^4$, welche unten angegeben sind, besteht; und worin $R^4$ gegebenenfalls unabhängig voneinander ausgewählt ist unter:
a) einer Trifluormethylgruppe;
b) einem Halogenatom;
c) einem unsubstituierten $C_1$-$C_4$-Alkoxylrest;
d) einer Hydroxygruppe;
e) einem unsubstituierten ($C_1$-$C_6$ Alkyl)carbonyloxyrest;
f) einem Carbamoyloxyrest, welcher unsubstituiert ist oder am Stickstoff mit einer oder zwei $C_1$-$C_4$-Alkylgruppen substituiert ist;
g) einem $C_1$-$C_6$-Alkylthiorest, einem $C_1$-$C_6$-Alsulfinylrest oder einem $C_1$-$C_6$-Alkylsulfonylrest, wovon jeder an der Alkylgruppe unsubstituiert ist;
h) einer Sulfamoylgruppe, welche unsubstituiert ist oder am Stickstoff durch eine oder zwei $C_1$-$C_4$-Alkylgruppen substituiert ist;
i) einer Aminogruppe;
j) einer Mono($C_1$-$C_4$-alkyl)-amino- oder Di($C_1$-$C_4$-alkyl)aminogruppe wovon jede an der Alkylgruppe unsubstituiert ist;
k) einer Formylaminogruppe;
1) einem unsubstituierten ($C_1$-$C_6$-Alkyl)carbonylaminorest;
m) einem ($C_1$-$C_4$-Alkoxy)carbonylaminorest;
n) einer Ureidogruppe, in welcher der endständige Stickstoff unsubstituiert ist oder mit einer oder zwei $C_1$-$C_4$-Alkylgruppen substiert ist;
o) einer ($C_1$-$C_6$-Alkyl)sulfonamidogruppe;
p) einer Cyanogruppe;
q) einem Formyl- oder einem acetalisierten Formylrest;
r) einem unsubstituierten $C_1$-$C_6$-Alkyl)carbonylrest, worin das Carbonyl frei oder acetalisiert ist;
s) einem unsubstierten Phenylcarbonylrest;
t) einem Hydroxyiminomethylrest in welchem das Sauerstoff- oder Kohlenstoffatom gegebenenfalls durch eine $C_1$-$C_4$-Alkylgruppe substituiert ist;
u) einem ($C_1$-$C_6$-Alkoxy)carbonylrest;
v) einem Carbamoylrest, welcher unsubstitiert ist oder am Stickstoff durch eine oder zwei $C_1$-$C_4$-Alkylgruppen substituiert ist;
w) einem N-Hydroxycarbamoyl- oder N($C_1$-$C_4$-alkoxy)carbamoylrest, in welchem das Stickstoffatom zusätzlich durch eine $C_1$-$C_4$-Alkylgruppe substituiert sein kann;
x) einer Thiocarbamoylgruppe;

y) einer Amidinogruppe

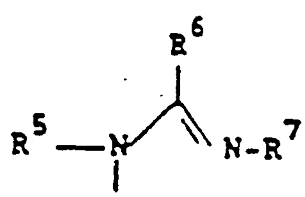
,
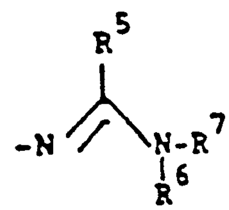

worin $R^5$, $R^6$ und $R^7$ unäbhängig voneinander Wasserstoff, $C_1$-$C_4$-Alkyl sind, oder worin zwei der Alkylgruppe gemeinsam einen $C_2$-$C_6$-Alkylidenrest bilden, welcher gegebenenfalls durch ein Heteroatom unterbrochen ist, und welche aneinander gebunden sind, um einen Ring auszubilden;

z) einer Carboxamidinogruppe 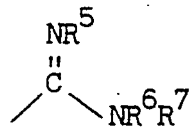 , worin $R^5$, $R^6$ und $R^7$ wie oben definiert sind;

aa) einer Guanidinylgruppe, worin $R^6$ in y) oben $NR^8R^9$ ist, und $R^8$ und $R^9$ wie oben für $R^5$ bis $R^7$ definiert sind;
ab) Wasserstoff;
ac) einem unsubstituierten $C_1$-$C_6$-Alkylrest;
ad) einem unsubstituierten $C_1$-$C_6$-Alkenylrest;
ae) einem unsubstituierten $C_1$-$C_6$-Alkinylrest;
af) einem $C_3$-$C_7$-Cycloalkylrest, in welchem der Ring unsubstituiert ist und ein oder mehrere Atome durch ein Heteroatom ersetzt sein können;
ag) einem $C_3$-$C_7$-Cycloalkylmethylrest, in welchem ein oder mehrere Atome durch ein Heteroatom ersetzt sein können;
ah) einem unsubstituierten $C_5$-$C_7$-Cycloalkenylrest;
ai) einem unsubstituierten Phelrest; und aj) einem unsubstituierten Phenyl($C_1$-$C_4$-alkyl)-Rest; und worin Heteroatom in den obigen Definitionen von A, $R^3$ und $R^4$ Stickstoff, Sauerstoff oder Schwefel bedeutet, und welches unabhängig voneinander ausgewählt ist, wenn mehr als ein Heteroatom auftritt; und

Y ausgewählt ist unter:
i) COCH oder einem pharmazeutisch annehmbaren Ester oder Salz hievon,
ii) $COOR^1$ , worin $R^1$ eine entfernbare Carboxyschutzgruppe ist,
iii) COOM, worin M ein Alkalimetall ist, oder
iv) $COO^-$; mit der Maßgabe, daß, wenn Y eine andere Bedeutung als iv) besitzt, ein Gegenion $Z^-$ zur Verfügung steht.

**2.** Verbindung nach Anspruch 1, worin L ein substituiertes oder unsubstituiertes, verzweigtes oder lineares $C_1$-$C_4$-Alkyl ist.

**3.** Verbindung nach Anspruch 2, worin $R^3$ eine unsubstituierte oder substituierte $C_1$-$C_4$-Alkylgruppe ist.

**4.** Verbindung nach Anspruch 3, worin L $-CH_2-$, $-CH(CH_3)-$ oder $-(CH_2)_2-$ ist.

**5.** Verbindung nach Anspruch 1, worin die Verbindung eine Verbindung ist, welche aus der Gruppe bestehend aus:

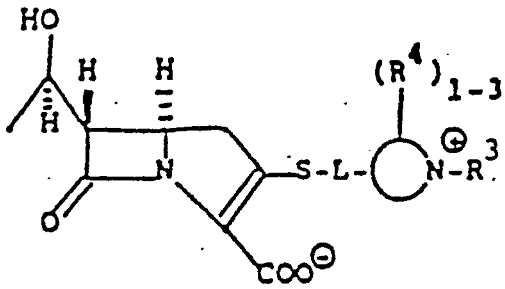

| Verbindung Nr. | L | 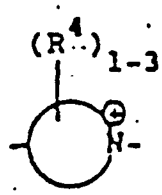 | $R^3$ |
|---|---|---|---|
| 1 | $-CH_2-$ | 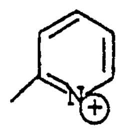 | $CH_2CO_2H$ |
| 2 | " | " | $CH_2\overset{\overset{O}{\uparrow}}{P}(OH)OCH_3$ |
| 3 | " | " | $CH_2SO_3H$ |
| 4 | " | " | 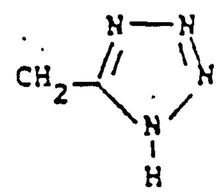 |
| 5 | " | 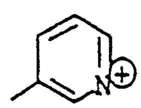 | $CH_2CO_2H$ |
| 6 | " | " | $CH_2\overset{\overset{O}{\uparrow}}{P}(OH)OCH_3$ |
| 7 | " | " | $CH_2SO_3H$ |
| 8 | " | 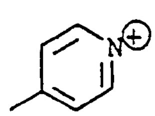 | $CH_2CO_2H$ |
| 9 | " | " | $CH_2\overset{\overset{O}{\uparrow}}{P}(OH)OCH_3$ |
| 10 | " | " | $CH_2SO_3H$ |

| | | | |
|---|---|---|---|
| 11 | $-CH_2-$ | 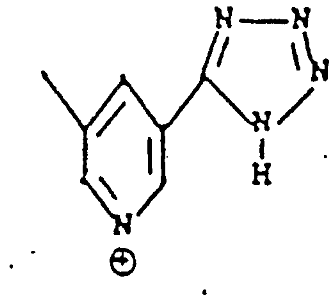 | $CH_3$ |
| 12 | " | 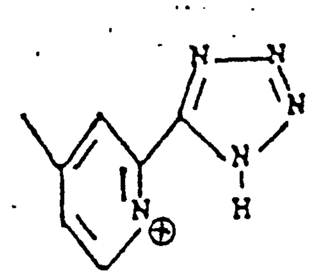 | " |
| 13 | " | 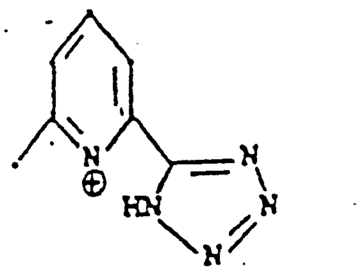 | " |
| 14 | " | 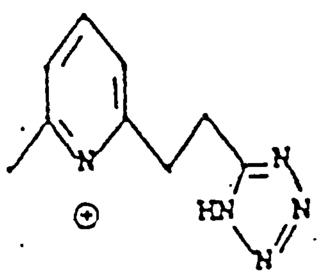 | " |
| 15 | Bindung | 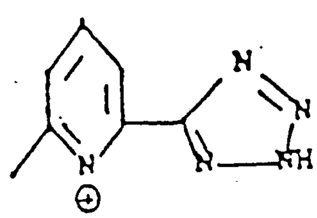 | " |

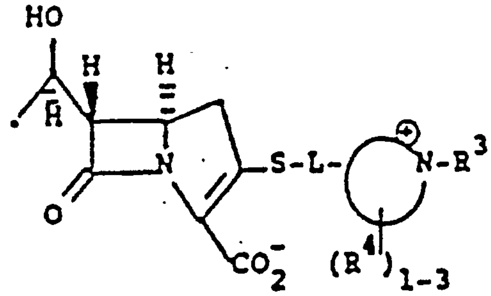

| Verbindung Nr. | L | 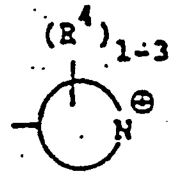 | $R^3$ | $R^4$ |
|---|---|---|---|---|
| 1 | $-CH_2-$ | 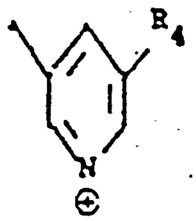 | $CH_3$ | $CO_2H$ |
| 2 | " | " | " | $SO_3H$ |
| 3 | " | " | " | $CH_2CO_2H$ |
| 4 | " | " | " | 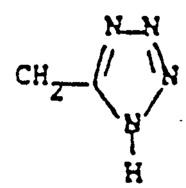 |
| 5 | " | " | " | $CH_2CH_2CO_2H$ |
| 6 | " | " | " | $CH_2SO_3H$ |
| 7 | " | " | " | 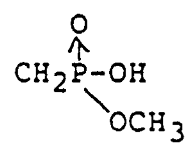 |
| 8 | " | " | " | $CH_2CH_2SO_3H$ |
| 9 | " | 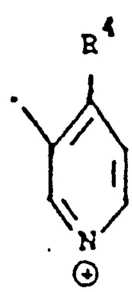 | " | $CO_2H$ |

| | | | | |
|---|---|---|---|---|
| 10 | " | " | " | $SO_3H$ |
| 11 | " | " | " | $CH_2CO_2H$ |
| 12 | " | " | " | 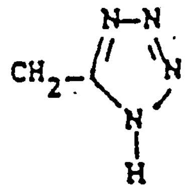 |
| 13 | " | " | " | $CH_2CH_2CO_2H$ |
| 14 | " | " | " | $CH_2SO_3H$ |
| 15 | " | " | " | 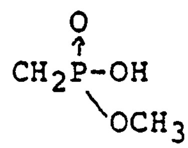 |
| 16 | " | " | " | $CH_2CH_2SO_3H$ |
| 17 | " | 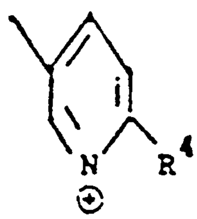 | " | $CO_2H$ |
| 18 | " | " | " | $SO_3H$ |
| 19 | " | " | " | $CH_2CO_2H$ |
| 20 | " | " | " | 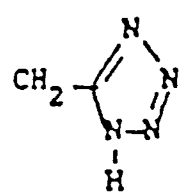 |
| 21 | " | " | " | $CH_2CH_2CO_2H$ |
| 22 | " | " | " | $CH_2SO_3H$ |

| | | | | |
|---|---|---|---|---|
| 23 | " | " | " | 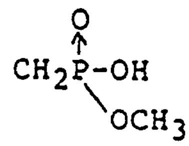 |
| 24 | " | " | " | $CH_2CH_2SO_3H$ |
| 25 | " | " | " | 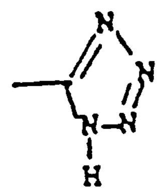 |
| 26 | " | 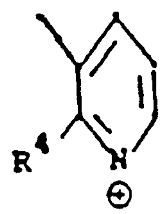 | " | $CO_2H$ |
| 27 | " | " | " | $SO_3H$ |
| 28 | " | " | " | $CH_2CO_2H$ |
| 29 | " | " | " | 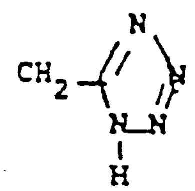 |
| 30 | " | " | " | $CH_2CH_2CO_2H$ |
| 31 | " | " | " | $CH_2SO_3H$ |
| 32 | " | " | " | 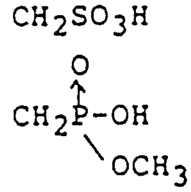 |
| 33 | " | " | " | $CH_2CH_2SO_3H$ |

| | | | | |
|---|---|---|---|---|
| 34 | " | " | " | 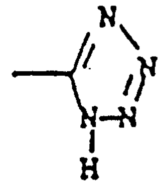 |
| 35 | " | 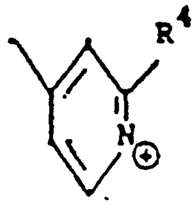 | " | $CO_2H$ |
| 36 | " | " | " | $SO_3H$ |
| 37 | " | " | " | $CH_2CO_2H$ |
| 38 | " | " | " | 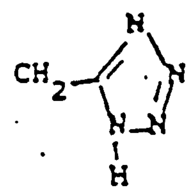 |
| 39 | " | " | " | $CH_2CH_2CO_2H$ |
| 40 | " | " | " | $CH_2SO_3H$ |
| 41 | " | " | " | 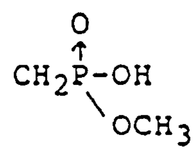 |
| 42 | " | " | " | $CH_2CH_2SO_3H$ |
| 43 | " | " | " | 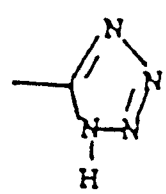 |
| 44 | " | 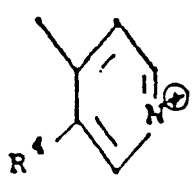 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 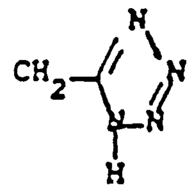 |
| 48 | " | " | " | $CH_2CH_2CO_2H$ |
| 49 | " | " | " | $CH_2SO_3H$ |
| 50 | " | " | " | 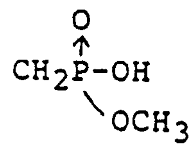 |
| 51 | " | " | " | $CH_2CH_2SO_3H$ |
| 52 | " | " | " | 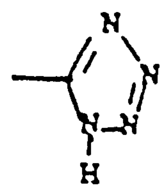 |
| 53 | " | 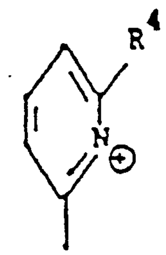 | " | $CO_2H$ |
| 54 | " | " | " | $SO_3H$ |
| 55 | " | " | " | $CH_2CO_2H$ |
| 56 | " | " | " | 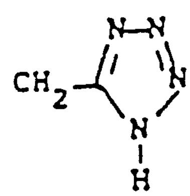 |

| | | | | |
|---|---|---|---|---|
| 57 | " | " | " | $CH_2CH_2CO_2H$ |
| 58 | " | " | " | $CH_2SO_3H$ |
| 59 | " | " | " | 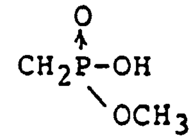 |
| 60 | " | " | " | $CH_2CH_2SO_3H$ |
| 61 | " | 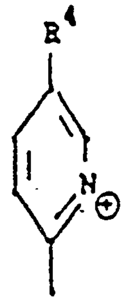 | " | $CO_2H$ |
| 62 | " | " | " | $SO_3H$ |
| 63 | " | " | " | $CH_2CO_2H$ |
| 64 | " | " | " | 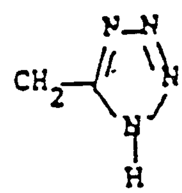 |
| 65 | " | " | " | $CH_2CH_2CO_2H$ |
| 66 | " | " | " | $CH_2SO_3H$ |
| 67 | " | " | " | 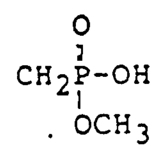 |
| 68 | " | " | " | $CH_2CH_2SO_3H$ |
| 69 | " | 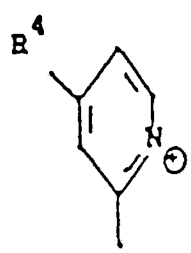 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 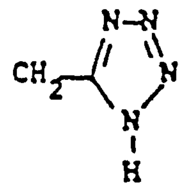 |
| 73 | " | " | " | $CH_2CH_2CO_2H$ |
| 74 | " | " | " | $CH_2SO_3H$ |
| 75 | " | " | " | 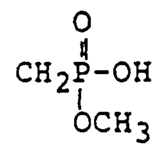 |
| 76 | " | " | " | $CH_2CH_2SO_3H$ |
| 77 | " | 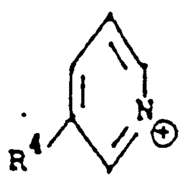 | " | $CO_2H$ |
| 78 | " | " | " | $SO_3H$ |
| 79 | " | " | " | $CH_2CO_2H$ |
| 80 | " | " | " | 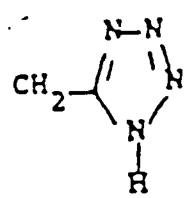 |
| 81 | " | " | " | $CH_2CH_2CO_2H$ |
| 82 | " | " | " | $CH_2SO_3H$ |

| | | | | |
|---|---|---|---|---|
| 83 | " | " | " | 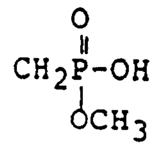 |
| 84 | " | " | " | $CH_2CH_2SO_3H$ |

ausgewählt ist.

6. Kombination aus einer Verbindung nach Anspruch 1 und einem DHP-In-hibitor.

7. Kombination nach Anspruch 6, worin DHP-Inhibitor 7-(L-2-Amino-2-carboxyethylthio)-2-(2,2-dimethylcyclopropancarboxamid)-2-heptensäure ist.

8. Pharmazeutische Zusammensetzung zur Verwendung als Antibiotikum, umfassend eine antibakteriell wirksame Menge einer Verbindung nach Anspruch 1, eine inhibierend wirksame Menge eines DHP-Inhibitors und gegebenfalls einen pharmazeutisch annehmbaren Träger.

9. Pharmazeutische Zusammensetzung nach Anspruch 8,worin der DHP-Inhibitor 7-(L-2-Amino-2-carboxyethylthio)-2-(2,2-dimethylcyclopropancarboxamid)-2-heptensäure ist.

10. Verbindung nach Anspruch 1 mit der Struktur:

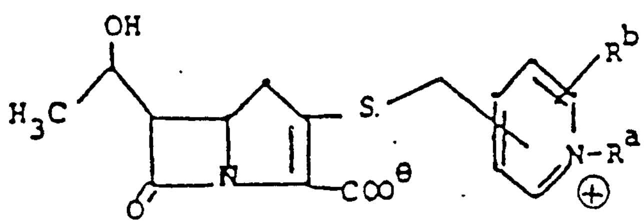

worin $R^a$ $C_1$-$C_4$-Alkyl; oder eine saure Seitenkette der Struktur $-CH_2)_n$-X-$(CH_2)_m$-Y'-A ist, worin:
n = 0-4
m = 0-4
x = $CHR^s$, CH=CH Phenylen(-$C_6H_4$-), NH, N($C_1$-$C_4$-Alkyl), O, S, S=O, C=O, $SO_2$, $SO_2NH$, $CO_2$,CONH, $OCO_2$, OC=O, NCH=O; $R^s$ = H, O($C_1$-$C_4$-Alkyl), $NH_2$, NH($C_1$-$C_4$-Alkyl), N($C_1$-$C_4$-Alkyl)$_2$, CN,$CONH_2$, CON($C_1$-$C_4$-Alkyl)$_2$, $CO_2H$, $SO_2NH_2$, $SO_2NH$($C_1$-$C_4$-Alkyl); Y' = eine Einfachbindung, NH, N($C_1$-$C_4$-Alkyl), O, S;

A wie in Anspruch 1 definiert ist;

$R^b$ Wassertoff; Cyano; oder eine saure Seitenkette der Struktur -A oder $-(CH_2)_n$-X-$(CH_2)_m$ -Y'-A ist, worin:
n = 0=4
m = 0-4
X = $CHR^s$, CH=CH, Phenylen(-$C_6H_4$-), NH, N($C_1$-$C_4$-Alkyl), O, S, S=O, C=O, $SO_2$, $SO_2NH$, $CO_2$, CONH, $OCO_2$, OC=O, NHC=O; $R^s$ = H, O($C_1$-$C_4$-Alkyl), $NH_2$, NH($C_1$-$C_4$-Alkyl), N($C_1$-$C_4$-Alkyl)$_2$, CN, $CONH_2$, CON($C_1$-$C_4$-Alkyl)$_2$, $CO_2H$, $SO_2NH_2$, $SO_2NH$($C_1$-$C_4$-Alkyl);

Y' = eine Einfachbindung, NH, N($C_1$-$C_4$-Alkyl), O,S;

A wie in Anspruch 1 definiert ist;
mit der Maßgabe, daß $R^a$ oder $R^b$ eine saure Seitenkette sein muß.

**11.** Pharmazeutische Zusammensetzung zur Verwendung als Antibiotikum, umfassend eine antibakteriell wirksame Menge einer Verbindung nach Anspruch 10, eine inhibierend wirksame Menge eines DHP-Inhibitors und gegebenenfalls einen pharmazeutischen Träger.

Patentansprüche für folgenden Vertragsstaat: AT

**1.** Verfahren zur Herstellung einer Verbindung mit der Formel:

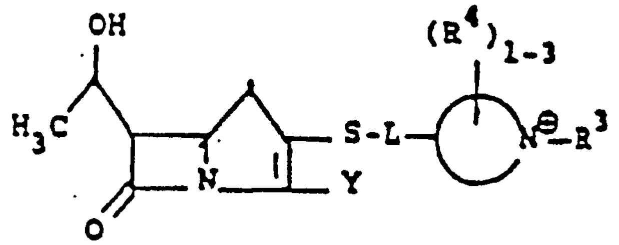

I

worin:

L eine covalente Bindung oder eine überbrückende Gruppe ist, ausgewählt unter-$(CH_2)_{1-4}S$-; --$(CH_2)_{1-4}$-O-; -$(CH_2)_{1-4}$-X"-$(CH_2)_{1-4}$, worin X" für O oder S steht; substituierten oder unsubstituierten linearen $C_1$-$C_4$-, verzweigten $C_1$-$C_6$-Alkyl- oder $C_3$-$C_7$-Cycloalkylgruppen, worin die Substituenten unter $C_1$-$C_6$-Alkyl, O-$C_1$-$C_6$-Alkyl, S-$C_1$-$C_6$-Alkyl, Halogen, OH, $CF_3$, CN, $NH_2$, NH($C_1$-$C_6$-Alkyl), N($C_1$-$C_6$-Alkyl$)_2$, $CO_2H$, $CONH_2$, CONH($C_1$-$C_6$-Alkyl) und CON($C_1$-$C_6$-Alkyl$)_2$ ausgewählt sinf;

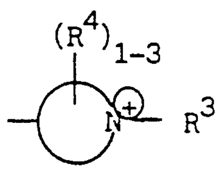 eine Pyridiniumgruppe ist,

worin mindestens ein Substituent $R^3$ und $R^4$ eine saure Seitenkette der Struktur -$(CH_2)_n$-X-$(CH_2)_m$-Y'-A ist, welche im Fall von $R^4$ auch einfach -A sein kann, worin:

n = 0-4

m = 0-4

welches -A ein Rest ist, welcher aus der Gruppe ausgewählt ist, die im westentlichen aus Carboxy($CO_2H$), Phosphono [$P=O(OH_2)_2$], Alkyl-phosphono{$P=O(OH)[O(C_1$-$C_4$-alkyl}, Alkylphosphinyl[$P=O(OH)(C_1$-$C_4$-alkyl)], substituiertem Phosphoramido[$P=O(OH)NH(C_1$-$C_4$-alkyl) und $P=O(OH)NHR^x$], Sulfino($SO_2H$), Sulfo($SO_3H$), 5-Tetrazolyl($CN_4H$), Aryl-sulfonamido-($SO_2NHR^x$) und Acylsulfonamiden, dargestellt durch die Strukturen $CONHSO_2$($C_1$-$C_4$-alkyl), $CONHSO_2N$($C_1$-$C_4$-alkyl$)_2$, $SO_2NHCO$($C_1$-$C_4$-alkyl) und $SO_2NHCOR^x$, worin $R^x$ eine Heteroarylgruppe mit 5 bis 11 Ringatom ist, wovon bis zu fünf Heteroatome sind, besteht;

X = $CHR^s$, CH=CH, Phenylen(-$C_6H_4$-), NH, N ($C_1$-$C_4$-Alkyl), O, S, S=O, C=O, $SO_2$, $SO_2NH$, $CO_2$, CONH, $OCO_2$, OC=O, NHC=O; $R^s$ = H, O($C_1$-$C_4$-Alkyl), $NH_2$, NH($C_1$-$C_4$-Alkyl), N($C_1$-$C_4$-Alkyl$)_2$,

Y' = eine Einfachbindung, NH, N($C_1$-$C_4$-Alkyl), O, S;

worin $R^3$ gegebenfalls:

1) ein unsubstituierter oder substuierter $C_1$-$C_6$-Alkylrest;

2) ein unsubstituierter oder substituierter $C_1$-$C_6$-Alkenylrest;

3) ein unsubstituierter oder substituierter $C_1$-$C_6$-Alkinylrest;

4) ein $C_3$-$C_7$-Cycloalkylrest, worin der Ring substituiert oder unsubstituiert ist und ein oder mehrere Atome durch ein Heteroatom ersetzt sein können;

5) ein $C_3$-$C_7$-Cycloalkylmethylrest, worin der Ring substituiert sein kann und ein oder mehrere Atome

durch ein Heteroatom ersetzt sein können;
6) ein unsubstituierter oder substituierter $C_5$-$C_7$ Cycloalkenylrest;
7) ein unsubstituierter oder substituierter zweiwertiger $C_2$-$C_6$-Alkylidenrest, welcher gegebenenfalls durch ein Heteroatom unterbröchen ist und welcher an die Heteroaryliumgruppe gebunden ist, um einen Ring auszubilden, welcher carbocyclisch ist oder in welchem ein oder mehrere Atome durch ein Heteroatom ersetzt sind; der neue Ring kann eine oder mehrere Dopplbindungen enthalten;
8) ein unsubstituierter oder substituierter Phenylrest;
9) ein unsubstituierter oder substituierter Phenyl($C_1$-$C_4$-alkyl)-Rest;
10) ein Cyano($C_1$-$C_4$-alkyl)-Rest;
11) ein Carbamoyl($C_1$-$C_4$-alkyl)-Rest;
12) ein Hydroxy($C_1$-$C_4$-alkyl)-Rest; oder
13) ein Amino($C_1$-$C_4$-alkyl)-Rest, worin das Stickstoffatom unsubstituiert oder mit ein bis drei $C_1$-$C_4$-Alkylgruppen substituiert ist, ist;
worin die Substituenten in den obigen Definitionen von $R^3$ unabhängig voneinander aus der Gruppe ausgewählt sind, welche aus den Definitionen von $R^4$, welche unten angegeben sind, besteht; und worin $R^4$ gegebenenfalls unabhängig voneinander ausgewählt ist unter:
a) einer Trifluormethylgruppe;
b) einem Halogenatom;
c) einem unsubstituierten $C_1$-$C_4$-Alkoxylrest;
d) einer Hydroxygruppe;
e) einem unsubstituierten ($C_1$-$C_6$-Alkyl)carbonyloxyrest;
f) einem Carbamoyloxyrest, welcher unsubstituiert ist oder am Stickstoff mit einer oder zwei $C_1$-$C_4$-Alkylgruppen substituiert ist;
g) einem $C_1$-$C_6$-Alkylthiorest, einem $C_1$-$C_6$-Alkylsulfinylrest oder einem $C_1$-$C_6$-Alkylsulfonylrest, wovon jeder an der Alkylgruppe unsubstituiert ist;
h) einer Sulfamoylgruppe,welche unsubstituiert ist oder am Stickstoff durch eine oder zwei $C_1$-$C_4$-Alkylgruppen substituiert ist;
i) einer Aminogruppe;
j) einer Mono($C_1$-$C_4$-alkyl)-amino oder Di($C_1$-$C_4$-alkyl)aminogruppe, wovon jede an der Alkylgruppe unsubstituiert ist;
k) einer Formylamninogruppe;
l) einem unsubstituierten ($C_1$-$C_6$-Alkyl)carbonylaminorest;
m) einem ($C_1$-$C_4$-Alkoxy)carbonylaminorest;
n) einer Ureidogruppe, in welcher der endständige Stickstoff unsubstituiert ist oder mit einer oder zwei $C_1$-$C_4$-Alkylgruppen substituiert ist;
o) einer ($C_1$-$C_6$-Alkyl)sulfonamidogruppe;
p) einer Cyanogruppe;
q) einem Formyl- oder einem acetalisierten Formylrest;
r) einem unsubstituierten ($C_1$-$C_6$-Alkyl)carbonylrest, worin das Carbonyl frei oder acetalisiert ist;
s) einem unsubstituierten Phenylcarbonylrest;
t) einem Hydroximinomethylrest, in welchem das Sauerstoff- oder Kohlenstoffatom gegebenenfalls durch eine $C_1$-$C_4$-Alkylgruppe substituiert ist;
u) einem ($C_1$-$C_6$-Alkoxy)carbonylrest;
v) einem Carbamoylrest, welcher unsubstituiert ist oder am Stickstoff durch eine oder zwei $C_1$-$C_4$-Alkylgruppen substituiert ist;
w) einem N-Hydroxycarbamoyl- oder N($C_1$-$C_4$-alkoxy)carbamoylrest, in welchem das Stickstoffatom zusätzlich durch eine $C_1$-$C_4$-Alkylgruppe substituiert sein kann;
x) einer Thiocarbamoylgruppe;

y) einer Amidinogruppe 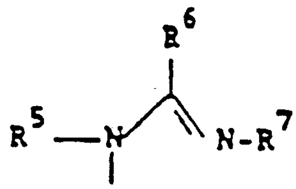 , 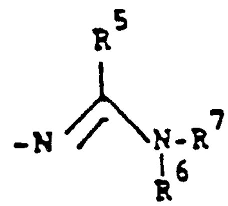

worin $R^5$, $R^6$ und $R^7$ unabhängig voneinander Wasserstoff, $C_1$-$C_4$-Alkyl sind, oder worin zwei der

Allgruppen gemeinsam einen $C_2$-$C_6$-Alkylidenrest bilden, welcher gegebenenfalls durch ein Heteroatom unterbrochen ist, und welche aneinander gebunden sind, um einen Ring auszubilden;

z) einer Carboxamidinogruppe 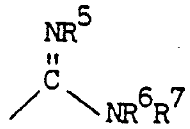 , worin $R^5$, $R^6$ und $R^7$ wie oben definiert sind;

aa) einer Guanidinylgruppe, worin $R^6$ in y) oben $NR^8R^9$ ist, und $R^8$ und $R^9$ wie oben für $R^5$ bis $R^7$ definiert sind;
ab) Wasserstoff;
ac) einem unsubstituierten $C_1$-$C_6$-Alkylrest;
ad) einem unsubstituierten $C_1$-$C_6$-Alkenylrest;
ae) einem unsubstituierten $C_1$-$C_6$-Alkinylrest;
af) einem $C_3$-$C_7$-Cycloalkylrest, in welchem der Ring unsubstituiert ist und ein oder mehrere Atome durch ein Heteroatom ersetzt sein können;
ag) einem $C_3$-$C_7$-Cycloalkylmethylrest, in welchem ein oder mehrere Atome durch ein Heteroatom ersetzt sein können;
ah) einem unsubstituierten $C_5$-$C_7$-Cycloalkenylrest;
ai) einem unsubstituierten Phenylrest; und
aj) einem unsubstituierten Phenyl($C_1$-$C_4$-alkyl)-Rest; und worin Heteroatom in den obigen Definitionen von A, $R^3$ und $R^4$ Stickstoff, Sauerstoff oder Schwefel bedeutet, und welches unabhängig voneinander ausgewählt ist, wenn mehr als ein Heteroatom auftritt; und

Y ausgewählt ist unter: i) COCH oder einem pharmazeutisch annehmbaren Ester oder Salz hievon,
ii) COOR', worin R' eine entfernbare Carboxyschutzgruppe ist,
iii) COOM, worin M ein Alkalimetall ist, oder
iv) $COO^-$; mit der Maßgabe, daß, wenn Y eine andere Bedeutung als iv) besitzt, ein Gegenion $Z^-$ zur Verfügung steht; welches umfaßt

A. das Umsetzen einer Verbindung der Formel:

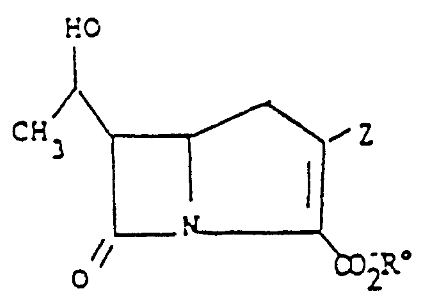

mit einer Verbindung der Formel:

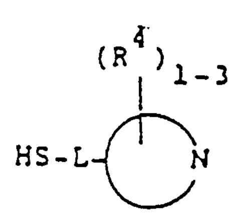

in Gegenwart einer Base, das Behandeln der erhaltenen Verbindung der Formel:

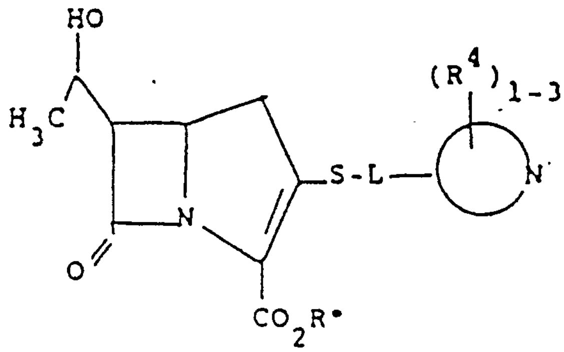

mit $R^3X'$ oder $R^3O^{\oplus\ominus}X'^{\ominus}$, und das wahlweise Deblockieren der erhaltenen Verbindung der Formel:

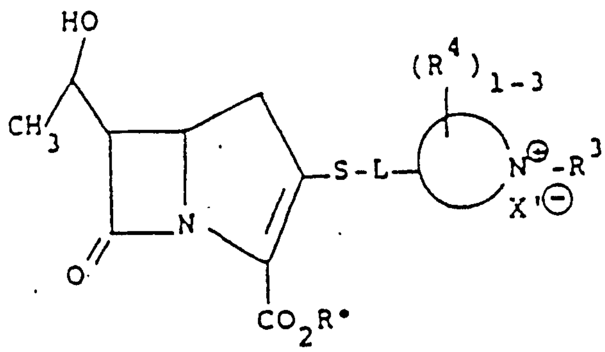

,

um

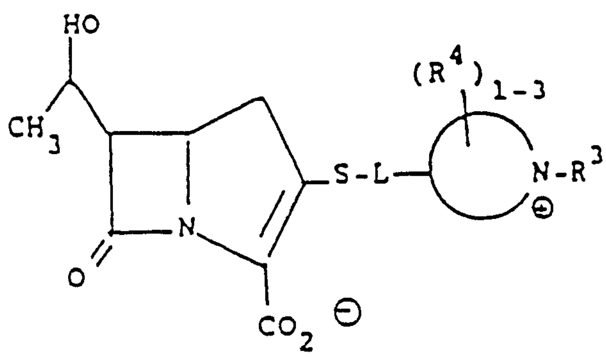

I

zu erhalten,
worin Z und X' Leaving-Gruppen sind, oder X' eine anionische Gruppe ist: und R° eine Schutzgruppe darstellt, und $R^3$, L,

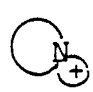

und $R^4$ wie oben definiert sind,
B. das Umsetzen einer Verbindung der Formel:

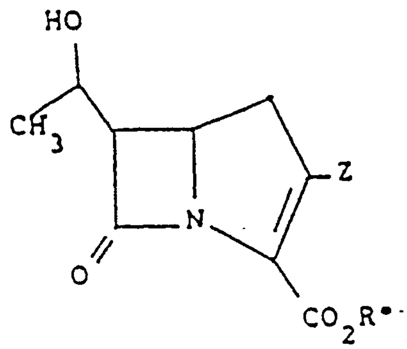

mit einer Verbindung der Formel:

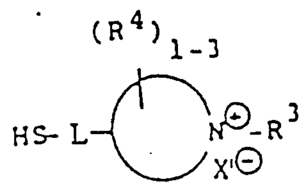

in Gegenwart einer Base, und das wahlweise Deblockieren der erhaltenen Verbindung der Formel:

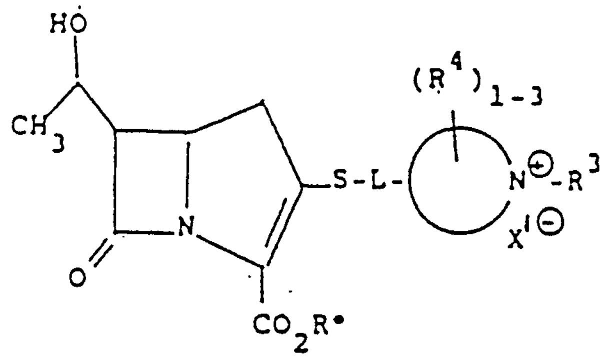

,

um

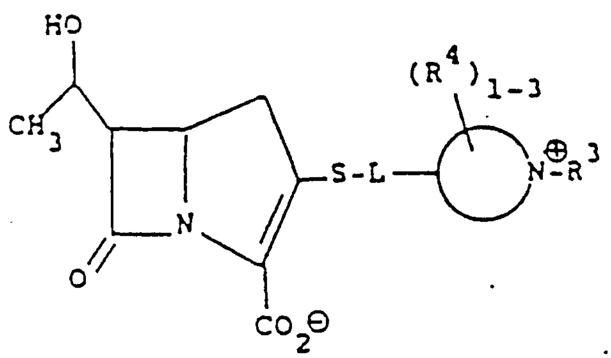

.

zu erhalten,
worin Z, X', L, R°, $R^3$,

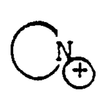

und $R^4$ wie oben definiert sind,

C. das Umsetzen einer Verbindung der Formel:

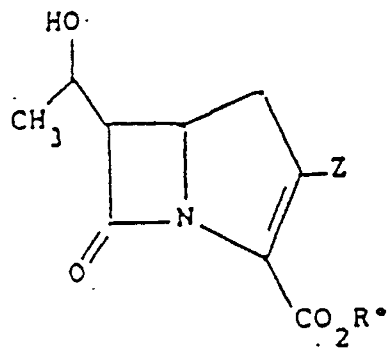

mit NaHS,

das Behandeln der erhaltenen Verbindung der Formel:

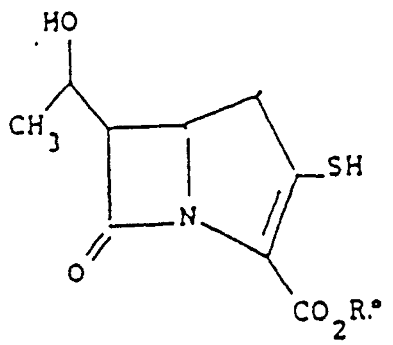

mit

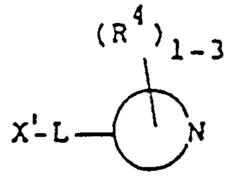

,

das Behandeln der erhaltenen Verbindung der Formel:

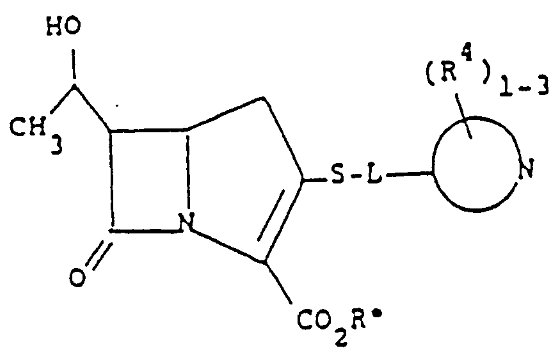

mit $R^3X'$ oder $R^3O^{\oplus}X'^{\ominus}$

und das wahlweise Deblockieren der erhaltenen Verbindung der Formel:

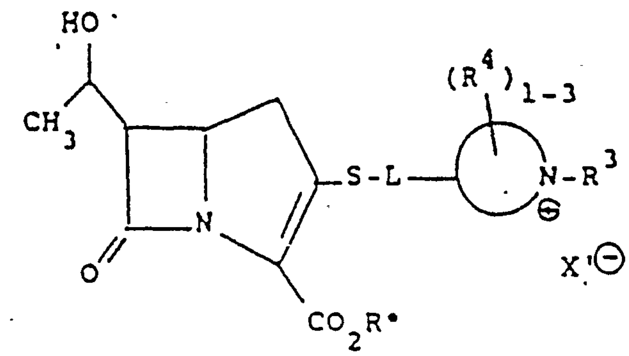

,

um

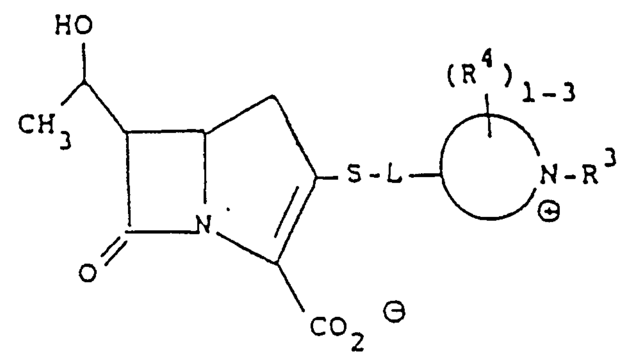

I

zu erhalten,
worin Z, X', L, R°, $R^3$,

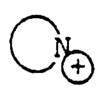

und $R^4$ wie vorstehend definiert sind,
D. das Umsetzen einer Verbindung der Formel:

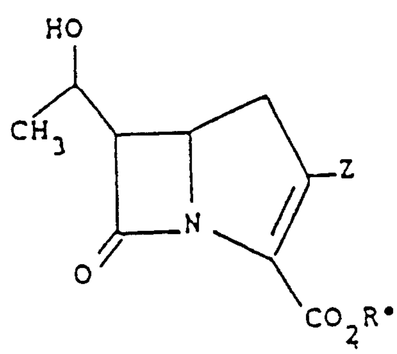

mit NaHS,

das Behandeln der erhaltenen Verbindung der Formel:

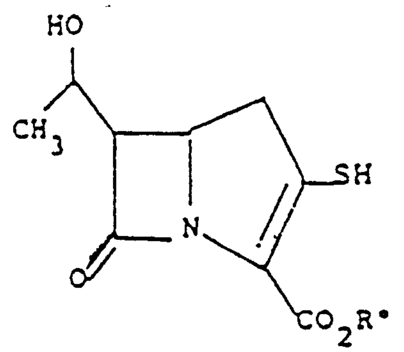

mit

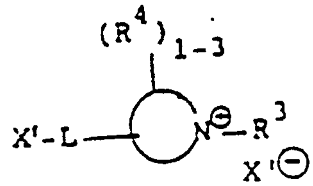

in Gegenwart einer Base, und das wahlweise Deblockieren der erhaltenen Verbindung der Formel:

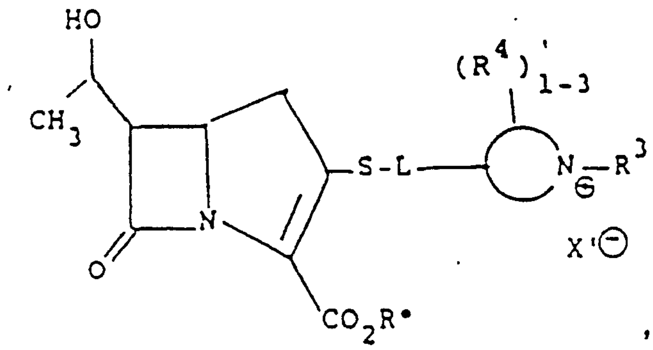

,

um

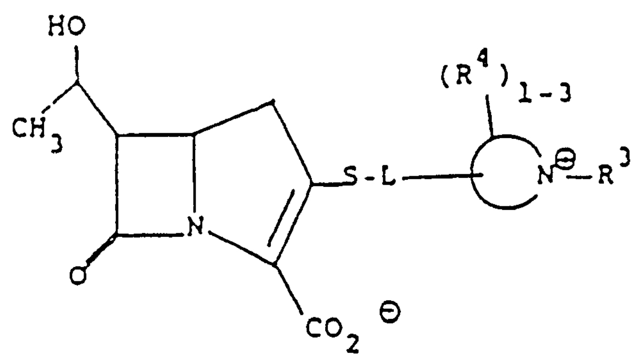

I

zu erhalten,
worin Z, X', R°, $R^3$, L,

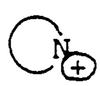

und $R^4$ wie vorstehend definiert sind.

2. Verfahren nach Anspruch 1, zur Herstellung einer Verbindung, worin L ein substituiertes oder unsubstituiertes, verzweigtes oder lineares $C_1$-$C_4$-Alkyl ist.

3. Verfahren nach Anspruch 2, zur Herstellung einer Verbindung, worin $R^3$ eine unsubstituierte oder substituierte $C_1$-$C_4$-Alkylgruppe ist.

4. Verfahren nach Anspruch 3, zur Herstellung einer Verbindung, worin L $-CH_2-$, $-CH(CH_3)-$ oder $-(CH_2)_2-$

ist.

5. Verfahren nach Anspruch 1, zur Herstellung einer Verbindung, worin die Verbindung eine Verbindung ist, welche aus der Gruppe bestehend aus:

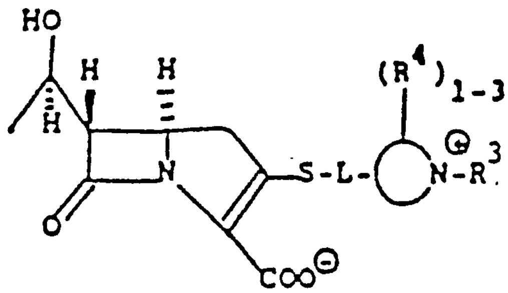

| Verbindung Nr. | L | 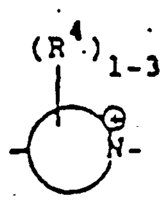 | $R^3$ |
|---|---|---|---|
| 1 | $-CH_2-$ | 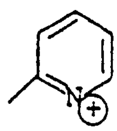 | $CH_2CO_2H$ |
| 2 | " | " | $CH_2\overset{\overset{O}{\uparrow}}{P}(OH)OCH_3$ |
| 3 | " | " | $CH_2SO_3H$ |
| 4 | " | " | 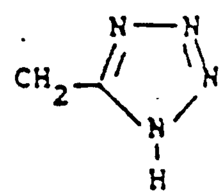 |
| 5 | " | 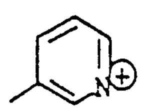 | $CH_2CO_2H$ |
| 6 | " | " | $CH_2\overset{\overset{O}{\uparrow}}{P}(OH)OCH_3$ |
| 7 | " | " | $CH_2SO_3H$ |

| | | | |
|---|---|---|---|
| 8 | " | 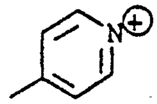 | $CH_2CO_2H$ |
| 9 | " | " | $CH_2\overset{O}{\overset{\uparrow}{P}}(OH)OCH_3$ |
| 10 | " | " | $CH_2SO_3H$ |
| 11 | $-CH_2-$ | 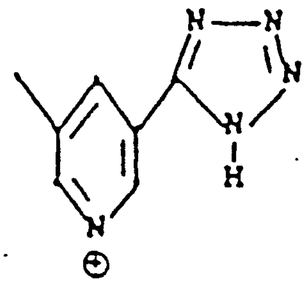 | $CH_3$ |
| 12 | " | 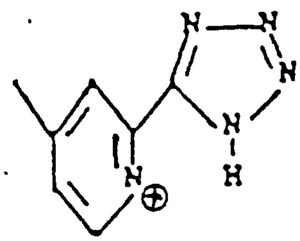 | " |
| 13 | " | 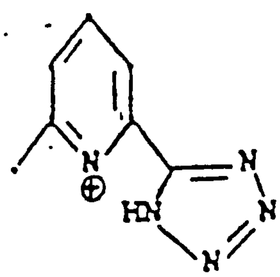 | " |
| 14 | " | 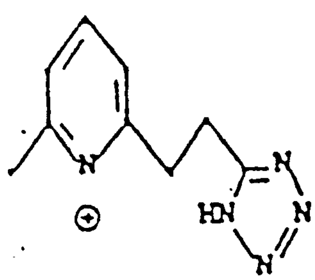 | " |

| | | | |
|---|---|---|---|
| 15 | Bindung | 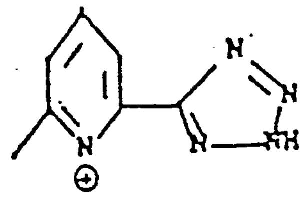 | " |

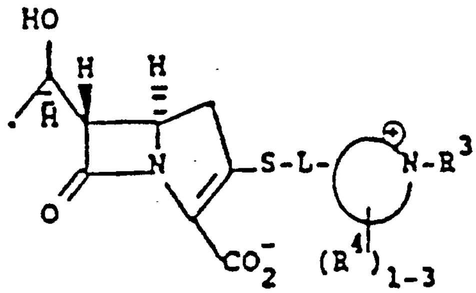

| Verbindung Nr. | L | 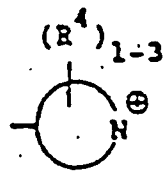 | $R^3$ | $R^4$ |
|---|---|---|---|---|
| 1 | $-CH_2-$ | 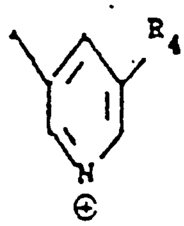 | $CH_3$ | $CO_2H$ |
| 2 | " | " | " | $SO_3H$ |
| 3 | " | " | " | $CH_2CO_2H$ |
| 4 | " | " | " | 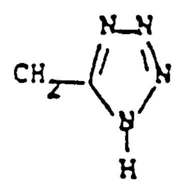 |
| 5 | " | " | " | $CH_2CH_2CO_2H$ |
| 6 | " | " | " | $CH_2SO_3H$ |
| 7 | " | " | " | 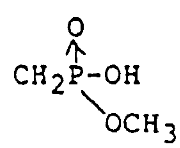 |
| 8 | " | " | " | $CH_2CH_2SO_3H$ |

| | | | | |
|---|---|---|---|---|
| 9 | " | 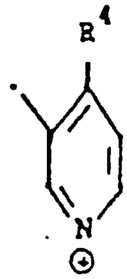 | " | $CO_2H$ |
| 10 | " | " | " | $SO_3H$ |
| 11 | " | " | " | $CH_2CO_2H$ |
| 12 | " | " | " | 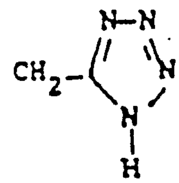 |
| 13 | " | " | " | $CH_2CH_2CO_2H$ |
| 14 | " | " | " | $CH_2SO_3H$ |
| 15 | " | " | " | 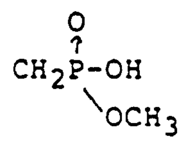 |
| 16 | " | " | " | $CH_2CH_2SO_3H$ |
| 17 | " | 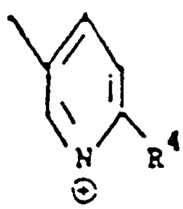 | " | $CO_2H$ |
| 18 | " | " | " | $SO_3H$ |
| 19 | " | " | " | $CH_2CO_2H$ |
| 20 | " | " | " | 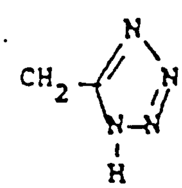 |

| | | | | |
|---|---|---|---|---|
| 21 | " | " | " | $CH_2CH_2CO_2H$ |
| 22 | " | " | " | $CH_2SO_3H$ |
| 23 | " | " | " | 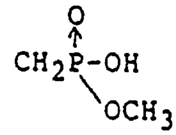 |
| 24 | " | " | " | $CH_2CH_2SO_3H$ |
| 25 | " | " | " | 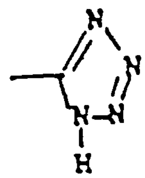 |
| 26 | " | 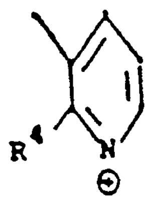 | " | $CO_2H$ |
| 27 | " | " | " | $SO_3H$ |
| 28 | " | " | " | $CH_2CO_2H$ |
| 29 | " | " | " | 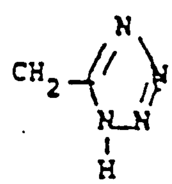 |
| 30 | " | " | " | $CH_2CH_2CO_2H$ |
| 31 | " | " | " | $CH_2SO_3H$ |
| 32 | " | " | " | 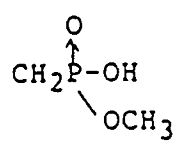 |
| 33 | " | " | " | $CH_2CH_2SO_3H$ |

| | | | | |
|---|---|---|---|---|
| 34 | " | " | " | 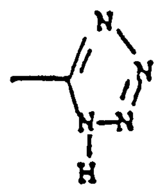 |
| 35 | " | 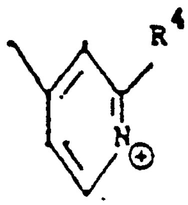 | " | $CO_2H$ |
| 36 | " | " | " | $SO_3H$ |
| 37 | " | " | " | $CH_2CO_2H$ |
| 38 | " | " | " | 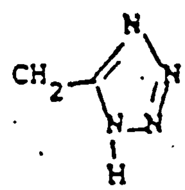 |
| 39 | " | " | " | $CH_2CH_2CO_2H$ |
| 40 | " | " | " | $CH_2SO_3H$ |
| 41 | " | " | " | 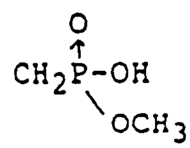 |
| 42 | " | " | " | $CH_2CH_2SO_3H$ |
| 43 | " | " | " | 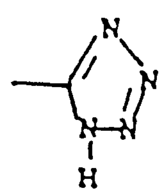 |
| 44 | " | 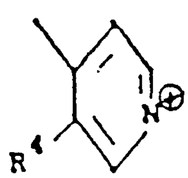 | " | $CO_2H$ |

| | | | | |
|---|---|---|---|---|
| 45 | " | " | " | $SO_3H$ |
| 46 | " | " | " | $CH_2CO_2H$ |
| 47 | " | " | " | 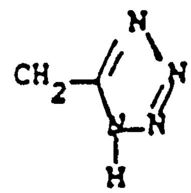 |
| 48 | " | " | " | $CH_2CH_2CO_2H$ |
| 49 | " | " | " | $CH_2SO_3H$ |
| 50 | " | " | " | 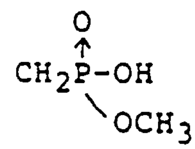 |
| 51 | " | " | " | $CH_2CH_2SO_3H$ |
| 52 | " | " | " | 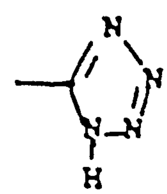 |
| 53 | " | 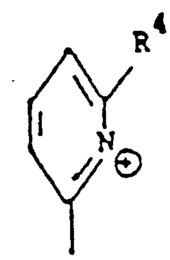 | " | $CO_2H$ |
| 54 | " | " | " | $SO_3H$ |
| 55 | " | " | " | $CH_2CO_2H$ |
| 56 | " | " | " | 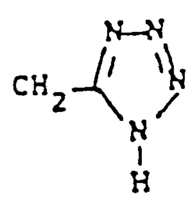 |

| | | | | |
|---|---|---|---|---|
| 57 | " | " | " | $CH_2CH_2CO_2H$ |
| 58 | " | " | " | $CH_2SO_3H$ |
| 59 | " | " | " | 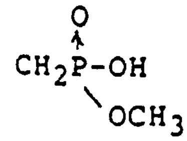 |
| 60 | " | " | " | $CH_2CH_2SO_3H$ |
| 61 | " | 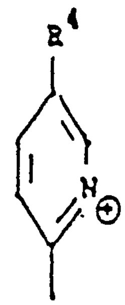 | " | $CO_2H$ |
| 62 | " | " | " | $SO_3H$ |
| 63 | " | " | " | $CH_2CO_2H$ |
| 64 | " | " | " | 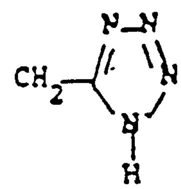 |
| 65 | " | " | " | $CH_2CH_2CO_2H$ |
| 66 | " | " | " | $CH_2SO_3H$ |
| 67 | " | " | " | 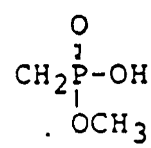 |
| 68 | " | " | " | $CH_2CH_2SO_3H$ |
| 69 | " | 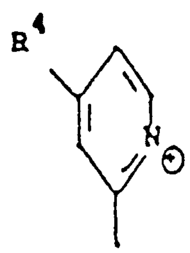 | " | $CO_2H$ |

| | | | | |
|---|---|---|---|---|
| 70 | " | " | " | $SO_3H$ |
| 71 | " | " | " | $CH_2CO_2H$ |
| 72 | " | " | " | 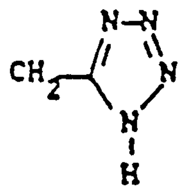 |
| 73 | " | " | " | $CH_2CH_2CO_2H$ |
| 74 | " | " | " | $CH_2SO_3H$ |
| 75 | " | " | " | 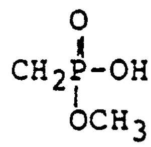 |
| 76 | " | " | " | $CH_2CH_2SO_3H$ |
| 77 | " | 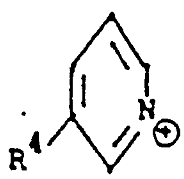 | " | $CO_2H$ |
| 78 | " | " | " | $SO_3H$ |
| 79 | " | " | " | $CH_2CO_2H$ |
| 80 | " | " | " | 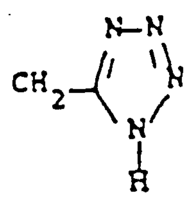 |
| 81 | " | " | " | $CH_2CH_2CO_2H$ |
| 82 | " | " | " | $CH_2SO_3H$ |

| | | | | |
|---|---|---|---|---|
| 83 | " | " | " | 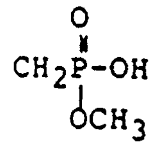 |
| 84 | " | " | " | $CH_2CH_2SO_3H$ |

ausgewählt ist.

6. Verfahren nach Anspruch 1, worin die erhaltene Verbindung zusätzlich mit einem DHP-Inhibitor kombiniert wird.

7. Verfahren nach Anspruch 6, worin der DHP-Inhibitor 7-(L-2-Amino-2-carboxyethylthio)-2-(2,2-dimethylcyclopropancarboxamid)-2-heptensäure ist.

8. Verfahren nach Anspruch 1, worin eine antibakteriell wirksame Menge der erhaltenen Verbindung zusätzlich mit einer inhibierend wirksamen Menge eines DHP-Inhibitors und gegebenenfalls mit einem pharmazeutisch annehmbaren Träger kombiniert wird.

9. Verfshren nach Anspruch 8, worin der DHP-Inhibitor 7-(L-2-Amino-2-carboxyethylthio)-2-(2,2-dimethylcyclopropancarboxamid)-2-heptensäure ist.

10. Verfahren nach Anspruch 1, zur Herstellung einer Verbindung mit der Struktur:

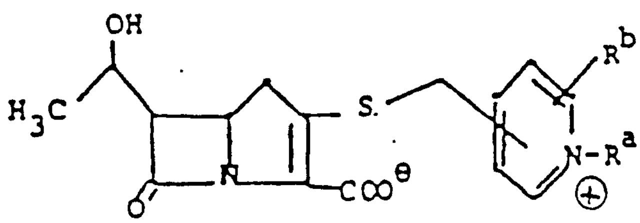

,

worin $R^a$ $C_1$-$C_4$-Alkyl; oder eine saure Seitenkette der Struktur $-CH_2)_n-X-(CH_2)_m-Y'-A$ ist, worin:
n = 0-4
m = 0-4

X = $CHR^s$, CH = CH, Phenylen(-$C_6H_4$-), NH (N$C_1$-$C_4$-Alkyl), O, S, S = O, C = O $SO_2$, $SO_2NH$, $CO_2$, CONH, $OCO_2$, OC = O, NHC = O; $R^s$ = H, O($C_1$-$C_4$-Alkyl), $NH_2$, NH($C_1$-$C_4$-Alkyl), N($C_1$-$C_4$-Alkyl)$_2$, CN,$CONH_2$ CON($C_1$-$C_4$-Alkyl)$_2$ $CO_2H$ $SO_2NH_2$, $SO_2NH$($C_1$-$C_4$-Alkyl);
Y' = eine Einfachbindung, NH, N($C_1$-$C_4$-Alkyl), O, S;
A wie in Anspruch 1 definiert ist;
$R^b$ Wasserstoff; Cyano; oder eine saure Seitenkette der Struktur -A oder $-(CH_2)_n-X-(CH_2)_m-Y'-A$ ist, worin:
n = 0 = 4
m = 0-4

x = $CHR^s$, CH = CH Phenylen(-$C_6H_4$-), NH, N($C_1$-$C_4$-Alkyl), O, S, S = O, C = O, $SO_2$, $SO_2NH$, $CO_2$, CONH, $OCO_2$, OC = O, NHC = O;
C = O, $SO_2$,$SO_2NH$, $CO_2$, CONH, $OCO_2$, OC = O, NHC = O; $R^s$ = H, O($C_1$-$C_4$-Alkyl), $NH_2$, NH($C_1$-$C_4$-Alkyl), N($C_1$-$C_4$-Alkyl)$_2$, CN, $CONH_2$, CON($C_1$-$C_4$-Alkyl)$_2$, $CO_2H$, $SO_2NH_2$, $SO_2NH$($C_1$-$C_4$-Alkyl);

Y = eine Einfachbindung, NH, N($C_1$-$C_4$-Alkyl), O, S;

A wie in Anspruch 1 definiert ist;
mit der Maßgabe, daß $R^a$ oder $R^b$ eine saure Seitenkette sein muß.

**11.** Verfahren nach Anspruch 10, worin eine antibakteriell wirksame Menge der erhaltenen Verbindung zusätzlich mit einer inhibierend wirksamen Menge eines DHP-Inhibitors und gegebenenfalls mit einem pharmazeutisch annehmbaren Träger kombiniert wird.